# EUROPEAN PATENT SPECIFICATION

(11) **EP 1 592 701 B1**
(45) Date of publication and mention of the grant of the patent: **10.04.2013**
(21) Application number: 04707507.2
(22) Date of filing: 03.02.2004
(51) Int. Cl.: C07H 17/08, A01N 43/90

(54) **AVERMECTINS AND AVERMECTIN MONOSACCHARIDES SUBSTITUTED IN THE 4'- AND 4''- POSITION HAVING PESTICIDAL PROPERTIES**
AVERMECTINE UND IN DER 4'- UND 4''- STELLUNGSUBSTITUIERTE AVERMECTINMONOSACCHARIDE MIT PESTIZIDEN EIGENSCHAFTEN
AVERMECTINES ET MONOSACCHARIDES D'AVERMECTINE SUBSTITUES EN POSITION 4' ET 4'' A PROPRIETES PESTICIDES

(30) Priority: 04.02.2003 GB 0302547
(43) Date of publication of application: 09.11.2005
(73) Proprietor: Merial Limited, Harlow, Essex CM19 5TG (GB)
(72) Inventor: MURPHY KESSABI, Fiona, Syngenta Crop Protection AG, CH-4058 Basel (CH); CASSAYRE, Jérôme, Syngenta Crop Protection AG, CH-4058 Basel (CH); QUARANTA, Laura, Syngenta Crop Protection AG, CH-4058 Basel (CH); PITTERNA, Thomas, Syngenta Crop Protection AG, CH-4058 Basel (CH); MAIENFISCH, Peter, Syngenta Crop Protection AG, CH-4058 Basel (CH)
(74) Representative: Clyde-Watson, Zöe
(86) International application number: PCT/EP2004/000977
(87) International publication number: WO 2004/069853

(56) References cited:
- EP-A- 1 160 252
- P.T. MEINKE ET AL: "Synthesis of avermectin B1-4'', 4''a-oxide: A precursor of potent anthelmintic agents" BIOORGANIC MEDICINAL CHEMISTRY LETTERS, vol. 2, 1992, pages 537-540, XP002284017

## Description

The invention provides (1) a compound of the formula 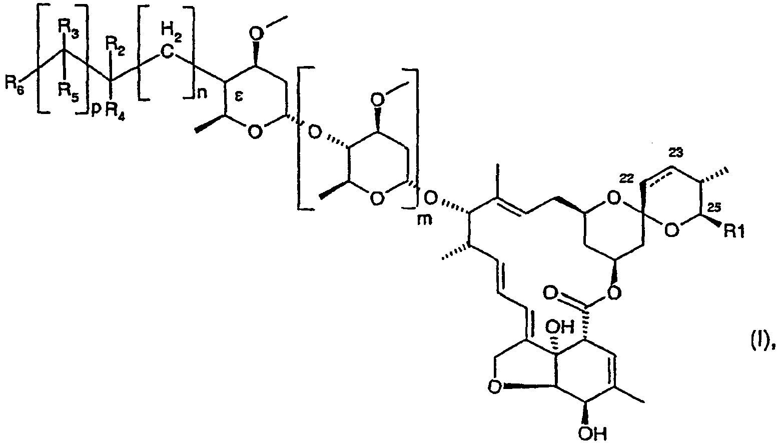 wherein the bond of atoms C₂₂ and C₂₃ is a single or double bond;
m is 0;
n is 0, 1 or 2;
p is 0 or 1;
R₁ is C₁-C₁₂-alkyl, C₃-C₈ₐ-cycloalkyl or C₂-C₁₂-alkenyl;
R₂ is H, C₁-C₁₂-alkyl, C₁-C₁₂-haloalkyl, C₁-C₁₂-hydroxyalkyl, OH, halogen, -N₃, SCN, NO₂, CN, C₃-C₈cycloalkyl unsubstituted or substituted by from one to three methyl groups, C₃-C₈halocycloalkyl, C₁-C₁₂alkoxy, C₁-C₆alkoxy-C₁-C₆alkyl, C₁-C₆alkoxy-C₁-C₆alkoxy, C₁-C₆alkoxy-C₁-C₆alkoxy-C₁-C₆alkyl, C₂-C₁₂alkenyl, C₂-C₁₂haloalkenyl, C₂-C₁₂haloalkenyloxy, C₂-C₁₂alkynyl, C₂-C₁₂haloalkynyl, C₃-C₁₂alkynyloxy, C₃-C₁₂haloalkynyloxy, -P(=O)(OC₁-C₆alkyl)₂, -Si(C₁-C₆alkyl)₃, -(CH₂)-Si(C₁-C₆alkyl)₃, -Si(OC₁-C₆alkyl)₃, -N(R₉)₂, -(CH₂)-N(R₉)₂, wherein the two substituents R₉ are independent of each other, -C(=X)-R₇, -(CH₂)-C(=X)-R₇, -O-C(=X)-R₇, -(CH₂)-O-C(=X)-R₇, -S-C(=X)-R₇, -(CH₂)-S-C(=X)-R₇, -NR₉C(=X)R₇, -(CH₂)-NR₉C(=X)R₇, -NR₉NHC(=X)-R₇, -NR₉-OR₁₀, -(CH₂)-NR₉-OR₁₀, -SR₉, -S(=O)₁₁, -S(=O)₂R₁₁, aryl, heterocyclyl, aryloxy or heterocyclyloxy; wherein the aryl, heterocyclyl, aryloxy and heterocyclyloxy radicals are unsubstituted or, depending upon the possibilities of substitution at the ring, mono- to penta-substituted by substituents selected from the group consisting of OH, halogen, CN, NO₂, SCN, -N₃, C₁-C₁₂alkyl, C₃-C₈cycloalkyl, C₁-C₁₂haloalkyl, C₁-C₁₂alkoxy, C₁-C₁₂haloalkoxy, C₁-C₁₂alkylthio, C₁-C₁₂haloalkylthio, C₁-C₆alkoxy-C₁-C₆alkyl, C₂-C₈alkenyl, C₂-C₈alkynyl, C₂-C₁₂haloalkenyl, C₂-C₁₂haloalkenyloxy, C₂-C₁₂haloalkynyl, C₃-C₁₂alkynyloxy, C₃-C₁₂haloalkynyloxy and phenoxy;
or, when p is 1, R₂ together with R₃ is a bond;
or R₂ together with R₄ is =O or =S;
or R₂ together with R₄ form with the carbon to which they are bound a three- to seven-membered ring, which may be monocyclic or bicyclic, and may be saturated or unsaturated, and that may contain one or two hetero atoms selected from the group consisting of N, O and S, and which is either unsubstituted or independently of one another mono- to penta-substituted with substituents selected from OH, =O, SH, =S, halogen, CN, -N₃, SCN, NO₂, aryl, C₁-C₁₂alkyl, C₃-C₈cycloalkyl, C₁-C₁₂haloalkyl, C₁-C₁₂alkoxy, C₁-C₁₂haloalkoxy, C₁-C₁₂alkylthio, C₁-C₁₂haloalkylthio, C₁-C₆alkoxy-C₁-C₆alkyl, C₂-C₈alkenyl, C₂-C₈alkynyl, C₂-C₁₂haloalkenyl, C₂-C₁₂haloalkenyloxy, C₂-C₁₂haloalkynyl, C₃-C₁₂alkynyloxy, C₃-C₁₂haloalkynyloxy, phenoxy, phenyl-C₁-C₆alkyl, -N(R₉)₂ wherein the two R₉ are independent of each other, C₁-C₆alkylsulfinyl, C₃-C8cycloalkylsulfinyl, C₁-C₆haloalkylsulfinyl, C₃-C₈halocycloalkylsulfinyl, C₁-C₆alkylsulfonyl, C₃-C₈cycloalkylsulfonyl, C₁-C₆haloalkylsulfonyl and C₃-C₈halocycloalkylsulfonyl; or
R₂ together with R₄ is =NN(R₁₂)₂, wherein the two substituents R₉ are independent of each other;
or, when p is 0, R₂ together with R₄ and R₆ is ≡N;
or when p is 0, R₂ together with R₆ is =NOR₁₂ or =NN(R₁₂)₂, wherein the two substituents R₉ are independent of each other;
R₃ is H, C₁-C₁₂-alkyl, halogen, halo-C₁-C₂alkyl, CN, -N₃, SCN, NO₂, C₃-C₈cycloalkyl unsubstituted or substituted by from one to three methyl groups, C₃-C₈halocycloalkyl, C₁-C₁₂alkoxy, C₁-C₆alkoxy-C₁-C₆alkyl, C₁-C₆alkoxy-C₁-C₆alkoxy-C₁-C₆alkyl, C₃-C₈cycloalkoxy, C₁-C₁₂haloalkoxy, C₁-C₁₂alkylthio, C₃-C₈cycloalkylthio, C₁-C₁₂haloalkylthio, C₁-C₁₂alkylsulfinyl, C₃-C₈cycloalkylsulfinyl, C₁-C₁₂haloalkylsulfinyl, C₃-C₈halocycloalkylsulfinyl, C₁-C₁₂alkylsulfonyl, C₃-C₈cycloalkylsulfonyl, C₁-C₁₂haloalkylsulfonyl, C₃-C₈halocycloalkylsulfonyl, C₂-C₈-alkenyl, C₂-C₈alkynyl, C₂-C₁₂haloalkenyl, C₂-C₁₂haloalkenyloxy, C₂-C₁₂haloalkynyl, C₃-C₁₂haloalkynyloxy, -N(R₉)₂, wherein the two substituents R₉ are independent of each other, aryl, heterocyclyl, aryloxy or heterocyclyloxy; wherein the aryl, heterocyclyl, aryloxy and heterocyclyloxy radicals are unsubstituted or, depending upon the possibilities of substitution at the ring, mono- to penta-substituted by substituents selected from the group consisting of halogen, CN, NO₂, C₁-C₁₂alkyl, C₃-C₈cycloalkyl, C₁-C₁₂haloalkyl, C₁-C₁₂alkoxy, C₁-C₁₂haloalkoxy,C₁-C₁₂alkylthio,C₁-C₁₂haloalkylthio, C₁-C₆alkoxy-C₁-C₆alkyl, C₂-C₈alkenyl, C₂-C₈-alkynyl, C₂-C₁₂haloalkenyl, C₂-C₁₂haloalkenyloxy, C₂-C₁₂haloalkynyl and C₃-C₁₂haloalkynyloxy;
or when p is 1, R₃ together with R₂ is a bond;
R₄ is H, C₁-C₁₂-alkyl, C₁-C₁₂-haloalkyl, C₁-C₁₂-hydroxyalkyl, OH, halogen, NO₂, CN, C₃-C₈-cycloalkyl unsubstituted or substituted by from one to three methyl groups, C₃-C₈halocycloalkyl, C₁-C₁₂alkoxy, C₁-C₆alkoxy-C₁-C₆alkyl, C₁-C₆alkoxy-C₁-C₆alkoxy, C₁-C₆alkoxy-C₁-C₆alkoxy-C₁-C₆alkyl, C₂-C₁₂alkenyl, C₂-C₁₂haloalkenyl, C₂-C₁₂haloalkenyloxy, C₂-C₁₂alkynyl, C₂-C₁₂haloalkynyl, C₃-C₁₂haloalkynyloxy, -P(=O)(OC₁-C₆alkyl)₂, -Si(C₁-C₆alkyl)₃, -(CH₂)-Si(C₁-C₆alkyl)₃, -Si(OC₁-C₆alkyl)₃, -N(R₉)₂, -(CH₂)-N(R₉)₂, wherein the two substituents R₉ are independent of each other, -C(=X)-R₇, -(CH₂)-C(=X)-R₇, -O-C(=X)-R₇, -(CH₂)-O-C(=X)-R₇, -S-C(=X)-R₇, -(CH₂)-S-C(=X)-R₇, -NR₉C(=X)R₇-(CH₂)-NR₉C(=X)R₇, -NR₉NHC(=X)-R₇, -NR₉-OR₁₀, -(CH₂)-NR₉-OR₁₀, -SR₉, -S(=O)R₁₁, -S(=O)_{2R11}, aryl, heterocyclyl, aryloxy or heterocyclyloxy; wherein the aryl, heterocyclyl, aryloxy and heterocyclyloxy radicals are unsubstituted or, depending upon the possibilities of substitution at the ring, mono- to penta-substituted by substituents selected from the group consisting of OH, halogen, CN, NO₂, C₁-C₁₂alkyl, C₃-C₈cycloalkyl, C₁-C₁₂haloalkyl, C₁-C₁₂alkoxy, C₁-C₁₂haloalkoxy, C₁-C₁₂alkylthio, C₁-C₁₂haloalkylthio, C₁-C₆alkoxy-C₁-C₆alkyl, C₂-C₈alkenyl, C₂-C₈alkynyl, C₂-C₁₂haloalkenyl,C₂-C₁₂haloalkenyloxy,C₂-C₁₂haloalkynyl, C₃-C₁₂haloalkynyloxy and phenoxy;
or R₄ together with R₂ forms =O or =S;
or when p is 1, R₄ together with R₅ is a bond;
or, when p is 0, together with R₂ and R₆ is ≡N;
R₅ and R₆ independently of each other are H, C₁-C₁₂-alkyl, -N₃, CN, NO₂, OH, SH, halogen, halo-C₁-C₂alkyl, hydroxy-C₁-C₂alkyl, C₃-C₈cycloalkyl that is unsubstituted or substituted by from one to two methyl groups, C₃-C₈halocycloalkyl, C₁-C₁₂alkoxy, C₁-C₆alkoxy-C₁-C₆alkyl, C₁-C₆alkoxy-C₁-C₆alkoxy, C₁-C₆alkoxy-C₁-C₆alkoxy-C₁-C₆alkyl, C₃-C₈cycloalkoxy, C₁-C₁₂haloalkoxy, C₁-C₁₂haloalkylthio, C₂-C₈alkenyl, C₂-C₈alkynyl, C₂-C₁₂haloalkenyl, C₂-C₁₂haloalkenyloxy, C₂-C₁₂haloalkynyl, C₃-C₁₂haloalkynyloxy, -P(=O)(OC₁-C₆alkyl)₂, -CH₂-P(=O)(OC₁-C₆alkyl)₂, -Si(OC₁-C₆alkyl)₃, -N(R₉)₂; -O-N(R₉)₂, wherein the two substituents R₉ are independent of each other, -C(=X)-R₇, -CH=NOH, -CH=NOC₁-C₆alkyl, -O-C(=X)-R₇, -S-C(=X)-R₇, -NR₉C(=X)R₇, -NR₉NHC(=X)-R₇, -NR₉-OR₁₀, -SR₉, -S(=O)R₁₁, -S(=O)₂R₁₁, -CH₂-S(=O)₂R₁₁, aryl, aryloxy, benzyloxy, -NR₉-aryl, heterocyclyl, heterocyclyloxy, -NR₉-heterocyclyl, -CH₂-aryl, -CH₂-O-aryl, -CH₂-NR₉-aryl, -CH₂-NR₉-C₁-C₂alkyl, -CH₂-heterocyclyl, -CH₂-O-heterocyclyl and -CH₂-NR₉-heterocyclyl; wherein the aryl, aryloxy, benzyloxy, -NR₉-aryl, heterocyclyl, heterocyclyloxy and -NR₉-heterocyclyl radicals are unsubstituted or, depending upon the possibilities of substitution at the ring, mono- to penta-substituted by substituents selected from the group consisting of OH, =O, SH, =S, halogen, CN, NO₂, C₁-C₁₂alkyl, C₃-C₈cycloalkyl, C₁-C₁₂haloalkyl, C₁-C₁₂alkoxy, C₁-C₁₂haloalkoxy, C₁-C₁₂alkylthio, C₁-C₁₂haloalkylthio, C₁-C₆alkoxy-C₁-C₆alkyl, C₂-C₈alkenyl, C₂-C₈alkynyl, C₂-C₁₂haloalkenyl, C₂-C₁₂haloalkenyloxy, C₂-C₁₂haloalkynyl, C₃-C₁₂haloalkynyloxy, phenoxy, methylenedioxy, NH₂, NH(C₁-C₁₂alkyl), N(C₁-C₁₂alkyl)₂ and C₁-C₆alkylsulfinyl; or
R₅ and R₆ are, together with the carbon atom to which they are bound, a five- to seven-membered ring, which may be saturated or unsaturated, and which may contain one or two members selected from the group consisting of O, NR₈ and S; and which is optionally substituted with one to three substituents selected from C₁-C₁₂-alkyl, CN, NO₂, OH, halogen, halo-C₁-C₂alkyl, C₃-C₈cycloalkyl C₃-C₈halocycloalkyl, C₁-C₁₂alkoxy, C₁-C₆alkoxy-C₁-C₆alkyl, C₁-C₆alkoxy-C₁-C₆alkoxy-C₁-C₆alkyl, C₃-C₈cycloalkoxy, C₁-C₁₂haloalkoxy, C₁-C₁₂alkylthio, C₃-C₈cycloalkylthio, C₁-C₁₂haloalkylthio, C₂-C₁₂alkenyl, C₂-C₁₂haloalkenyl, C₂-C₁₂haloalkenyloxy, C₂-C₁₂alkynyl, C₂-C₁₂haloalkynyl and C₃-C₁₂haloalkynyloxy;
or when p is 1, R₅ together with R₄ is a bond;
or, when p is 0, R₆ together with R₂ and R₄ is ≡N;
R₇ is H, OH, C₁-C₁₂alkyl, C₁-C₁₂haloalkyl, C₂-C₁₂alkenyl, C₂-C₁₂alkynyl, C₂-C₁₂haloalkenyloxy, C₂-C₁₂haloalkynyl, C₃-C₁₂haloalkynyloxy, C₁-C₁₂alkoxy, C₁-C₁₂haloalkoxy, C₁-C₆-alkoxy-C₁C₆alkyl, C₁-C₆alkoxy-C₁-C₆alkoxy, C₂-C₈alkenyloxy, C₃-C₈alkinyloxy, -N(R_{B})₂ wherein the two R₈ are independent of each other, aryl, aryloxy, benzyloxy, heterocyclyl, heterocyclyloxy or heterocyclylmethoxy; and wherein the aryl, aryloxy, benzyloxy, heterocyclyl and heterocyclyloxy radicals are unsubstituted or, depending upon the possibilities of substitution at the ring, mono- to penta-substituted by substituents selected from the group consisting of halogen, CN, NO₂, C₁-C₁₂alkyl, C₃-C₈cycloalkyl, C₁-C₁₂haloalkyl, C₁-C₁₂alkoxy, C₁-C₁₂haloalkoxy, C₁-C₁₂alkylthio, C₁-C₁₂haloalkylthio, C₁-C₆alkoxy-C₁-C₆alkyl, C₂-C₈alkenyl, C₂-C₁₂haloalkenyl, C₂-C₁₂haloalkenyloxy, C₂-C₈alkynyl, C₂-C₁₂haloalkynyl and C₃-C₁₂haloalkynyloxy;
R₈ is H, C₁-C₆alkyl that is optionally substituted with one to five substituents selected from the group consisting of halogen, C₁-C₆alkoxy, C₁-C₆alkoxy-C₁-C₆alkoxy, C₂-C₁₂alkenyl, C₂-C₁₂haloalkenyl, C₂-C₁₂haloalkenyloxy, C₂-C₁₂alkynyl, C₂-C₁₂haloalkynyl, C₃-C₁₂haloalkynyloxy, hydroxy and cyano, C₃-C₈-cycloalkyl, aryl, benzyl or heteroaryl; wherein the aryl, benzyl and heteroaryl radicals are unsubstituted or, depending on the possibilities of substitution on the ring, mono- to trisubstituted by substituents selected from the group consisting of OH, halogen, CN, NO₂, C₁-C₁₂alkyl, C₁-C₁₂haloalkyl, C₁-C₁₂alkoxy, C₁-C₁₂haloalkoxy, C₁-C₁₂alkylthio, C₂-C₁₂alkenyl, C₂-C₁₂haloalkenyl, C₂-C₁₂haloalkenyloxy, C₂-C₁₂alkynyl, C₂-C₁₂haloalkynyl, C₃-C₁₂haloalkynyloxy and C₁-C₁₂haloalkylthio;
R₉ is H, C₁-C₆alkyl, C₁-C₆cycloalkyl, C₁-C₆alkoxy-C₁-C₆alkyl, C₁-C₆alkoxy-C₁-C₆alkoxy-C₁-C₆alkyl, C₂-C₁₂alkenyl, C₂-C₁₂alkynyl, benzyl, aryl or heteroaryl;
R₁₀ H, C₁-C₆alkyl that is optionally substituted with one to five substituents selected from the group consisting of halogen, C₁-C₆alkoxy, NO₂, hydroxy and cyano, C₁-C₁₂haloalkyl, C₂-C₁₂alkenyl, C₂-C₁₂haloalkynyl, C₂-C₁₂haloalkenyl, C₂-C₁₂alkynyl, C₃-C₈-cycloalkyl, aryl, benzyl or heteroaryl; wherein the aryl, benzyl and heteroaryl radicals are unsubstituted or, depending on the possibilities of substitution on the ring, mono- to trisubstituted by substituents selected from the group consisting of OH, halogen, CN, NO₂, C₁-C₁₂alkyl, C₁-C₁₂haloalkyl, C₁-C₁₂alkoxy, C₁-C₁₂haloalkoxy, C₁-C₁₂alkylthio, C₁-C₁₂haloalkylthio, C₂-C₁₂alkenyl, C₂-C₁₂haloalkenyl, C₂-C₁₂haloalkenyloxy, C₂-C₁₂alkynyl, C₃-C₁₂haloalkynyl and C₃-C₁₂haloalkynyloxy;
R₁₁ is H, C₁-C₆alkyl that is optionally substituted with one to five substituents selected from the group consisting of halogen, C₁-C₆alkoxy, hydroxy and cyano, -N(R₉)₂ wherein the two substituents R₉ are independent of each other, C₃-C₈cycloalkyl, C₃-C₈halocycloalkyl, C₂-C₁₂alkenyl, C₂-C₁₂haloalkenyl, C₂-C₁₂haloalkenyloxy, C₂-C₁₂alkynyl, C₃-C₁₂haloalkynyl, C₃-C₁₂haloalkynyloxy, aryl, benzyl or heteroaryl; wherein the aryl, benzyl and heteroaryl radicals are unsubstituted or, depending on the possibilities of substitution on the ring, mono- to trisubstituted by substituents selected from the group consisting of OH, halogen, CN, NO₂, C₁-C₁₂alkyl, C₁-C₁₂haloalkyl, C₁-C₁₂alkoxy, C₁-C₁₂haloalkoxy, C₁-C₁₂alkylthio, C₁-C₁₂haloalkylthio, C₂-C₁₂alkenyl, C₂-C₁₂haloalkenyl, C₂-C₁₂haloalkenyloxy, C₂-C₁₂alkynyl, C₂-C₁₂haloalkynyl and C₃-C₁₂haloalkynyloxy;
R₁₂ is H, C-1-C₆alkyl, C₁-C₆cycloalkyl, C₁-C₆alkoxy-C₁-C₆alkyl, C₁-C₆alkoxy-C₁-C₆alkoxy-C₁-C₆alkyl, C₂-C₁₂alkenyl, C₂-C₁₂alkynyl, -C(=O)C₁-C₆alkyl, -C(=O)OC₁-C₆alkyl, -SO₂C₁-C₆alkyl, benzyl, aryl, heteroaryl;
X is O or S;
or, if appropriate, an E/Z isomer, E/Z isomer mixture and/or tautomer thereof, in each case in free form or in salt form;
a process for preparing these compounds, their isomers and tautomers and the use of these compounds, their isomers and tautomers; pesticidal compositions whose active compound is selected from these compounds and their tautomers; intermediates for the preparation of the said compounds of the formula (I), methods for the preparation of the compounds of the formula (I), and a method for controlling pests using these compositions.

Hereinbefore and hereinafter, the configuration at the ε-position (4'- or 4"-position) may be (S) as well as (R).

The literature proposes certain macrolide compounds for controllina pests. For instance, EP1160252 describes avermectin derivatives having antiparasitic activity. However, the biological properties of these known compounds are not entirely satisfactory, and, as a consequence, there is still a need for providing further compounds having pesticidal properties, in particular for the control of insects and representatives of the order Acarina. According to the invention, this object is achieved by providing the present compounds of the formula (I).

The compounds claimed according to the invention are derivatives of Avermectin. Avermectins are known to the person skilled in the art. They are a group of structurally closely related pesticidally active compounds which are obtained by fermenting a strain of the microorganism Streptomyces avermititis. Derivatives of Avermectins can be obtained by conventional chemical syntheses.

The Avermectins which can be obtained from Streptomyces avermitilis are referred to as A1a, A1b, A2a, A2b, B1a, B1b, B2a and B2b. The compounds referred to as "A" and "B" have a methoxy radical and an OH group, respectively, in the 5-position. The "a" series and the "b" series are compounds in which the substituent R₁ (in position 25) is a sec-butyl radical and an isopropyl radical, respectively. The number 1 in the name of the compounds means that atoms 22 and 23 are linked by double bonds; the number 2 means that they are linked by a single bond and that the C atom 23 carries an OH group. The above nomenclature is adhered to in the description of the present invention to denote the specific structure type in the not naturally occurring Avermectin derivatives according to the invention which corresponds to the naturally occurring Avermectin. What is for instance claimed according to the invention are derivatives of compounds of the B1 series, in particular mixtures of derivatives of Avermectin B1, especially B1a and B1b, along with derivatives having a single bond between carbon atoms 22 and 23, and derivatives having other substituents in the 25-position, as well as the corresponding monosaccharides.

Some of the compounds of the formula (I) can be present as tautomers. Accordingly, hereinabove and hereinbelow, the compounds of the formula (I) are, if appropriate, also to be understood as including the corresponding tautomers, even if the latter are not specifically mentioned in each case.

The compounds of formula (I) and, where applicable, their tautomers can form salts, for example acid addition salts. These acid addition salts are formed, for example, with strong inorganic acids, such as mineral acids, for example sulfuric acid, a phosphoric acid or a hydrohalic acid, with strong organic carboxylic acids, such as unsubstituted or substituted, for example halo-substituted, C₁-C₄alkanecarboxlic acids, for example acetic acid, unsaturated or saturated dicarboxylic acids, for example oxalic acid, malonic acid, maleic acid, fumaric acid or phthalic acid, hydroxycarboxylic acids, for example ascorbic acid, lactic acid, malic acid, tartaric acid or citric acid, or benzoic acid, or with organic sulfonic acids, such as unsubstituted or substituted, for example halo-substituted, C₁-C₄alkane- or aryl-sulfonic acids, for example methane- or p-toluene-sulfonic acid. Compounds of formula (I) that have at least one acidic group can furthermore form salts with bases. Suitable salts with bases are, for example, metal salts, such as alkali metal salts or alkaline earth metal salts, for example sodium, potassium or magnesium salts, or salts with ammonia or with an organic amine, such as morpholine, piperidine, pyrrolidine, a mono-, di- or tri-lower alkylamine, for example ethylamine, diethylamine, triethylamine or dimethylpropylamine, or a mono-, di- or trihydroxy-lower alkylamine, for example mono-, di- or tri-ethanolamine. Corresponding internal salts may also be formed where appropriate. The free form is preferred. Among the salts of the compounds of formula (I), the agrochemically advantageous salts are preferred. Hereinbefore and hereinafter, any reference to the free compounds of formula (I) or their salts is to be understood as including, where appropriate, also the corresponding salts or the free compounds of formula (I), respectively. The same applies to tautomers of compounds of formula (I) and salts thereof.

Unless defined otherwise, the general terms used hereinabove and hereinbelow have the meanings given below.

Unless defined otherwise, carbon-containing groups contain in each case 1 up to and including 6, preferably 1 up to and including 4, in particular 1 or 2, carbon atoms.

Halogen- as a group per se and also as a structural element of other groups and compounds, such as haloalkyl, haloalkoxy and haloalkylthio - is fluorine, chlorine, bromine or iodine, in particular fluorine, chlorine or bromine, especially fluorine or chlorine.

Alkyl - as a group per se and also as a structural element of other groups and compounds, such as haloalkyl, alkoxy and alkylthio - is, in each case taking into account the number of carbon atoms contained in each case in the group or compound in question, either straight-chain, i.e. methyl, ethyl, propyl, butyl, pentyl, hexyl, heptyl or octyl, or branched, for example isopropyl, isobutyl, sec-butyl, tert-butyl, isopentyl, neopentyl or isohexyl.

Cycloalkyl - as a group per se and also as a structural element of other groups and compounds, such as, for example, of halocycloalkyl, cycloalkoxy and cycloalkylthio - is, in each case taking into account the number of carbon atoms contained in each case in the group or compound in question, cyclopropyl, cyclobutyl, cyclopentyl, cyclohexyl, cycloheptyl or cyclooctyl.

Alkenyl - as a group per se and also as a structural element of other groups and compounds - is, taking into account the number of carbon atoms and conjugated or isolated double bonds contained in the group, either straight-chain, for example vinyl, allyl, 2-butenyl, 3-pentenyl, 1-hexenyl, 1-heptenyl, 1,3-hexadienyl or 1,3-octadienyl, or branched, for example isopropenyl, isobutenyl, isoprenyl, tert-pentenyl, isohexenyl, isoheptenyl or isooctenyl. Preference is given to alkenyl groups having 3 to 12, in particular 3 to 6, especially 3 or 4, carbon atoms.

Alkynyl - as a group per se and also as a structural element of other groups and compounds - is, in each case taking into account the number of carbon atoms and conjugated or isolated double bonds contained in the group or compound in question, either straight-chain, for example ethynyl, propargyl, 2-butynyl, 3-pentynyl, 1-hexynyl, 1-heptynyl, 3-hexen-1-ynyl or 1,5-heptadien-3-ynyl, or branched, for example 3-methylbut-1-ynyl, 4-ethylpent-1-ynyl, 4-methylhex-2-ynyl or 2-methylhept-3-ynyl. Preference is given to groups -CH₂-C₂-C₁₁alkynyl, in particular -CH₂-C₂-C₅alkynyl, especially -CH₂-C₂-C₃alkynyl.

Halogen-substituted carbon-containing groups and compounds, such as, for example, halogen-substituted alkyl, alkenyl, alkynyl, cycloalkyl, alkoxy or alkylthio, can be partially halogenated or perhalogenated, where in the case of polyhalogenation the halogen substituents can be identical or different. Examples of haloalkyl - as a group per se and also as a structural element of other groups and compounds, such as haloalkoxy or haloalkylthio - are methyl which is mono- to trisubstituted by fluorine, chlorine and/or bromine, such as CHF₂ or CF₃; ethyl which is mono- to pentasubstituted by fluorine, chlorine and/or bromine, such as CH₂CF₃, CF₂CF₃, CF₂CCl₃, CF₂CHCl₂, CF₂CHF₂, CF₂CFCl₂, CF₂CHBr₂, CF₂CHClF, CF₂CHBrF or CClFCHClF; propyl or isopropyl which is mono- to heptasubstituted by fluorine, chlorine and/or bromine, such as CH₂CHBrCH₂Br, CF₂CHFCF₃, CH₂CF₂CF₃, CF(CF₃)₂ or CH(CF₃)₂; butyl or one of its isomers, mono- to nonasubstituted by fluorine, chlorine and/or bromine, such as CF(CF₃)CHFCF₃ or CH₂(CF₂)₂CF₃; pentyl or one of its isomers, mono- to undecasubstituted by fluorine, chlorine and/or bromine, such as CF(CF₃)(CHF₂)CF₃ or CH₂(CF₂)₃CF₃; and hexyl or one of its isomers, mono- to tridecasubstituted by fluorine, chlorine and/or bromine, such as (CH₂)₄CHBrCH₂Br, CF₂(CHF)₄CF₃, CH₂(CF₂)₄CF₃ or C(CF₃)₂(CHF)₂CF₃.

Aryl is in particular phenyl, naphthyl, anthracenyl, phenanthrenyl, perylenyl or fluorenyl, preferably phenyl.

Heterocyclyl is understood as being a three- to seven-membered monocyclic ring, which may be saturated or unsaturated, and that contains from one to three hetero atoms selected from the group consisting of N, O and S, especially N and S; or a bicyclic ringsystem having from 8 to 14 ring atoms, which may be saturated or unsaturated, and that may contain either in only one ring or in both rings independently of one another, one or two hetero atoms selected from N, O and S; heterocyclyl is in particular piperidinyl, piperazinyl, oxiranyl, morpholinyl, thiomorpholinyl, pyridyl, N-oxidopyridinio, pyrimidyl, pyrazinyl, s-triazinyl, 1,2,4-triazinyl, thienyl, furanyl, dihydrofuranyl, tetrahydrofuranyl, pyranyl, tetrahydropyranyl, pyrrolyl, pyrrolinyl, pyrrolidinyl, pyrazolyl, imidazolyl, imidazolinyl, thiazolyl, isothiazolyl, triazolyl, oxazolyl, thiadiazolyl, thiazolinyl, thiazolidinyl, oxadiazolyl, dioxaborolanyl, phthalimidoyl, benzothienyl, quinolinyl, quinoxalinyl, benzofuranyl, benzimidazolyl, benzpyrrolyl, benzthiazolyl, indolinyl, isoindolinyl, cumarinyl, indazolyl, benzothiophenyl, benzofuranyl, pteridinyl or purinyl, which are preferably attached via a C atom; thienyl, benzofuranyl, benzothiazolyl, tetrahydropyranyl, dioxaborolanyl, or indolyl is preferred; in particular dioxaborolanyl, pyridyl or thiazolyl. The said heterocyclyl radicals may preferrably be unsubstituted or - depending on the substitution possibilities on the ring system - substituted by 1 to 3 substituents selected from the group consisting of halogen, =O, -OH, =S, SH, nitro, C₁-C₆alkyl, C₁-C₆hydroxyalkyl, C₁-C₆alkoxy, C₁-C₆haloalkyl, C₁-C₆haloalkoxy, phenyl and benzyl.

In the context of the present invention, preference is given to
(2) compounds according to group (1) of the formula (I) in which R₁ is isopropyl or sec-butyl, preferably to those in which a mixture of the isopropyl and the sec-butyl derivative is present;
(3) compounds according to group (1) or (2) of the formula (I), in which n is 0;
(4) compounds according to group (1) or (2) of the formula (I), in which n is 1;
(5) compounds according to group (1) or (2) of the formula (I), in which n is 2;
(6) compounds according to anyone of groups (1) to (5) of the formula (1), in which the substituent in the ε-position is -(CH₂)ₙ-CR₂(R₄)CR₃(R₅)(R₆);
(7) compounds according to anyone of groups (1) to (5) of the formula (I), in which p is 0;
(8) compounds according to anyone of groups (1) to (7) of the formula (I), in which the substituent in the ε-position is -(CH₂)ₙ-C(=O)-R₆ and R₆ is H, C₁-C₁₂-alkyl, OH, SH, halo-C₁-C₂alkyl, C₃-C₈cycloalkyl unsubstituted or substituted by from one to three methyl groups, C₃-C₈halocycloalkyl, C₁-C₁₂alkoxy, C₁-C₆alkoxy-C₁-C₆alkyl, C₁-C₆alkoxy-C₁-C₆alkoxy-C₁-C₆ₛalkyl, C₃-C₈cycloalkoxy, C₁-C₁₂haloalkoxy, C₁-C₁₂alkylthio, C₃-C₈cycloalkylthio, C₁-C₁₂haloalkylthio, C₂-C₈alkenyl, C₂-C₈alkynyl, -N(R₉)₂, wherein the two substituents R₉ are independent of each other, aryl, aryloxy, benzyloxy, -NR₉-aryl, heterocyclyl, heterocyclyloxy, -NR₉-heterocyclyl; or aryl, aryloxy, benzyloxy, -NR₉-aryl, heterocyclyl, heterocyclyloxy or -NR₉-heterocyclyl that, depending upon the possibilities of substitution at the ring, are mono- to penta-substituted by substituents selected from the group consisting of OH, =O, SH, =S, halogen, CN, NO_{2,} C₁-C₁₂alkyl, C₃-C₈cycloalkyl, C₁-C₁₂haloalkyl, C₁-C₁₂alkoxy, C₁-C₁₂haloalkoxy, C₁-C₁₂alkylthio, C₁-C₁₂haloalkylthio, C₁-C₆alkoxy-C₁-C₆alkyl, C₂-C₈alkenyl, C₂-C₈-alkynyl, phenoxy, methylenedioxy, NH₂, NH(C₁-C₁₂alkyl), N(C₁-C₁₂alkyl)₂ and C₁-C₆alkylsulfinyl;
(9) compounds according to anyone of groups (1) to (7) of the formula (I), in which the substituent in the ε-position is -(CH₂)ₙ-C(R₄)=CR₅(R₆);
(10) compounds according to anyone of groups (1) to (5) and (7) to (8) of the formula (I), in which R₃ is H or C₁-C₁₂-alkyl;
(11) compounds according to anyone of groups (1) to (6) of the formula (I), in which the substituent in the ε-position is -(CH₂)ₙ-C≡CR₆;
(12) compounds according to anyone of groups (1) to (7) of the formula (I), in which R₂ is is H, C₁-C₁₂-alkyl, C₁-C₁₂-hydroxyalkyl, -COOH or -COO-C₁-C₁₂-alkyl;
(13) compounds according to anyone of groups (1) to (7) and (9) to (12) of the formula (I), in which R₄ is H, C₁-C₁₂-alkyl, C₁-C₁₂-haloalkyl, halogen, NO₂, CN or C₃-C₈cycloalkyl;
(14) compounds according to anyone of groups (1) to (7) and (9) to (13) of the formula (I), in which R₅ together with R₄ is a bond;
(15) compounds according to anyone of groups (1) to (7) and (9) to (14) of the formula (I), in which R₅ is C₁-C₁₂-alkyl, CN, NO₂, OH, halogen, halo-C₁-C₂alkyl, C₃-C₈cycloalkyl C₁-C₁₂alkoxy, C₁-C₁₂haloalkoxy, C₂-C₈alkenyl, C₂-C₈alkynyl, -P(=O)(OC₁-C₆alkyl)₂, -CH₂-P(=O)(OC₁-C₆alkyl)₂, -Si(OC₁-C₆alkyl)₃ or -N(R₉)₂;
(16) compounds according to anyone of groups (1) to (15) of the formula (I), in which R₆ independently of each other are C₁-C₁₂-alkyl, CN, NO₂, OH, halogen, halo-C₁-C₂alkyl, -P(=O)(OC₁-C₆alkyl)₂, -CH₂-P(=O)(OC₁-C₆alkyl)₂z, -Si(OC₁-C₆alkyl)₃, -N(R₉)₂, wherein the two substituents R₉ are independent of each other, -C(=X)-R₇, -NR₉C(=X)R₇, -NR₉NHC(=X)-R₇, -NR₉-OR₁₀, -S(=O)R₁₁, -S(=O)₂R₁₁, aryl, aryloxy, benzyloxy, -NR₉-aryl, heterocyclyl, heterocyclyloxy, -NR₉-heterocyclyl; and aryl, aryloxy, benzyloxy, -NR₉-aryl, heterocyclyl, heterocyclyloxy and -NR₉-heterocyclyl that, depending upon the possibilities of substitution at the ring, are mono- to penta-substituted by substituents selected from the group consisting of OH, halogen, CN, NO₂, C₁-C₁₂alkyl, C₃-C₈-cycloalkyl, C₁-C₁₂haloalkyl, C₁-C₁₂alkoxy, C₁-C₁₂haloalkoxy, C₁-C₁₂alkylthio, C₁-C₁₂haloalkylthio, C₁-C₆alkoxy-C₁-C₆alkyl, C₂-C₆alkenyl, C₂-C₈alkynyl, phenoxy, methylenedioxy, NH₂, NH(C₁-C₁₂alkyl), N(C₁-C₁₂alkyl)₂ and C₁-C₆alkylsulfinyl;
(17)compounds according to anyone of groups (1) to (16) of the formula (I), in which the bond between atoms C₂₂ and C₂₃ is a single bond;
(18) compounds according to anyone of groups (1) to (16) of the formula (I), in which atoms C₂₂ and C₂₃ is a double bond;
(19) compounds according to anyone of groups (3) to (18) of the formula (I), wherein R₁ is cyclohexyl;
(20) compounds according to anyone of groups (3) to (18) of the formula (I), wherein R₁ is 1-methyl-butyl;
(21) compounds according to one of the groups (1) to (20) of the formula (I) in which the configuration at the ε-position is (R);
(22) compounds according to one of the groups (1) to (20) of the formula (I) in which the configuration at the ε-position is (S);
(23) compounds according to one of the groups (1) to (7) and (19) to (22) wherein the substituent in the ε-position is -C(=O)R₆;
(24) compounds according to one of the groups (1) to (7) and (19) to(23) wherein the substituent in the ε-position is -CH₂C(=O)R₆; wherein R₆ is C₁-C₁₂-alkyl, -N₃, CN, SH, halogen, halo-C₁-C₂alkyl, C₃-C₈cycloalkyl unsubstituted or substituted by from one to two methyl groups, C₃-C₈halocycloalkyl, C₁-C₁₂alkoxy, C₁-C₆alkoxy-C₁-C₆alkyl, C₁-C₆alkoxy-C₁-C₆alkoxy, C₁-C₆alkoxy-C₁-C₆alkoxy-C₁-C₆alkyl, C₃-C₈cycloalkoxy, C₁-C₁₂haloalkoxy, C₁-₁₂haloalkylthio, C₂-C₈alkenyl, C₂-C₈alkynyl, C₂-C₁₂haloalkenyl, C₂-C₁₂haloalkenyloxy, C₂-C₁₂haloalkynyl or C₃-C₁₂haloalkynyloxy;
(25) compounds according to one of the groups (1) to (7) and(19) to (23)wherein the substituent in the ε-position is -CH₂R₆ or -CH₂CH₂R₆ or -CH₂CH₂CH₂R₆; and R₆ is OH, C₁-C₆alkyl, C₁-C₁₂alkoxy, C₁-C₆alkoxy-C₁-C₆alkyl, C₁-C₆alkoxy-C₁-C₆alkoxy, C₁-C₆alkoxy-C₁-C₆alkoxy-C₁-C₆alkyl, C₃-C₈cycloalkoxy, C₁-C₁₂haloalkoxy, C₁-C₁₂haloalkylthio, C₂-C₁₂haloalkenyloxy, C₃-C₁₂haloalkynyloxy, -N(R₉)₂, -O-N(R₉)₂, wherein the two substituents R₉ are independent of each other, -O-C(=X)-R₇, -S-C(=X)-R₇, -NR₈C(=X)R₇, -NR₉NHC(=X)-R₇, -NR₉-OR₁₀, -SR₉, -S(=O)R₁₁, -S(=O)₂R₁₁, aryl, aryloxy, benzyloxy, -NR₉-aryl, heterocyclyl or heterocyclyloxy.

In the context of the invention, particular preference is given to the compounds of the formula (I) listed in the tables and, if appropriate, to their E/Z isomers and E/Z isomer mixtures.

The invention also provides a process for preparing the compounds of the formula (I) and, if appropriate, tautomers thereof, wherein R₁ to R₆ have the same meanings as given above under (1) for formula (I), and m is 0, n is 1 and p is 0 or 1, characterized in that
(A) a compound of the formula 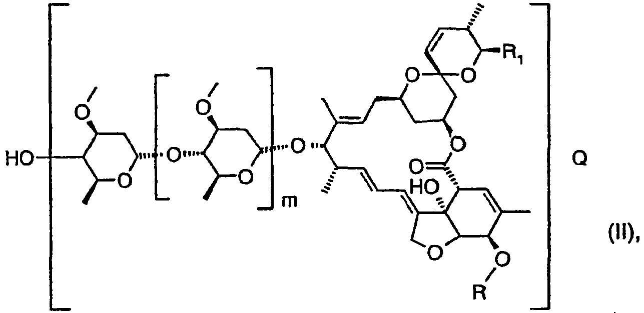 and which is known or can be prepared according to known procedures, wherin R₁ and m have the meanings as given in formula (I) and R is a protecting group, is converted into a compound of the formula 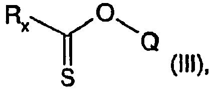 wherein Rₓ is Imidazolyl, aryloxy, thioalkyl or thioaryl and Q has the same meaning as the part of the formula (II) which is in the bracket marked with Q;
(B) the said compound of the formula (III) is converted into a compound of the formula 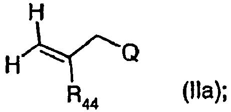 wherein R₄₄ is H, C₁-C₆alkyl, -Si(C₁-C₆alkyl)₃, -(CH₂)-Si(C₁-C₆alkyl)₃, -Sn(C₁-C₆alkyl)₃, -C(=O)R₇, R₇ is as defined under formula (I), and Q has the meaning as given above in formula (II);
(C) the said compound of formula (IIa) may - provided R₄₄ is H - further be converted into a compound of the formula 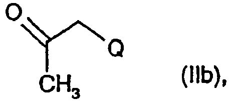 wherein Q has the meanings as defined above in formula (II); or
(D) the compound of the formula (IIa) is converted into a compound of the formula 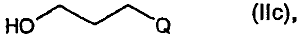 wherein Q has the meanings as defined above in formula (II); or
(E) the compound of the formula (IIa) as defined above is converted into a compound of the formula 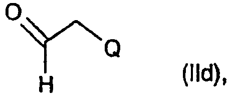 wherein Q has the meaning as defined above in formula (II).
   The invention further provides a process for preparing the compounds of the formula (I) and, if appropriate, tautomers thereof, wherein R₁ to R₆ have the same meanings as given above under (1) for formula (I), and m is 0, n is 0 and p is 0 or 1, characterized in that
(F) a compound of the above formula (II) is oxidized to a compound of the formula 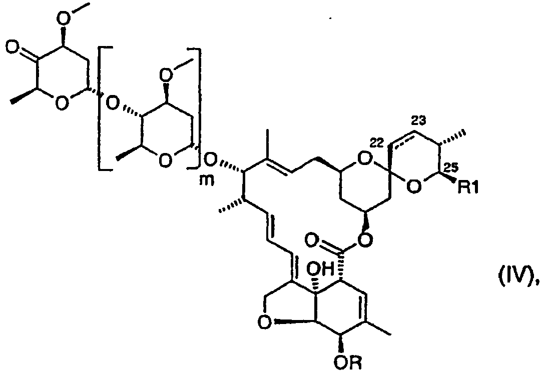 which are partly known or can be prepared according to methods known per se, and wherein R₁ and m are as defined for formula (I) and R is a protecting group; then
(G) converting the above compound of the formula (IV) into a compound of the formula 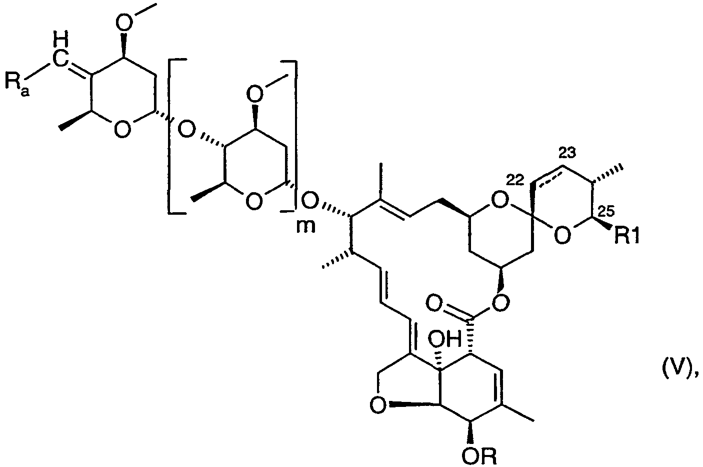 which are partly known or can be prepared according to methods known per se, wherein m and R₁ have the meanings as defined above, Ra is H, CN or -C(=O)R₅₅, R₅₅ is C₁-C₁₂alkoxy or C₂-C₁₂alkenyloxy and R is a protecting group, and which is known;
(H1) further converting the compound of the formula (V) wherein Ra is H, preferably via hydroboration and oxidative work-up, into a compound of the formula 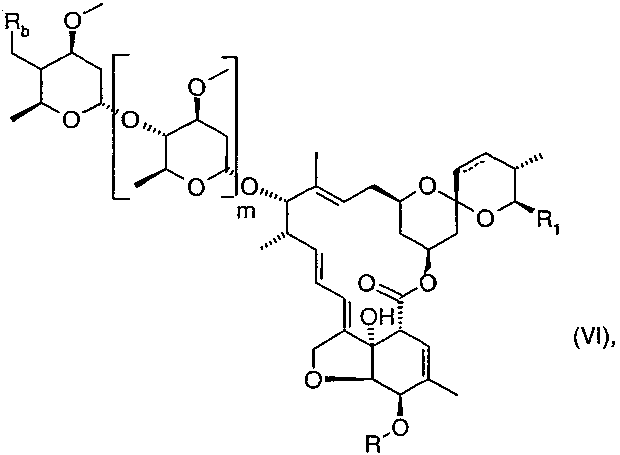 wherein m and R₁ have the meanings as defined above for formula and R_{b} is OH; or
(H2) converting a compound of the formula (V) wherein Rₐ is -C(=O)R₅₅, R₅₅ is C₁-C₁₂alkoxy or C₂-C₁₂alkenyloxy into a compound of the formula (VI), wherein R_{b} is -C(=O)R₅₅, R₅₅ is C₁-C₁₂alkoxy or C₂-C₁₂alkenyloxy; or
(H3) converting the compound of the formula (V) wherein Rₐ is CN into a compound of the formula (V), wherein R_{b} is -NCO; and
(H4) further converting the said compound of the formula (V) wherein Rₐ is -NCO into a compound of the formula (I), wherein n and p are 0, R₆ is H and R₂ and R₄ together are =O.
   The invention further provides a process for preparing the compounds of the formula (I) and, if appropriate, tautomers thereof, wherein R₁ to R₆ have the same meanings as given above under (1) for formula (I), and m is 0, n is 0 and p 0 or 1, characterized in that
(J) a compound of the formula (II) as defined above is converted into a compound of the formula 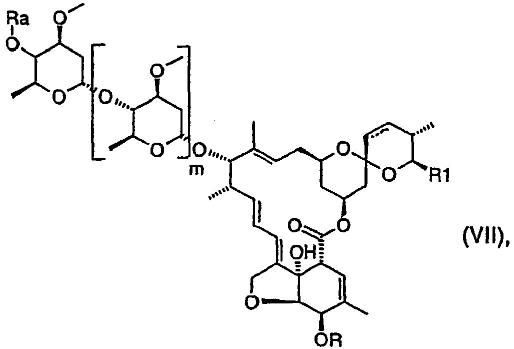 wherein m and R₁ have the meanings as defined above, R is a protecting group and Rₐ is an activating group such as an optionally substituted alkyl- or arylsulphonate;
(K) further converting the said compound of the formula (VII) into a compound of the formula 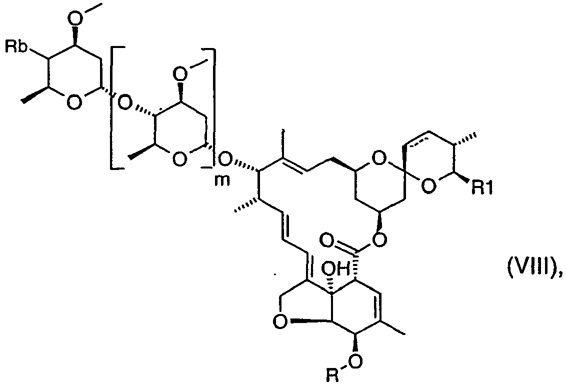 wherein R is a protecting group, m and R₁ have the meanings as defined above for formula (VIII), and R_{b} is either halogen or CN; and
(L1) further converting the compound of the formula (VIII) wherein R_{b} is iodine, into a compound of the formula 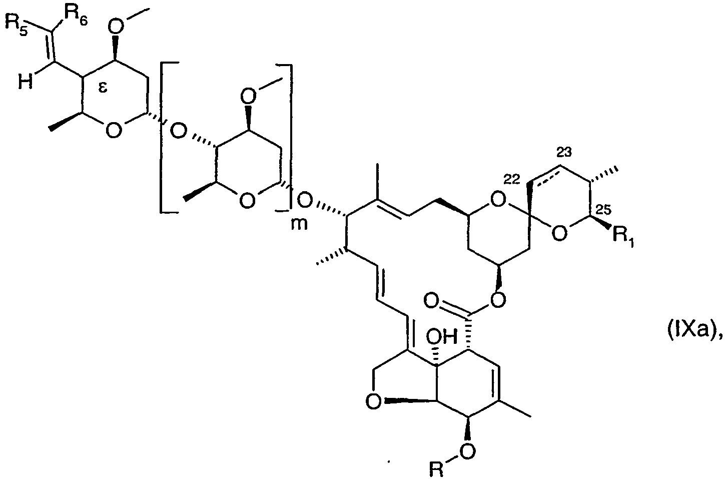 wherein R, m, R₁, R₅ and R₆ have the meanings as defined above for formula (I); or into a compound of formula (IIa) as defined before; or alternatively
(L2) converting a compound of the formula (IIa) wherein R₄₄ is H into a compound of the formula 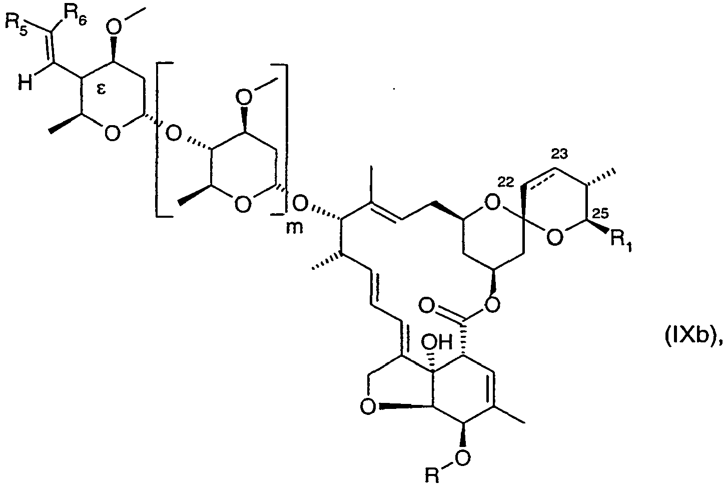 wherein R, m, R₁ have the meanings as defined above for formula (I) R₅ is CH₃ and R₆ is H; or
(M) converting the compound of the formula (VIII) wherein R_{b} is iodine, into acompound of the formula 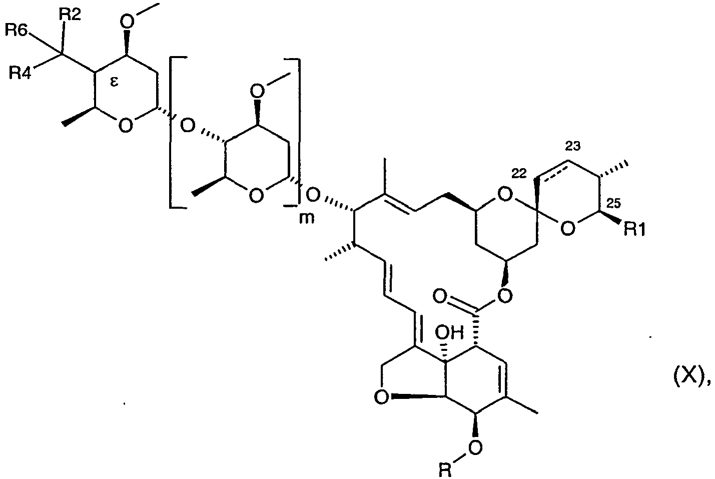 wherein R, m, R₁, R₂, R₄ and R₆ have the meanings as defined above for formula (I).
(N) Compounds of formula (I) can be obtained from compounds of formulae (IIa), (IIb), (IIc), (IId), (VI), (VIII), (IX) and (X) as defined above by removing the protecting group at the 5-position of the forementioned intermediates.
O) Compounds of the formula (IX) wherein R₅ and R₆ are H, may be used to obtain other compounds of the formula (IX), and by deprotection on the 5-position also compounds of the formula 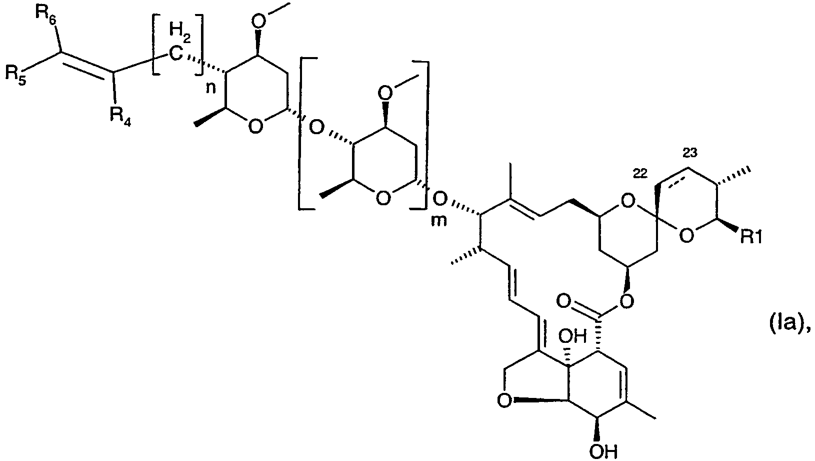
wherein m, n, R₁ and R₄ have the meanings as defined above for formula (I), and wherein at least one of the substituents R₅ or R₆ is not H.

The compounds of the formulae (I) and (II) can further be converted into other compounds of the formula (I) by methods known per se, for instance for preparing a compound of the formula (I), wherein R₂ and R₃ are a bond, by reacting compounds of the formulae (IIb) or (IId) with a compound of the formula R₅-CH₂-R₆, wherein R₅ and R₆ have the meaning as defined under (1); or by reacting a compound of the formulae (IIa), (V) or (XI) with a compound of the formula R₅R₆C=CH₂.

Furthermore compounds of formula (IIa), (IIb), (IIc), (IId), (VI), (VIII), (IX) and (X) bearing a functional group in its free or protected form can be used as starting materials for the preparation of further compounds of formula (I). For example, a compound of formula (IId) can be converted by deprotection into a compound of formula (I) wherein n = 1, R₂ and R₄ together are oxygen, p = 0 and R₆ is OH.

Furthermore compounds of formula (IIa), (IIb), (IIc), (IId), (VI), (VIII), (IX) and (X) bearing a substituent at the ε-position (4'- or 4"-position) in its free or protected form can be used as starting materials for the preparation of further compounds of formula (I). For example, a compound of formula (IId) can be converted by deprotection into a compound of formula (I) wherein n = 1, R₂ and R₄ together are oxygen, p = 0 and R₆ is OH.

The compound of the formula (VI) can further be derivatized by known procedures, for case by reacting the compound with a compound Hal-C(=X)-R₇, wherein R₇ is as defined under (1) and Hal is a halogen.

The comments made above in connection with tautomers of compounds of formula (I) apply analogously to the starting materials mentioned hereinabove and hereinbelow in respect of their tautomers and diasteromers.

The reactions described hereinabove and hereinbelow are carried out in a manner known per se, for example in the absence or, customarily, in the presence of a suitable solvent or diluent or of a mixture thereof, the reactions being carried out, as required, with cooling, at room temperature or with heating, for example in a temperature range of approximately from -80°C to the boiling temperature of the reaction medium, preferably from approximately 0°C to approximately +150°C, and, if necessary, in a closed vessel, under pressure, under an inert gas atmosphere and/or under anhydrous conditions. Especially advantageous reaction conditions can be found in the Examples.

The reaction time is not critical; a reaction time of from about 0.1 to about 24 hours, especially from about 0.5 to about 10 hours, is preferred.

The product is isolated by customary methods, for example by means of filtration, crystallisation, distillation or chromatography, or any suitable combination of such methods.

Protecting groups are as defined for instance in the compounds of formulae (II), (IV), (V), (VII) and (VIII) include: alkyl ether radicals, such as methoxymethyl, methylthiomethyl, tert-butylthiomethyl, benzyloxymethyl, p-methoxybenzyl, 2-methoxyethoxymethyl, 2,2,2-trichloroethoxymethyl, 2-(trimethylsilyl)ethoxymethyl, tetrahydropyranyl, tetrahydrofuranyl, 1-ethoxyethyl, 1-(2-chloroethoxy)ethyl, 1-methyl-1-methoxyethyl, 1-methyl-1-benzyloxyethyl, trichloroethyl, 2-trimethylsilylethyl, tert-butyl, allyl, p-methoxyphenyl, 2,4-dinitrophenyl, benzyl, p-methoxybenzyl, o-nitrobenzyl, p-nitrobenzyl, triphenylmethyl; trialkylsilyl radicals, such as trimethylsilyl, triethylsilyl, dimethyl-tert-butylsilyl, dimethyl-isopropylsilyl, dimethyl-1,1,2-trimethylpropylsilyl, diethyl-isopropylsilyl, dimethyl-tert-hexylsilyl, but also phenyl-tert-alkylsilyl groups, such as diphenyl-tert-butylsilyl; esters, such as formates, acetates, chloroacetates, dichloroacetates, trichloroacetates, trifluoroacetates, methoxyacetates, phenoxyacetates, pivaloates, benzoates; alkyl carbonates, such as methyl-, 9-fluorenylmethyl-, ethyl-, 2,2,2-trichloroethyl-, 2-(trimethylsilyl)ethyl-, vinyl-, allyl-, benzyl-, p-methoxybenzyl-, o-nitrobenzyl-, p-nitrobenzyl-, but also p-nitrophenyl-carbonate.

Preference is given to trialkylsilyl radicals, such as trimethylsilyl, triethylsilyl, dimethyl-tert-butylsilyl, diphenyl-tert-butylsilyl, esters, such as methoxyacetates and phenoxyacetates, and carbonates, such as 9-fluorenylmethylcarbonates and allylcarbonates. Dimethyl-tert-butylsilyl ether is especially preferred.

The starting materials mentioned hereinabove and hereinbelow that are used for the preparation of the compounds of formula (I) and, where applicable, their tautomers are known or can be prepared by methods known *per se,* e.g. as indicated below.

The compounds of formulae (II), (III), (IV), (V), (VI), (VII) and (VIII) are partly new, in which case they are also an aspect of the invention. They are valuable intermediates for the synthesis of compounds of formula (I), and can prepared by methods known *per se*. The use of compounds of formula (II) and of formula (III) for the synthesis of compounds of formula (I) are also a subject of this invention. The preferences for the substituents are the same as defined for the compound of the formula (I) in (2) to (20).

### Process variant (A):

Examples of solvents and diluents include: aromatic, aliphatic and alicyclic hydrocarbons and halogenated hydrocarbons, such as benzene, toluene, xylene, mesitylene, tetralin, chlorobenzene, dichlorobenzene, bromobenzene, petroleum ether, hexane, cyclohexane, dichloromethane, trichloromethane, tetrachloromethane, dichloroethane, trichloroethene or tetrachloroethene; ethers, such as diethyl ether, dipropyl ether, diisopropyl ether, dibutyl ether, tert-butyl methyl ether, ethylene glycol monomethyl ether, ethylene glycol monoethyl ether, ethylene glycol dimethyl ether, dimethoxydiethyl ether, tetrahydrofuran or dioxane; esters of carboxylic acids, such as ethyl acetate; amides, such as dimethylformamide, dimethylacetamide or 1-methyl-2-pyrrolidinones; nitriles, such as acetonitrile; sulfoxides, such as dimethyl sulfoxide; or mixtures of the mentioned solvents. Preference is given to amides, such as dimethylformamide and dimethylacetamide, especially dimethylacetamide, halogenated hydrocarbons, such as dichloromethane and aromatic hydrocarbons such as toluene.

The reactions are advantageously carried out in a temperature range of from approximately -70°C to +80°C, preferably at from 0°C to +60°C.

Examples of thiocarbonylating agents are known to a person skilled in the art; they include 1,1'-thiocarbonyldiimidazole and arylchlorothionoformates; especially suitable are 1,1'-thiocarbonyldiimidazole and *p*-tolyl chlorothionoformate.

In a preferred embodiment of Variant (A) the reaction is carried out with p-tolyl chlorothionoformate in the presence, or absence, of 4-(dimethylamino)pyridine at room temperature in dichloromethane or with 1,1'-thiocarbonyldiimidazole at 20-60°C in N,N-dimethylformamide.

Especially preferred conditions for the reaction are described in Examples P1 (Step A) and P13 (Step A).

### Process variant (B):

Examples of solvents and diluents include: aromatic, aliphatic and alicyclic hydrocarbons and halogenated hydrocarbons, such as benzene, toluene, xylene, mesitylene, tetralin, chlorobenzene, dichlorobenzene, petroleum ether, hexane, and cyclohexane; alcohols such as diisopropyl alcohol and *tert*-butanol; or mixtures of the mentioned solvents. Preference is given to aromatic hydrocarbons, such as benzene, and toluene.

The reactions are advantageously carried out in a temperature range from approximately -70°C to to the boiling point of the solvent used, preferably from +60°C to +120°C.

Examples of allylating agents are known to a person skilled in the art; they include optionally substituted allylic stannanes, for example, allyl-tri-*n*-butyltin, and sulphones. Especially suitable substituted allylic stannanes are stannanes with carboxyalkyl, alkyl, trialkylsilyl, tri-arylsilyl, CH₂(tri-alkylsilyl) and CH₂(tri-arylsilyl) substitutents at C2.

The reaction is carried out in the presence of radical initiators known to a person skilled in the art; they include 1,1'-azobis(cyclohexanecarbonitrile) and 2,2'-azobis-(2-methylbutyronitrile).

In a preferred embodiment of Variant (B) the allylation reaction is carried out in the presence of 1,1'-azobis(cyclohexanecarbonitrile) at +60 to +110 °C in chlorobenzene as the solvent.

Especially preferred conditions for the reaction are described in Examples P1 (Step B) and P13 (Step B).

### Process variant (C):

Examples of solvents and diluents include: aromatic, aliphatic and alicyclic hydrocarbons and hydrocarbons, such as benzene, toluene, xylene, mesitylene, Tetralin; alcohols, such as methanol, ethanol, propanol, isopropanol, butanol, ethylene glycol or glycerol; amides, such as N,N-dimethylformamide, N,N-diethylformamide, N,N-dimethylacetamide, N-methylpyrrolidone or hexamethylphosphoric acid triamide; nitriles, such as acetonitrile or propionitrile; and sulfoxides, such as dimethyl sulfoxide; and also water; or mixtures of the mentioned solvents; especially suitable are amides, alcohols, water, or mixtures thereof, more especially N,N-dimethylformamide, N,N-dimethylacetamide or water.

The reactions are advantageously carried out in a temperature range from room temperature to +60°C; preferance being given to reaction from +10 to +30°C.

In a preferred embodiment of Variant (C) the oxidation is carried out in the presence of palladium dichloride, copper (II) acetate and oxygen room temperature in N,N-dimethylacetamide and water as the solvent.

Especially preferred conditions for the reaction are described in Examples P8 and P14 (Step A).

### Process variant (D):

Examples of solvents and diluents include: aromatic, aliphatic and alicyclic hydrocarbons and halogenated hydrocarbons, such as benzene, toluene, xylene, mesitylene, Tetralin, chlorobenzene, dichlorobenzene, bromobenzene, petroleum ether, hexane, cyclohexane, dichloromethane, tetrachloromethane, dichloroethane, trichloroethene or tetrachloroethene; ethers, such as diethyl ether, dipropyl ether, diisopropyl ether, dibutyl ether, tert-butyl methyl ether, ethylene glycol monomethyl ether, ethylene glycol monoethyl ether, ethylene glycol dimethyl ether, dimethoxydiethyl ether, tetrahydrofuran or dioxane; or mixtures of the mentioned solvents; especially suitable are tetrahydrofuran, diethyl ether or dichlormethane; or the use of no solvent.

The hydroboration may be carried out in the presence, or absence, of transition-metal catalysts. Hydroborating agents include boron hydride reagents; especially suitable are borane-methyl sulphide complex, borane-tetrahydrofuran complex, dicyclohexylborane, 9-borabicyclo[3.3.1]nonane and sodium malonyloxyborohydride.

In a preferred embodiment of Variant D, the hydroboration is carried out with 9-borabicyclo[3.3.1]nonane or borane-tetrahydrofuran complex at room temperature, in tetrahydrofuran.

The alcohol products are obtained by standard oxidation of the organoboranes. Examples of standard oxidizing regents are known to a person skilled in the art; they include base/hydrogen peroxide mixtures and sodium perborate.

The reactions are advantageously carried out in a temperature range from 0 to +60°C; preferance being given to reaction from 10 to 30°C.

Especially preferred conditions for the reaction are described in Example P5 (Step A).

### Process variant (E):

Examples of solvents and diluents include: aromatic, aliphatic and alicyclic hydrocarbons and halogenated hydrocarbons, such as benzene, toluene, xylene, mesitylene, Tetralin, chlorobenzene, dichlorobenzene, bromobenzene, petroleum ether, hexane, cyclohexane, dichloromethane, trichloromethane, tetrachloromethane, dichloroethane, trichloroethene or tetrachloroethene; ethers, such as diethyl ether, dipropyl ether, diisopropyl ether, dibutyl ether, tert-butyl methyl ether, ethylene glycol monomethyl ether, ethylene glycol monoethyl ether, ethylene glycol dimethyl ether, dimethoxydiethyl ether, tetrahydrofuran or dioxane; alcohols, such as methanol, ethanol, propanol, isopropanol, butanol, ethylene glycol or glycerol; nitriles, such as acetonitrile or propionitrile; ketones, such as acetone and also water; or mixtures of the mentioned solvents; especially suitable are ethers, alcohols, water, or mixtures thereof, more especially tetrahydrofuran, tert-butanol or water.

Examples of agents for oxidatively cleaving the terminal alkene function are known to a person skilled in the art; they include for example reagent combinations of OsO₄, N-methylmorpholine-N-oxide and sodium periodate.

The reactions are advantageously carried out in a temperature range from room temperature to +60°C; preferance being given to reaction from +10 to +30°C.

Especially preferred conditions for the reaction are described in Example P3 (Step A).

### Process variant (F):

Examples of solvents include those listed above under process variant (A).

The reactions are advantageously carried out in a temperature range from approximately -70°C to +80°C.

Examples of oxidising agents are known to a person skilled in the art, for instance using oxalyl chloride or trifluoroacetic anhydride and dimethylsulfoxide or 1,1,1-tris(acetyl-oxy)-1,1-dihydro-1,2-benziodoxo)-3-(1H)-one.

### Process variant (G):

Examples of solvents include those listed above under process variant (A), especially suitable are aromatic, aliphatic and alicyclic hydrocarbons, ethers, and sulphoxides or mixtures thereof, more especially toluene, tetrahydrofuran, diethyl ether and dimethyl sulphoxide.

The reactions are advantageously carried out in a temperature range from approximately -78°C to +80°C, preferably from -40 to +60 °C.

Examples of methylenating agents for preparing compounds of the formula (V), wherein Ra is hydrogen are known to a person skilled in the art, for instance dimethyl titanocene or a reagent prepared from Zn, dihalomethane and TiCl₄, or methylene triphenylphosphorane. In a preferred embodiment for preparing compounds of the formula (V), wherein Ra is hydrogen the reaction is carried out in the presence of zinc, diiodomethane and a Lewis acid, such as titanium tetrachloride, in dichloromethane and tetrahydrofuran as the solvent at 0 to +30 °C.

For the preparation of compounds of the formula (V), wherein Ra is -CN or -C(=O)R₅₅ and R₅₅ is C₁-C₁₂alkoxy or C₂-C₁₂alkenyloxy, for instance phosphonium salts in the presence of a base are being used.

Especially preferred conditions for this process are described, for example, in Example P.10 (step A).

### Process variant (H1):

Examples of solvents and diluents include: aromatic, aliphatic and alicyclic hydrocarbons and halogenated hydrocarbons, such as benzene, toluene, xylene, mesitylene, Tetralin, chlorobenzene, dichlorobenzene, bromobenzene, petroleum ether, hexane, cyclohexane, dichloromethane, tetrachloromethane, dichloroethane, trichloroethene or tetrachloroethene; ethers, such as diethyl ether, dipropyl ether, diisopropyl ether, dibutyl ether, tert-butyl methyl ether, ethylene glycol monomethyl ether, ethylene glycol monoethyl ether, ethylene glycol dimethyl ether, dimethoxydiethyl ether, tetrahydrofuran or dioxane; or mixtures of the mentioned solvents; especially suitable are tetrahydrofuran, diethyl ether or dichlormethane; or the use of no solvent.

Examples of hydroboration-oxidation agents are known to a person skilled in the art. The hydroboration may be carried out in the presence, or absence, of transition-metal catalysts as known to those persons skilled in the art. Hydroborating agents include boron hydride reagents; especially suitable are borane-methyl sulphide complex and borane-tetrahydrofuran complex.

In a preferred embodiment of variant (H), the hydroboration is carried out with borane-tetrahydrofuran complex at room temperature, in tetrahydrofuran as the solvent.

The alcohol products are obtained by standard oxidation of the organoboranes. Examples of standard oxidizing reagents are known to a person skilled in the art; they include base/hydrogen peroxide mixtures and sodium perborate.

The reactions are advantageously carried out in a temperature range from 0 to +60°C; preference being given to reaction from +10 to +30°C.

Especially preferred conditions for the reaction are described in Example P.1 0 (step B).

### Process variant (H2):

Examples of solvents and diluents are the same as those mentioned under Process variant A. In addition, alcohols, such as methanol, ethanol or 2-propanol, and water are suitable.

The reactions are advantageously carried out in a temperature range of approximately from -70°C to 100°C, preferably at from -10°C to 25°C.

Examples of reducing agents are known to a person skilled in the art, for instance hydrogen donors in the presence of a catalyst or metal borohydride or borides.

In a preferred embodiment of Variant (Ac) the reaction is carried out in the presence of sodium borohydride and NiCl₂, in ethyl acetate and methanol as the solvent at 0°C to +30 °C.

### Process variant (H3):

Examples of solvents and diluents are the same as those mentioned under Process variant A.

The reactions are advantageously carried out in a temperature range of approximately from -70°C to 100°C, preferably at from -10°C to 25°C.

Examples of oxidising agents are known to a person skilled in the art, for instance peroxyacids, lead tetraacetate, DMSO/Br₂ or benzonitrile oxides.

In a preferred embodiment of Variant (Hb) the reaction is carried out in the presence of lead tetraacetate, in benzene as the solvent at 0°C to +30 °C.

### Process variant (H4):

Examples of solvents and diluents are the same as those mentioned under Process variant A.

The reactions are advantageously carried out in a temperature range of approximately from -70°C to 100°C, preferably at from -10°C to 25°C.

Hydrolysis is perfomed in the presence of agents like acids or bases, alumine or silica-gel.

In a preferred embodiment of Variant (H4) the reaction is carried out in the presence of neutral alumine, in dichloromethane as the solvent, at 0°C to +30 °C.

### Process variant (J):

Examples of solvents and diluents include those listed above under Process variant (A); especially suitable are halogenated hydrocarbons, for example, dichloromethane.

The reactions are advantageously carried out in a temperature range of from about -30 to +50°C; preference being given to reaction at -10°C to +30°C.

In a preferred embodiment of Variant (D) the reaction is carried out with trifluoromethanesulphonyl chloride in the presence of an organic base, such as triethyl amine or N,N-diisopropylethylamine and 4-(dimethylaminopyridine at 0 to +20°C in dichloromethane as the solvent.

Especially preferred conditions for this Process variant are described, for example, in Example P.2 (step A).

### Process variant (K):

Examples of solvents and diluents include those listed above under Process variant (A); especially suitable are amides, for example N,N-dimethylformamide.

The reactions are advantageously carried out in a temperature range of from about -10 °C to the boiling point of the solvent used; preference being given to reaction at 0°C to +30°C.

In a preferred embodiment of Variant (K) the reaction is carried out with tetra *n*-butyl ammonium cyanide, potassium iodide or sodium dimethylmalonate at 0 to +30°C in N,N-dimethylformamide as the solvent.

Especially preferred conditions for this Process variant are described, for example, in Example P.11 (step A).

### Process variant (L1):

Examples of solvents and diluents include those listed above under Process variant (A); especially suitable are aromatic hydrocarbons and halogenated aromatic hydrocarbons, for example, chlorobenzene.

The reactions are advantageously carried out in a temperature range of from about room temperature to the boiling point of the solvent used; preference being given to reaction at +60°C to +110°C.

Examples of vinylating agents are known to a person skilled in the art; they include optionally substituted vinylic stannanes and vinylic sulphones, for example, tri-*n*-butyl-((E/Z)-styryl)-stannane or 2,2-dichlorovinyl ethyl sulphone.

The reaction is carried out in the presence of radical initiators known to a person skilled in the art; they include 1,1'-azobis(cyclohexanecarbonitrile), 2,2'-azobis-(2-methylbutyronitrile) and di-*tert*-butylperoxide.

In a preferred embodiment of Variant (L1) the vinylation reaction is carried out in the presence of 1,1'-azobis(cyclohexanecarbonitrile) at +60 to +110°C in chlorobenzene as the solvent.

Especially preferred conditions for this Process variant are described, for example, in Example P.2 (step C).

### Process variant (L2):

Examples of solvents and diluents include those listed above under Process variant (A); especially suitable are halogenated solvents.

The reactions are advantageously carried out in a temperature range of from about room temperature to the boiling point of the solvent used; preference being given to reaction at +60°C to +110°C.

Examples of agents used for isomerisation are known to a person skilled in the art, for example transition metal catalysts in the presence or in the absence of a base.

In a preferred embodiment of Variant (La) the reaction is carried out in the presence of a Ruthenium catalyst (Grubb's catalyst), at +20+ to +50° in dichloromethane as the solvent, and in the presece of a silyl or alkyl enolether.

### Process variant (M):

Examples of solvents and diluents include those listed above under Process variant (A); especially suitable are amides, for example N,N-dimethylformamide.

The reactions are advantageously carried out in a temperature range of from about 0°C to the boiling point of the solvent used; preference being given to reaction at 0°C to +40°C.

Examples of alkylating agents are known to a person skilled in the art; they include optionally substituted activated methylene compounds, for example, malononitrile, ethylcyanoacetate and malonic acid dimethyl ester.

In a preferred embodiment of Variant (M) the reaction is carried out at 0 to +30°C in N,N-dimethylformamide as the solvent.

Especially preferred conditions for this Process variant are described, for example, in Example P.12 (step A).

### Process variant (N):

Examples of solvents and diluents are the same as those mentioned under Process variant A. In addition, alcohols, such as methanol, ethanol or 2-propanol, and water are suitable.

The reactions are advantageously carried out in a temperature range of approximately from -70°C to 100°C, preferably at from -10°C to 25°C.

There are suitable for the removal of the protecting group Lewis acids, such as hydrochloric acid, methanesulfonic acid, HF in pyridine, p-toluenesulfonic acid; ammonium fluoride, such as tetrabutylammonium fluoride; bases, such as ammonia, trialkylamine or heterocyclic bases; hydrogenolysis with a catalyst, such as palladium-on-carbon; reducing agents, such as sodium borohydride or tributyltin hydride with a catalyst, such as Pd(PPh₃)₄, or also zinc with acetic acid.

Preference is given to acids, such as methanesulfonic acid or HF in pyridine; sodium borohydride with Pd(O); bases, such as ammonia, triethylamine or pyridine; especially acids, such as HF in pyridine or methanesulfonic acid.

Especially preferred conditions for the reaction are described in Examples P1 (Step C), P2 (Step D), P3 (Step B), P4 (Step B), P5 (Step B), P7 (Step B), P10 (Step C), P11 (Step B), P12 (Step B), P13 (Step C) and P14 (Step 2).

### Process variant (O):

Examples of solvents and diluents include those listed above under Process variant (A); especially suitable are aromatic, aliphatic and alicyclic hydrocarbons and halogenated hydrocarbons, such as benzene, toluene, xylene, mesitylene, tetralin, chlorobenzene, dichlorobenzene, bromobenzene, petroleum ether, hexane, cyclohexane, dichloromethane, trichloromethane, tetrachloromethane, dichloroethane, trichloroethene or tetrachloroethene.

The reactions are advantageously carried out in a temperature range of from about 30°C to the boiling point of the solvent used; preference being given to reaction at 30°C to +110°C.

Examples of cross-metathesis catalysts are known to a person skilled in the art; they include ruthenium alkylidene catalysts, for example, 1,3-bis-(2,4,6-trimethylphenyl)-2-imidazolidinylidene)dichloro(phenylmethylene)-tricyclohexylphosphine)-ruthenium.

In a preferred embodiment of Variant (O) the reaction is carried out at in refluxing dichloromethane as the solvent.

Especially preferred conditions for this Process variant are described, for example, in Example P.15 (step A).

The compounds of formula (I) may be in the form of one of the possible isomers or in the form of a mixture thereof, in the form of pure isomers or in the form of an isomeric mixture, i.e. in the form of a diastereomeric mixture; the invention relates both to the pure isomers and to the diastereomeric mixtures and is to be interpreted accordingly hereinabove and hereinbelow, even if stereochemical details are not mentioned specifically in every case.

The diastereomeric mixtures can be resolved into the pure isomers by known methods, for example by recrystallisation from a solvent, by chromatography, for example high pressure liquid chromatography (HPLC) on acetylcellulose, with the aid of suitable microorganisms, by cleavage with specific, immobilised enzymes, or *via* the formation of inclusion compounds, for example using crown ethers, only one isomer being complexed.

Apart from by separation of corresponding mixtures of isomers, pure diastereoisomers can be obtained according to the invention also by generally known methods of stereoselective synthesis, for example by carrying out the process according to the invention using starting materials having correspondingly suitable stereochemistry.

In each case it may be advantageous to isolate or synthesise the biologically more active isomer, where the individual components have different biological activity.

The compounds of formula (I) may also be obtained in the form of their hydrates and/or may include other solvents, for example solvents which may have been used for the crystallisation of compounds in solid form.

The invention relates to all those embodiments of the process according to which a compound obtainable as starting material or intermediate at any stage of the process is used as starting material and some or all of the remaining steps are carried out or a starting material is used in the form of a derivative or salt and/or its racemates or antipodes or, especially, is formed under the reaction conditions.

In the processes of the present invention it is preferable to use those starting materials and intermediates which result in the compounds of formula (I) that are especially preferred.

The invention relates especially to the preparation processes described in Examples 1.1 to 10.168.

In the area of pest control, the compounds of formula (I) according to the invention are active ingredients exhibiting valuable preventive and/or curative activity with a very advantageous biocidal spectrum and a very broad spectrum, even at low rates of concentration, while being well tolerated by warm-blooded animals, fish and plants. They are, surprisingly, equally suitable for controlling both plant pests and ecto- and endo-parasites in humans and more especially in productive livestock, domestic animals and pets. They are effective against all or individual development stages of normally sensitive animal pests, but also of resistant animal pests, such as insects and representatives of the order Acarina, nematodes, cestodes and trematodes, while at the same time protecting useful organisms. The insecticidal or acaricidal activity of the active ingredients according to the invention may manifest itself directly, i.e. in the mortality of the pests, which occurs immediately or only after some time, for example during moulting, or indirectly, for example in reduced oviposition and/or hatching rate. Good activity corresponds to a mortality of at least 50 to 60 %.

Successful control within the scope of the subject of the invention is possible, in particular, of pests from the orders Lepidoptera, Coleoptera, Orthoptera, Isoptera, Psocoptera, Anoplura, Mallophaga, Thysanoptera, Heteroptera, Homoptera, Hymenoptera, Diptera, Siphonaptera, Thysanura and Acarina, mainly Acarina, Diptera, Thysanoptera, Lepidoptera and Coleoptera. Very especially good control is possible of the following pests:

Abagrotis spp., Abraxas spp., Acantholeucania spp., Acanthoplusia spp., Acarus spp., Acarus siro, Aceria spp., Aceria sheldoni, Acleris spp., Acoloithus spp., Acompsia spp., Acossus spp., Acria spp., Acrobasis spp., Acrocercops spp., Acrolepia spp., Acrolepiopsis spp., Acronicta spp., Acropolitis spp., Actebia spp., Aculus spp., Aculus schlechtendali, Adoxophyes spp., Adoxophyes reticulana, Aedes spp., Aegeria spp., Aethes spp., Agapeta spp., Agonopterix spp., Agriopis spp., Agriotes spp., Agriphila spp., Agrochola spp., Agroperina spp., Alabama spp., Alabama argillaceae, Agrotis spp., Albuna spp., Alcathoe spp., Alcis spp., Aleimma spp., Aletia spp., Aleurothrixus spp., Aleurothrixus floccosus, Aleyrodes spp., Aleyrodes brassicae, Allophyes spp., Alsophila spp., Amata spp., Amathes spp., Amblyomma spp., Amblyptilia spp., Ammoconia spp., Amorbia spp., Amphion spp., Amphipoea spp., Amphipyra spp., Amyelois spp., Anacamptodes spp., Anagrapha spp., Anarsia spp., Anatrychyntis spp., Anavitrinella spp., Ancylis spp., Andropolia spp., Anhimella spp., Antheraea spp., Antherigona spp., Antherigona soccata, Anthonomus spp., Anthonomus grandis, Anticarsia spp., Anticarsia gemmatalis, Aonidiella spp., Apamea spp., Aphania spp., Aphelia spp., Aphididae, Aphis spp., Apotomis spp., Aproaerema spp., Archippus spp., Archips spp., Acromyrmex, Arctia spp., Argas spp., Argolamprotes spp., Argyresthia spp., Argyrogramma spp., Argyroploce spp., Argyrotaenia spp., Arotrophora spp., Ascotis spp., Aspidiotus spp., Aspilapteryx spp., Asthenoptycha spp., Aterpia spp., Athetis spp., Atomaria spp., Atomaria linearis, Atta spp., Atypha spp., Autographa spp., Axylia spp., Bactra spp., Barbara spp., Batrachedra spp., Battaristis spp., Bembecia spp., Bemisia spp., Bemisia tabaci, Bibio spp., Bibio hortulanis, Bisigna spp., Blastesthia spp., Blatta spp., Blatella spp., Blepharosis spp., Bleptina spp., Boarmia spp., Bombyx spp., Bomolocha spp., Boophilus spp., Brachmia spp., Bradina spp., Brevipalpus spp., Brithys spp., Bryobia spp., Bryobia praetiosa, Bryotropha spp., Bupalus spp., Busseola spp., Busseola fusca, Cabera spp., Cacoecimorpha spp., Cadra spp., Cadra cautella, Caenurgina spp., Calipitrimerus spp., Callierges spp., Callophpora spp., Callophpora erythrocephala, Calophasia spp., Caloptilia spp., Calybites spp., Capnoptycha spp., Capua spp., Caradrina spp., Caripeta spp., Carmenta spp., Carposina spp., Carposina nipponensis, Catamacta spp., Catelaphris spp., Catoptria spp., Caustoloma spp., Celaena spp., Celypha spp., Cenopis spp., Cephus spp., Ceramica spp., Cerapteryx spp., Ceratitis spp, Ceratophyllus spp., Ceroplaster spp., Chaetocnema spp., Chaetocnema tibialis, Chamaesphecia spp., Charanvca spp., Cheimophila spp., Chersotis spp., Chiasmia spp., Chilo spp., Chionodes spp., Chorioptes spp., Choristoneura spp., Chrysaspidia spp., Chrysodeixis spp., Chrysomya spp., Chrysomphalus spp., Chrysomphalus dictyospermi, Chrysomphalus aonidium, Chrysoteuchia spp., Cilix spp., Cimex spp., Clysia spp., Clysia ambiguella, Clepsis spp., Cnaemidophorus spp., Cnaphalocrocis spp., Cnephasia spp., Coccus spp., Coccus hesperidum, Cochylis spp., Coleophora spp., Colotois spp., Commophila spp., Conistra spp., Conopomorpha spp., Corcyra spp., Cornutiplusia spp., Cosmia spp., Cosmopolites spp., Cosmopterix spp., Cossus spp., Costaeonvexa spp., Crambus spp., Creatonotos spp., Crocidolomia spp., Crocidolomia binotalis, Croesia spp., Crymodes spp., Cryptaspasma spp., Cryptoblabes spp., Cryptocala spp., Cryptophlebia spp., Cryptophlebia leucotreta, Cryptoptila spp., Ctenopseustis spp., Ctenocephalides spp., Cucullia spp., Curculio spp., Culex spp., Cuterebra spp., Cydia spp., Cydia pomonella, Cymbalophora spp., Dactylethra spp., Dacus spp., Dadica spp., Damalinea spp., Dasychira spp., Decadarchis spp., Decodes spp., Deilephila spp., Deltodes spp., Dendrolimus spp., Depressaria spp., Dermestes spp., Dermanyssus spp., Dermanyssus gallinae, Diabrotica spp., Diachrysia spp., Diaphania spp., Diarsia spp., Diasemia spp., Diatraea spp., Diceratura spp., Dichomeris spp., Dichrocrocis spp., Dichrorampha spp., Dicycla spp., Dioryctria spp., Diparopsis spp., Diparopsis castanea, Dipleurina spp., Diprion spp., Diprionidae, Discestra spp., Distantiella spp., Distantiella theobroma, Ditula spp., Diurnea spp., Doratopteryx spp., Drepana spp., Drosphila spp., Drosphila melanogaster, Dysauxes spp., Dysdercus spp., Dysstroma spp., Eana spp., Earias spp., Ecclitica spp., Ecdytolopha spp., Ecpyrrhorrhoe spp., Ectomyelois spp., Eetropis spp., Egira spp., Elasmopalpus spp., Emmelia spp., mpoasca spp., Empyreuma spp., Enargia spp., Enarmonia spp., Endopiza spp., Endothenia spp., Endotricha spp., Eoreuma spp., Eotetranychus spp., Eotetranychus carpini, Epagoge spp., Epelis spp., Ephestia spp., Ephestiodes spp., Epiblema spp., Epiehoristodes spp., Epinotia spp., Epiphyas spp., Epiplema spp., Epipsestis spp., Epirrhoe spp., Episimus spp., Epitymbia spp., Epllachna spp., Erannis spp., Erastria spp., Eremnus spp., Ereunetis spp., Eriophyes spp., Eriosoma spp., Eriosoma lanigerum, Erythroneura spp., Estigmene spp., Ethmia spp., Etiella spp., Euagrotis spp., Eucosma spp., Euehlaena spp., Euelidia spp., Eueosma spp., Euchistus spp., Eucosmomorpha spp., Eudonia spp., Eufidonia spp., Euhyponomeutoides spp., Eulepitodes spp., Eulia spp., Eulithis spp., Eupithecia spp., Euplexia spp., Eupoecilia spp., Eupoecilia ambiguella, Euproctis spp., Eupsilia spp., Eurhodope spp., Eurois spp., Eurygaster spp., Eurythmia spp., Eustrotia spp., Euxoa spp., Euzophera spp., Evergestis spp., Evippe spp., Exartema spp., Fannia spp., Faronta spp., Feltia spp., Filatima spp., Fishia spp., Frankliniella spp., Fumibotys spp., Gaesa spp., Gasgardia spp., Gastrophilus spp., Gelechia spp., Gilpinia spp., Gilpinia polytoma, Glossina spp., Glyphipterix spp., Glyphodes spp., Gnorimoschemini spp., Gonodonta spp., Gortyna spp., Gracillaria spp., Graphania spp., Grapholita spp., Grapholitha spp., Gravitarmata spp., Gretchena spp., Griselda spp., Gryllotalpa spp., Gynaephora spp., Gypsonoma spp., Hada spp., Haematopinus spp., Halisidota spp., Harpipteryx spp., Harrisina spp., Hedya spp., Helicoverpa spp., Heliophobus spp., Heliothis spp., Hellula spp., Helotropa spp., Hemaris spp., Hercinothrips spp., Herculia spp., Hermonassa spp., Heterogenea spp., Holomelina spp., Homadaula spp., Homoeosoma spp., Homoglaea spp., Homohadena spp., Homona spp., Homonopsis spp., Hoplocampa spp., Hoplodrina spp., Hoshinoa spp., Hxalomma spp., Hydraecia spp., Hydriomena spp., Hyles spp., Hyloicus spp., Hypagyrtis spp., Hypatima spp., Hyphantria spp., Hyphantria cunea, Hypocala spp., Hypocoena spp., Hypodema spp., Hyppobosca spp., Hypsipyla spp., Hyssia spp., Hysterosia spp., Idaea spp., Idia spp., Ipimorpha spp., Isia spp., Isochorista spp., Isophrictis spp., Isopolia spp., Isotrias spp., Ixodes spp., Itame spp., Jodia spp., Jodis spp., Kawabea spp., Keiferia spp., Keiferia lycopersicella, Labdia spp., Lacinipolia spp., Lambdina spp., Lamprothritpa spp., Laodelphax spp., Lasius spp., Laspeyresia spp., Leptinotarsa spp., Leptinotarsa decemlineata, Leptocorisa spp., Leptostales spp., Lecanium spp., Lecanium comi, Lepidosaphes spp., Lepisma spp., Lepisma saccharina , Lesmone spp., Leucania spp., Leucinodes spp., Leucophaea spp., Leucophaea maderae, Leucoptera spp., Leucoptera scitella, Linognathus spp., Liposcelis spp., Lissorhoptrus spp., Lithacodia spp., Lithocolletis spp., Lithomoia spp., Lithophane spp., Lixodessa spp., Lobesia spp., Lobesia botrana, Lobophora spp., Locusta spp., Lomanaltes spp., Lomographa spp., Loxagrotis spp., Loxostege spp., Lucilia spp., Lymantria spp., Lymnaecia spp., Lyonetia spp., Lyriomyza spp., Macdonnoughia spp., Macrauzata spp., Macronoctua spp., Macrosiphus spp., Malacosoma spp., Maliarpha spp., Mamestra spp., Mamestra brassicae, Manduca spp., Manduca sexta, Marasmia spp., Margaritia spp., Matratinea spp., Matsumuraeses spp., Melanagromyza spp., Melipotes spp., Melissopus spp., Melittia spp., Melolontha spp., Meristis spp., Meritastis spp., Merophyas spp., Mesapamea spp., Mesogona spp., Mesoleuca spp., Metanema spp., Metendothenia spp., Metzneria spp., Micardia spp., Microcorses spp., Microleon spp., Mnesictena spp., Mocis spp., Monima spp., Monochroa spp., Monomorium spp., Monomorium pharaonis, Monopsis spp., Morrisonia spp., Musca spp., Mutuuraia spp., Myelois spp., Mythimna spp., Myzus spp., Naranga spp., Nedra spp., Nemapogon spp., Neodiprion spp., Neosphaleroptera spp., Nephelodes spp., Nephotettix spp., Nezara spp., Nilaparvata spp., Niphonympha spp., Nippoptilia spp., Noctua spp., Nola spp., Notocelia spp., Notodonta spp., Nudaurelia spp., Ochropleura spp., Ocnerostoma spp., Oestrus spp., Olethreutes spp., Oligia spp., Olindia spp., Olygonychus spp., Olygonychus gallinae, Oncocnemis spp., Operophtera spp., Ophisma spp., Opogona spp., Oraesia spp., Orniodoros spp., Orgyia spp., Oria spp., Orseolia spp., Orthodes spp., Orthogonia spp., Orthosia spp., Oryzaephilus spp., Oscinella spp., Oscinella frit, Osminia spp., Ostrinia spp., Ostrinia nubilalis, Otiorhynchus spp., Ourapteryx spp., Pachetra spp., Pachysphinx spp., Pagyda spp., Paleacrita spp., Paliga spp., Palthis spp., Pammene spp., Pandemis spp., Panemeria spp., Panolis spp., Panolis flammea, Panonychus spp., Parargyresthia spp., Paradiarsia spp., Paralobesia spp., Paranthrene spp., Parapandemis spp., Parapediasia spp., Parastichtis spp., Parasyndemis spp., Paratoria spp., Pareromeme spp., Pectinophora spp., Pectinophora gossypiella, Pediculus spp., Pegomyia spp., Pegomyia hyoscyami, Pelochrista spp., Pennisetia spp., Penstemonia spp., Pemphigus spp., Peribatodes spp., Peridroma spp., Perileucoptera spp., Periplaneta spp., Perizoma spp., Petrova spp., Pexicopia spp., Phalonia spp., Phalonidia spp., Phaneta spp., Phlyctaenia spp., Phlyctinus spp., Phorbia spp., Phragmatobia spp., Phricanthes spp., Phthorimaea spp., Phthorimaea operculella, Phyllocnistis spp., Phyllocoptruta spp., Phyllocoptruta oleivora, Phyllonorycter spp., Phyllophila spp., Phylloxera spp., Pieris spp., Pieris rapae, Piesma spp., Planococus spp., Planotortrix spp., Platyedra spp., Platynota spp., Platyptilia spp., Platysenta spp., Plodia spp., Plusia spp., Plutella spp., Plutella xylostella, Podosesia spp., Polia spp., Popillia spp., Polymixis spp., Polyphagotarsonemus spp., Polyphagotarsonemus latus, Prays spp., Prionoxystus spp., Probole spp., Proceras spp., Prochoerodes spp., Proeulia spp., Proschistis spp., Proselena spp., Proserpinus spp., Protagrotis spp., Proteoteras spp., Protobathra spp., Protoschinia spp., Pselnophorus spp., Pseudaletia spp., Pseudanthonomus spp., Pseudaternelia spp., Pseudaulacaspis spp., Pseudexentera spp., Pseudococus spp., Pseudohermenias spp., Pseudoplusia spp., Psoroptes spp., Psylla spp., Psylliodes spp., Pterophorus spp., Ptycholoma spp., Pulvinaria spp., Pulvinaria aethiopica, Pyralis spp., Pyrausta spp., Pyrgotis spp., Pyrreferra spp., Pyrrharctia spp., Quadraspidiotus spp., Rancora spp., Raphia spp., Reticultermes spp., Retinia spp., Rhagoletis spp, Rhagoletis pomonella, Rhipicephalus spp., Rhizoglyphus spp., Rhizopertha spp., Rhodnius spp., Rhophalosiphum spp., Rhopobota spp., Rhyacia spp., Rhyacionia spp., Rhynchopacha spp., Rhyzosthenes spp., Rivula spp., Rondotia spp., Rusidrina spp., Rynchaglaea spp., Sabulodes spp., Sahlbergella spp., Sahlbergella singularis, Saissetia spp., Samia spp., Sannina spp., Sanninoidea spp., Saphoideus spp., Sarcoptes spp., Sathrobrota spp., Scarabeidae, Sceliodes spp., Schinia spp., Schistocerca spp., Schizaphis spp., Schizura spp., Schreckensteinia spp., Sciara spp., Scirpophaga spp., Scirthrips auranti, Scoparia spp., Scopula spp., Scotia spp., Scotinophara spp., Scotogramma spp., Scrobipalpa spp., Scrobipalpopsis spp., Semiothisa spp., Sereda spp., Sesamia spp., Sesia spp., Sicya spp., Sideridis spp., Simyra spp., Sineugraphe spp., Sitochroa spp., Sitobion spp., Sitophilus spp., Sitotroga spp., Solenopsis spp., Smerinthus spp., Sophronia spp., Spaelotis spp., Spargaloma spp., Sparganothis spp., Spatalistis spp., Sperchia spp., Sphecia spp., Sphinx spp., Spilonota spp., Spodoptera spp., Spodoptera littoralis, Stagmatophora spp., Staphylinochrous spp., Stathmopoda spp., Stenodes spp., Sterrha spp., Stomoxys spp., Strophedra spp., Sunira spp., Sutyna spp., Swammerdamia spp., Syllomatia spp., Sympistis spp., Synanthedon spp., Synaxis spp., Syncopacma spp., Syndemis spp., Syngrapha spp., Synthomeida spp., Tabanus spp., Taeniarchis spp., Taeniothrips spp., Tannia spp., Tarsonemus spp., Tegulifera spp., Tehama spp., Teleiodes spp., Telorta spp., Tenebrio spp., Tephrina spp., Teratoglaea spp., Terricula spp., Tethea spp., Tetranychus spp., Thalpophila spp., Thaumetopoea spp., Thiodia spp., Thrips spp., Thrips palmi, Thrips tabaci, Thyridopteryx spp., Thyris spp., Tineola spp., Tipula spp., Tortricidia spp., Tortrix spp., Trachea spp., Trialeurodes spp., Trialeurodes vaporariorum, Triatoma spp., Triaxomera spp., Tribolium spp., Tricodectes spp., Trichoplusia spp., Trichoplusia ni, Trichoptilus spp., Trioza spp., Trioza erytreae, Triphaenia spp., Triphosa spp., Trogoderma spp., Tyria spp., Udea spp., Unaspis spp., Unaspis citri, Utetheisa spp., Valeriodes spp., Vespa spp., Vespamima spp., Vitacea spp., Vitula spp., Witlesia spp., Xanthia spp., Xanthorhoe spp., Xanthotype spp., Xenomicta spp., Xenopsylla spp., Xenopsylla cheopsis, Xestia spp., Xylena spp., Xylomyges spp., Xyrosaris spp., Yponomeuta spp., Ypsolopha spp., Zale spp., Zanclognathus spp., Zeiraphera spp., Zenodoxus spp., Zeuzera spp., Zygaena spp.,

It is also possible to control pests of the class Nematoda using the compounds according to the invention. Such pests include, for example,
root knot nematodes, cyst-forming nematodes and also stem and leaf nematodes;
especially of Heterodera spp., e.g. Heterodera schachtii, Heterodora avenae and Heterodora trifolii; Globodera spp., e.g. Globodera rostochiensis; Meloidogyne spp., e.g. Meloidogyne incognita and Meloidogyne javanica; Radopholus spp., e.g. Radopholus similis; Pratylenchus, e.g. Pratylenchus neglectans and Pratylenchus penetrans; Tylenchulus, e.g. Tylenchulus semipenetrans; Longidorus, Trichodorus, Xiphinema, Ditylenchus, Apheenchoides and Anguina; especially Meloidogyne, e.g. Meloidogyne incognita, and Heterodera, e.g. Heterodera glycines.

An especially important aspect of the present invention is the use of the compounds of formula (I) according to the invention in the protection of plants against parasitic feeding pests.

The action of the compounds according to the invention and the compositions comprising them against animal pests can be significantly broadened and adapted to the given circumstances by the addition of other insecticides, acaricides or nematicides. Suitable additives include, for example, representatives of the following classes of active ingredient: organophosphorus compounds, nitrophenols and derivatives, formamidines, ureas, carbamates, pyrethroids, chlorinated hydrocarbons, neonicotinoids and Bacillus thuringiensis preparations.

Examples of especially suitable mixing partners include: azamethiphos; chlorfenvinphos; cypermethrin, cypermethrin high-cis; cyromazine; diafenthiuron; diazinon; dichlorvos; dicrotophos; dicyclanil; fenoxycarb; fluazuron; furathiocarb; isazofos; iodfenphos; kinoprene; lufenuron; methacriphos; methidathion; monocrotophos; phosphamidon; profenofos; diofenolan; a compound obtainable from the Bacillus thuringiensis strain GC91 or from strain NCTC11821; pymetrozine; bromopropylate; methoprene; disulfoton; quinalphos; taufluvalinate; thiocyclam; thiometon; aldicarb; azinphos-methyl; benfuracarb; bifenthrin; buprofezin; carbofuran; dibutylaminothio; cartap; chlorfluazuron; chlorpyrifos; clothianidin; cyfluthrin; lambda-cyhalothrin; alpha-cypermethrin; zeta-cypermethrin; deltamethrin; diflubenzuron; endosulfan; ethiofencarb; fenitrothion; fenobucarb; fenvalerate; formothion; methiocarb; heptenophos; imidacloprid; isoprocarb; methamidophos; methomyl; mevinphos; parathion; parathion-methyl; phosalone; pirimicarb; propoxur; teflubenzuron; terbufos; triazamate; fenobucarb; tebufenozide; fipronil; beta-cyfluthrin; silafluofen; fenpyroximate; pyridaben; pyridalyl; fenazaquin; pyriproxyfen; pyrimidifen; nitenpyram; acetamiprid; emamectin; emamectin-benzoate; spinosad; a plant extract that is active against insects; a preparation that comprises nematodes and is active against insects; a preparation obtainable from Bacillus subtilis; a preparation that comprises fungi and is active against insects; a preparation that comprises viruses and is active against insects; chlorfenapyr; acephate; acrinathrin; alanycarb; alphamethrin; amitraz; AZ 60541; azinphos A; azinphos M; azocyclotin; bendiocarb; bensultap; beta-cyfluthrin; brofenprox; bromophos A; bufencarb; butocarboxin; butylpyridaben; cadusafos; carbaryl; carbophenothion; chloethocarb; chlorethoxyfos; chlormephos; cis-resmethrin; clocythrin; clofentezine; cyanophos; cycloprothrin; cyhexatin; demeton M; demeton S; demeton-S-methyl; dichlofenthion; dicliphos; diethion; dimethoate; dimethylvinphos; dioxathion; edifenphos; esfenvalerate; ethion; ethofenprox; ethoprophos; etrimphos; fenamiphos; fenbutatin oxide; fenothiocarb; fenpropathrin; fenpyrad; fenthion; fluazinam; flucycloxuron; flucythrinate; flufenoxuron; flufenprox; fonophos; fosthiazate; fubfenprox; HCH; hexaflumuron; hexythiazox; flonicamid; iprobenfos; isofenphos; isoxathion; ivermectin; malathion; mecarbam; mesulfenphos; metaldehyde; metolcarb; milbemectin; moxidectin; naled; NC 184; nithiazine; omethoate; oxamyl; oxydemethon M; oxydeprofos; permethrin; phenthoate; phorate; phosmet; phoxim; pirimiphos M; pirimiphos E; promecarb; propaphos; prothiofos; prothoate; pyrachlophos; pyradaphenthion; pyresmethrin; pyrethrum; tebufenozide; salithion; sebufos; sulfotep; sulprofos; tebufenpyrad; tebupirimphos; tefluthrin; temephos; terbam; tetrachlorvinphos; thiacloprid; thiafenox; thiamethoxam; thiodicarb; thiofanox; thionazin; thuringiensin; tralomethrin; triarathene; triazophos; triazuron; trichlorfon; triflumuron; trimethacarb; vamidothion; xylylcarb; etoxazole; zetamethrin; indoxacarb; methoxyfenozide; bifenazate; XMC (3,5-xylyl methylcarbamate); or the fungus pathogen Metarhizium anisopliae.

The compounds according to the invention can be used to control, i.e. to inhibit or destroy, pests of the mentioned type occurring on plants, especially on useful plants and ornamentals in agriculture, in horticulture and in forestry, or on parts of such plants, such as the fruits, blossoms, leaves, stems, tubers or roots, while in some cases plant parts that grow later are still protected against those pests.

Target crops include especially cereals, such as wheat, barley, rye, oats, rice, maize and sorghum; beet, such as sugar beet and fodder beet; fruit, e.g. pomes, stone fruit and soft fruit, such as apples, pears, plums, peaches, almonds, cherries and berries, e.g. strawberries, raspberries and blackberries; leguminous plants, such as beans, lentils, peas and soybeans; oil plants, such as rape, mustard, poppy, olives, sunflowers, coconut, castor oil, cocoa and groundnuts; cucurbitaceae, such as marrows, cucumbers and melons; fibre plants, such as cotton, flax, hemp and jute; citrus fruits, such as oranges, lemons, grapefruit and mandarins; vegetables, such as spinach, lettuce, asparagus, cabbages, carrots, onions, tomatoes, potatoes and paprika; lauraceae, such as avocado, cinnamon and camphor; and tobacco, nuts, coffee, aubergines, sugar cane, tea, pepper, vines, hops, bananas, natural rubber plants and ornamentals.

Further areas of use of the compounds according to the invention are the protection of stored goods and storerooms and the protection of raw materials, and also in the hygiene sector, especially the protection of domestic animals and productive livestock against pests of the mentioned type, more especially the protection of domestic animals, especially cats and dogs, from infestation by fleas, ticks and nematodes.

The invention therefore relates also to pesticidal compositions, such as emulsifiable concentrates, suspension concentrates, directly sprayable or dilutable solutions, spreadable pastes, dilute emulsions, wettable powders, soluble powders, dispersible powders, wettable powders, dusts, granules and encapsulations of polymer substances, that comprise at least one of the compounds according to the invention, the choice of formulation being made in accordance with the intended objectives and the prevailing circumstances.

The active ingredient is used in those compositions in pure form, a solid active ingredient, for example, in a specific particle size, or preferably together with at least one of the adjuvants customary in formulation technology, such as extenders, e.g. solvents or solid carriers, or surface-active compounds (surfactants). In the area of parasite control in humans, domestic animals, productive livestock and pets it will be self-evident that only physiologically tolerable additives are used.

Solvents are, for example: non-hydrogenated or partly hydrogenated aromatic hydrocarbons, preferably fractions C₈ to C₁₂ of alkylbenzenes, such as xylene mixtures, alkylated naphthalenes or tetrahydronaphthalene, aliphatic or cycloaliphatic hydrocarbons, such as paraffins or cyclohexane, alcohols, such as ethanol, propanol or butanol, glycols and ethers and esters thereof, such as propylene glycol, dipropylene glycol ether, ethylene glycol or ethylene glycol monomethyl or -ethyl ether, ketones, such as cyclohexanone, isophorone or diacetone alcohol, strongly polar solvents, such as N-methylpyrrolid-2-one, dimethyl sulfoxide or N,N-dimethylformamide, water, non-epoxidized or epoxidized plant oils, such as non-epoxidized or epoxidized rapeseed, castor, coconut or soya oil, and silicone oils.

The solid carriers used, for example for dusts and dispersible powders, are as a rule natural rock powders, such as calcite, talc, kaolin, montmorillonite or attapulgite. Highly disperse silicic acids or highly disperse absorbent polymers can also be added to improve the physical properties. Granular adsorptive granule carriers are porous types, such as pumice, crushed brick, sepiolite or bentonite, and non-sorbent carrier materials are calcite or sand. A large number of granular materials of inorganic or organic nature can furthermore be used, in particular dolomite or comminuted plant residues.

Surface-active compounds are, depending on the nature of the active compound to be formulated, nonionic, cationic and/or anionic surfactants or surfactant mixtures with good emulsifying, dispersing and wetting properties. The surfactants listed below are to be regarded only as examples; many other surfactants which are customary in formulation technology and are suitable according to the invention are described in the relevant literature.

Nonionic surfactants are, in particular, polyglycol ether derivatives of aliphatic or cycloaliphatic alcohols, saturated or unsaturated fatty acids and alkylphenols, which can contain 3 to 30 glycol ether groups and 8 to 20 carbon atoms in the (aliphatic) hydrocarbon radical and 6 to 18 carbon atoms in the alkyl radical of the alkylphenols. Substances which are furthermore suitable are water-soluble polyethylene oxide adducts, containing 20 to 250 ethylene glycol ether and 10 to 100 propylene glycol ether groups, on propylene glycol, ethylene diaminopolypropylene glycol and alkyl polypropylene glycol having 1 to 10 carbon atoms in the alkyl chain. The compounds mentioned usually contain 1 to 5 ethylene glycol units per propylene glycol unit. Examples are nonylphenol-polyethoxyethanols, castor oil polyglycol ethers, polypropylene-polyethylene oxide adducts, tributylphenoxypolyethoxyethanol, polyethylene glycol and octylphenoxypolyethoxyethanol. Other substances are fatty acid esters of polyoxyethylene sorbitan, such as polyoxyethylene sorbitan trioleate.

The cationic surfactants are, in particular, quaternary ammonium salts which contain, as substituents, at least one alkyl radical having 8 to 22 C atoms and, as further substituents, lower, non-halogenated or halogenated alkyl, benzyl or lower hydroxyalkyl radicals. The salts are preferably in the form of halides, methyl-sulfates or ethyl-sulfates. Examples are stearyl-trimethyl-ammonium chloride and benzyl-di-(2-chloroethyl)-ethylammonium bromide.

Suitable anionic surfactants can be both water-soluble soaps and water-soluble synthetic surface-active compounds. Suitable soaps are the alkali metal, alkaline earth metal and substituted or unsubstituted ammonium salts of higher fatty acids (C₁₀-C₂₂), such as the sodium or potassium salts of oleic or stearic acid, or of naturally occurring fatty acid mixtures, which can be obtained, for example, from coconut oil or tall oil; and furthermore also the fatty acid methyl-taurine salts. However, synthetic surfactants are more frequently used, in particular fatty sulfonates, fatty sulfates, sulfonated benzimidazole derivatives or alkylarylsulfonates. The fatty sulfonates and sulfates are as a rule in the form of alkali metal, alkaline earth metal or substituted or unsubstituted ammonium salts and in general have an alkyl radical of 8 to 22 C atoms, alkyl also including the alkyl moiety of acyl radicals; examples are the sodium or calcium salt of ligninsulfonic acid, of dodecylsulfuric acid ester or of a fatty alcohol sulfate mixture prepared from naturally occurring fatty acids. These also include the salts of sulfuric acid esters and sulfonic acids of fatty alcohol-ethylene oxide adducts. The sulfonated benzimidazole derivatives preferably contain 2 sulfonic acid groups and a fatty acid radical having about 8 to 22 C atoms. Alkylarylsulfonates are, for example, the sodium, calcium or triethanolammonium salts of dodecylbenzenesulfonic acid, of dibutylnaphthalenesulfonic acid or of a naphthalenesulfonic acid-formaldehyde condensation product. Corresponding phosphates, such as salts of the phosphoric acid ester of a p-nonylphenol-(4-14)-ethylene oxide adduct or phospholipids, can further also be used.

The compositions as a rule comprise 0.1 to 99 %, in particular 0.1 to 95 %, of active compound and 1 to 99.9 %, in particular 5 to 99.9 %, of - at least - one solid or liquid auxiliary, it being possible as a rule for 0 to 25 %, in particular 0.1 to 20 %, of the composition to be surfactants (% is in each case per cent by weight). While concentrated compositions are more preferred as commercial goods, the end user as a rule uses dilute compositions which comprise considerably lower concentrations of active compound. Preferred compositions are composed, in particular, as follows (% = per cent by weight):

### Emulsifiable concentrates:

| | |
|---|---|
| active ingredient: | 1 to 90%, preferably 5 to 20% |
| surfactant: | 1 to 30%, preferably 10 to 20% |
| solvent: | 5 to 98%, preferably 70 to 85% |

### Dusts:

| | |
|---|---|
| active ingredient: | 0.1 to 10%, preferably 0.1 to 1% |
| solid carrier: | 99.9 to 90%, preferably 99.9 to 99% |

### Suspension concentrates:

| | |
|---|---|
| active ingredient: | 5 to 75%, preferably 10 to 50% |
| water: | 94 to 24%, preferably 88 to 30% |
| surfactant: | 1 to 40%, preferably 2 to 30% |

### Wettable powders:

| | |
|---|---|
| active ingredient: | 0.5 to 90%, preferably 1 to 80% |
| surfactant: | 0.5 to 20%, preferably 1 to 15% |
| solid carrier: | 5 to 99%, preferably 15 to 98% |

### Granules:

| | |
|---|---|
| active ingredient: | 0.5 to 30%, preferably 3 to 15% |
| solid carrier: | 99.5 to 70%, preferably 97 to 85% |

The compositions according to the invention may also comprise further solid or liquid adjuvants, such as stabilisers, e.g. vegetable oils or epoxidised vegetable oils (e.g. epoxidised coconut oil, rapeseed oil or soybean oil), antifoams, e.g. silicone oil, preservatives, viscosity regulators, binders and/or tackifiers as well as fertilisers or other active ingredients for obtaining special effects, e.g. acaricides, bactericides, fungicides, nematicides, molluscicides or selective herbicides.

The crop protection products according to the invention are prepared in known manner, in the absence of adjuvants, e.g. by grinding, sieving and/or compressing a solid active ingredient or mixture of active ingredients, for example to a certain particle size, and in the presence of at least one adjuvant, for example by intimately mixing and/or grinding the active ingredient or mixture of active ingredients with the adjuvant(s). The invention relates likewise to those processes for the preparation of the compositions according to the invention and to the use of the compounds of formula (I) in the preparation of those compositions.

The invention relates also to the methods of application of the crop protection products, i.e. the methods of controlling pests of the mentioned type, such as spraying, atomising, dusting, coating, dressing, scattering or pouring, which are selected in accordance with the intended objectives and the prevailing circumstances, and to the use of the compositions for controlling pests of the mentioned type. Typical rates of concentration are from 0.1 to 1000 ppm, preferably from 0.1 to 500 ppm, of active ingredient. The rates of application per hectare are generally from 1 to 2000 g of active ingredient per hectare, especially from 10 to 1000 g/ha, preferably from 20 to 600 g/ha, more especially from 20 to 100 g/ha.

A preferred method of application in the area of crop protection is application to the foliage of the plants (foliar application), the frequency and the rate of application being dependent upon the risk of infestation by the pest in question. However, the active ingredient can also penetrate the plants through the roots (systemic action) when the locus of the plants is impregnated with a liquid formulation or when the active ingredient is incorporated in solid form into the locus of the plants, for example into the soil, e.g. in granular form (soil application). In the case of paddy rice crops, such granules may be applied in metered amounts to the flooded rice field.

The crop protection products according to the invention are also suitable for protecting plant propagation material, e.g. seed, such as fruits, tubers or grains, or plant cuttings, against animal pests. The propagation material can be treated with the composition before planting: seed, for example, can be dressed before being sown. The active ingredients according to the invention can also be applied to grains (coating), either by impregnating the seeds in a liquid formulation or by coating them with a solid formulation. The composition can also be applied to the planting site when the propagation material is being planted, for example to the seed furrow during sowing. The invention relates also to such methods of treating plant propagation material and to the plant propagation material so treated. In the following examples those compounds of formula (I) wherein m=1 are included for reference only. The remaining examples serve to illustrate the invention. They do not limit the invention. Temperatures are given in degrees Celsius; mixing ratios of solvents are given in parts by volume.

### Preparation Examples:

### Example P1: 4"-Deoxy-4"-(S)-allyl-avermectin B₁ and 4"-deoxy-4"-(R)-allyl-avermectin B₁

Step A: To a solution of 15 g 5-O-*tert*-butyldimethylsilyl-avermectin B₁ and 6 ml pyridine in 150 ml dichloromethane at 0°C is added 7 ml *p*-tolyl chlorothionoformate and a catalytic amount of 4-(dimethylamino)pyridine. The reaction mixture is stirred for 2 hours at room temperature, poured into water, extracted with CH₂Cl₂, dried over Na₂SO₄, and concentrated. Flash chromatography (silica gel, hexanelethyl acetate 9/1) affords the corresponding 4"-O-(4-methylphenoxythionocarbonyl)-5-O-*tert*-butyldimethylsilyl avermectin B₁.

Step B: A degassed solution of 1.5 g of 4"-O-(4-methylphenoxythionocarbonyl)-5-O-tert-butyldimethylsilyl avermectin B, 10 ml in chlorobenzene is heated at 100°C under argon. 4 ml Allyltributyltin, then 72 mg 1,1'-azobis(cyclohexanecarbonitrile) are added and the solution is stirred at 100°C for 20 hours. The mixture is concentrated and the residue purified by flash chromatography (silica gel, hexane/ethyl acetate 9/1) providing 5-O-*tert*-butyldimethylsilyl-4"-deoxy-4"-(*S*)-allyl-avermectin B₁ and 5-O-*tert*-butyldimethylsilyl-4"-deoxy-4"-(*R*)-allyl-avermectin B₁ along with 5-OTBDMS-4"-deoxy-Avermectin B₁.

Step C: To a solution of 100 mg of 5-O-*tert*-butyldimethylsilyl-4"-deoxy-4"-(*S*)-allyl-avermectin B₁ in 5 ml tetrahydrofuran is added 1 ml of a solution of HF-pyridine (25 g commercial 70% HF-pyridine solution, 27.5 ml tetrahydrofuran, 12.5 ml pyridine) and the mixture is stirred overnight at room temperature, poured into water, extracted with diethyl ether, washed with saturated NaHCO₃, brine, dried over Na₂SO₄, and concentrated. Flash chromatography (silica gel, hexane/ethyl acetate 7/3) affords 4"-deoxy-4"-(*S*)-allyl-avermectin B₁, (compound 5.1).

The corresponding 5-O-*tert*-butyldimethylsilyl-4"-deoxy-4"-(*R*)-allyl-avermectin B₁ epimer provides 4"-deoxy-4"-(*R*)-allyl-avermectin B₁ in an identical manner.

4"-Deoxy-4"-(*S*)-allyl-avermectin B₁: C₅₁H₇₆O₁₃, MW: 896.5. LCMS: *t_{RT}*, B₁ₐ: 11-99 min., 919.5 (M+Na); 1 H NMR (500 MHz, CDCl₃) selected data, δH (ppm): 1.41 (m, 1H, CH-4"), 3.26 (t, *J* = 8.5 Hz, 1H, CH-4'), 3.31 (dd, *J*= 2.2, 3.8 Hz, 1H, CH-2), 3.37 (s, 3H, OCH₃), 3.44 (s, 3H, OCH₃), 3.75 (dq, *J* = 10.3, 6.2 Hz, 1H, CH-5"), 3.98 (d, 1H, *J* = 5.4 Hz, CH-6), 4.30 (m, 1H, CH-5), 5.04 (d, J = 10.2 Hz, 1H, =CH₂), 5.07 (d, J = 16.6 Hz, 1H, =CH₂), 5.81 (m, 1H, -CH=).

4"-Deoxy-4"-(*R*)-allyl-avermectin B₁: C₅₁H₇₆O₁₃, MW: 896.5. LCMS: *t_{RT}*, B₁ₐ: 12.38 min., 919.5 (M+Na); 1 H NMR (500 MHz, CDCl₃) selected data, δH (ppm): 1.96 (m, 1H, CH-4"), 3.24 (t, *J* = 8.5 Hz, 1 H, CH-4'), 3.31 (dd, *J*= 2.2, 3.8 Hz, 1 H, CH-2), 3.34 (s, 3H, OCH₃), 3.44 (s, 3H, OCH₃), 3.70 (dt, *J* = 12.0, 4.4 Hz, 1H, CH-3"), 3.98 (d, 1H, *J* = 5.4 Hz, CH-6), 4.30 (m, 1H, CH-5), 4.95 (d, J = 10.0 Hz, 1H, =CH₂), 5.07 (d, J = 16.1 Hz, 1H, =CH₂), 5.90 (m, 1 H, -CH=).

### Example P2: 4"-Deoxy-4"-(S)-β-Styryl-Avermectin B₁

Step A: To a solution of 5 g of 5-O-*tert*-butyldimethylsilyl-4"-epi-avermectin B₁ in 35 ml CH₂Cl₂ at -30°C are added 3.7 g 4-(dimethylamino)pyridine, 5.2 ml diisopropylethylamine, and finally 3.4 ml trifluoromethanesulfonic anhydride dropwise. The dark orange mixture is allowed to warm to 0°C and stirring at this temperature is continued for 3 hours. The reaction mixture is diluted with CH₂ClI₂, washed with water, dried over Na₂SO₄ and concentrated. The residue is filtered on silica gel (hexane/ethyl acetate 3/1) affording 5-O-*tert*-butyldimethylsilyl -4"-*ep*i-avermectin B₁-trifluoromethanesulfonate.

Step B: 742 mg Potassium iodide are added to a solution 5 g of 5-O-*tert*-butyldimethylsilyl-4"-*ep*i-avermectin B₁ trifluoromethanesulfonate in 40 ml N,N-dimethylformamide at - 10°C, and the reaction mixture is stirred for 2 hours. Water is added, and the mixture is extracted with CH₂Cl₂, dried over Na₂SO₄, and concentrated. Filtration on silica gel (hexane/ethyl acetate 3/1) affords 5-O-*tert*-butyldimethylsilyl-4"-deoxy-4"-(*S*)-iodo-avermectin B₁.

Step C: 2 g β-Tributylstannylstyrene and 100 mg of 1,1'-azobis(cyclohexanecarbonitrile) are added to a solution of 570 mg of 5-O-*tert*-butyldimethylsilyl-4"-deoxy-4"-(*S*)-iodo-avermectin B₁ in 5 ml chlorobenzene under argon. The solution is heated at 100°C and stirred at this temperature for 8 hours. Evaporation of the solvent under reduced pressure followed by flash chromatography (silica gel, hexane/ethyl acetate 85/15) affords 5-O-*tert-*butyldimethylsilyl-4"-deoxy-4"-(*S*)-β-styryl-avermectin B₁ along with 5-O-*tert*-butyldimethylsilyl-4"-deoxy-avermectin B₁.

Step D: 5-O-*tert*-butyldimethylsilyl-4"-deoxy-4"-(*S*)-β-styryl-avermectin B₁ is treated as described in Example P.1, Step C to give 4"-deoxy-4"-(*S*)-β-styryl-avermectin B₁ (compound 5.5).

4"-Deoxy-4"-(*S*)-β-styryl-avermectin B₁: C₅₆H₇₈O₁₃, MW: 958.5. LCMS: *t_{RT}*, B₁ₐ: 12.19 min., 981.5 (M+Na), 959.6 (M+H).

### Example P3: 4"-Deoxy-Avermectin B₁-4"-(S)-Acetaldehyde

Step A: To a solution of 1.7 g of 5-O-*tert*-butyldimethylsilyl-4"-deoxy-4"-(*S*)-allyl-avermectin B₁ (Example P1, Step B) in 25 ml tetrahydrofuran is added a solution of sodium 900 mg periodate in 25 ml water, then 1 ml OsO₄ (2.5% in tBuOH) is added; the resulting mixture is stirred for 24 hours, filtered, washed with water, brine, dried over Na₂SO₄, and concentrated. Flash chromatography (silica gel, hexane/ethyl acetate 7/3) affords 5-O-*tert-*butyldimethylsilyl-4"-deoxy-avermectin B₁-4"-(*S*)-acetaldehyde.

Step B: 5-O-*tert*-Butyldimethylsilyl-4"-deoxy-avermectin B₁-4"-(*S*)-acetaldehyde is treated as described in Example P1, Step C affording 4"-deoxy-Avermectin B₁-4"-(*S*)-acetaldehyde (compound 4.1).

4"-Deoxy-avermectin B₁-4"-(*S*)-acetaldehyde: C₅₀H₇₄O₁₄, MW: 898.5. LCMS: *t_{RT}*, B₁ₐ: 9.81 min., 921.6 (M+Na), 899.5 (M+H); 1 H NMR (300 MHz, CDCl₃) selected data, δH (ppm): 3.28 (s, 3H, OCH₃), 3.45 (s, 3H, OCH₃), 9.66 (s, 1H, CHO).

### Example P4: 4"-Deoxy-4"-(S)-(2-Hydroxy-ethyl)-Avermectin B₁

Step A: 250 mg of 5-O-*tert*-butyldimethylsilyl-4"-deoxy-4"-(*S*)-acetaldehydic-Avermectin B₁ in 10 ml methanol is reduced with 28 mg sodium borohydride at 0°C. After 30 min., the reaction mixture is poured into water, extracted with diethyl ether, dried over Na₂SO₄, and concentrated.

Step B: Crude residue of step A is treated as described in Example P.1, Step C affording 4"-deoxy-4"-(*S*)-(2-hydroxy-ethyl)-avermectin B₁ (compound 2.1).

4"-Deoxy-4"-(*S*)-(2-hydroxy-ethyl)-avermectin B₁: C₅₀H₇₆O₁₄, MW: 900.5. LCMS: *t_{RT},* B₁ₐ: 8.71 min., 923.5 (M+Na).

### Example P5: 4"-Deoxy-4"-(S)-(3-Hydroxy-propyl)-Avermectin B₁

Step A: To a solution of 200 mg 5-O-*tert*-butyldimethylsilyl-4"-deoxy-4"-(*S*)-allyl-avermectin B₁ in tetrahydrofuran (12 ml) is added 0.7 ml BH₃-tetrahydrofuran (1 M in tetrahydrofuran) and the mixture is stirred at room temperature for 5 hours. The solution is cooled to 0°C, and 2,8 ml 3N sodium hydroxide is added, followed by 2.8 ml 30% aqueous hydrogen peroxide. After 1 h at 0°C, the reaction mixture is poured into water, extracted with ethyl acetate, washed with water, brine, dried over Na₂SO₄, and concentrated. Flash chromatography of the residue (silica gel, hexane/ethyl acetate 65/35) affords 5-O-*tert*-butyldimethylsilyl -4"-deoxy-4"-(*S*)-(3-hydroxy-propyl)-avermectin B₁.

Step B: 71 mg of 5-O-*tert*-butyldimethylsilyl-4"-deoxy-4"-(*S*)-(3-hydroxy-propyl)-Avermectin B₁ are treated as described in Example P.1, step C, affording 4"-deoxy-4"-(*S*)-(2-hydroxy-propyl)-avermectin B₁ (compound 1.1).

4"-Deoxy-4"-(*S*)-(3-hydroxy-propyl)-avermectin B₁: C₅₁H₇₈O₁₄, MW: 914.5. LCMS: *t_{RT}*, B₁ₐ: 8.64 min., 937.4 (M+Na).

### Example P6: 4"-Deoxy-4"-(S)-(2,2-Dimethylamino-ethyl)-Avermectin B₁

To a solution of 105 mg of 4"-deoxy-avermectin B₁-4"-(*S*)-acetaldehyde in 3 ml of methanol are added 0.1 ml acetic acid, 0.08 ml dimethylamine, and finally sodium 28 mg of cyanoborohydride. The reaction mixture is stirred at room temperature for 30 min, poured into sat. NaHCO₃, extracted with ethyl acetate, dried over Na₂SO₄, and concentrated. 4"-deoxy-4"-(*S*)-(2,2-dimethylamino-ethyl)-avermectin B₁ is obtained, which requires no further purification (compound 2.83).

4"-Deoxy-4"-(*S*)-(2,2-dimethylamino-ethyl)-avermectin B₁: C₅₂H₈₁NO₁₃, MW: 927.6. LCMS: *t_{RT},* B₁ₐ: 5.16 min., 928.5 (M+H).

### Example P7: 4"-Deoxy-4"-(S)-(2-Hydroxypropyl)-Avermectin B₁

Step A: A solution of 440 mg of 5-O-*tert*-butyldimethylsilyl-4"-deoxy-avermectin B₁-4"-(*S*)-acetaldehyde in 10 ml tetrahydrofuran at -10°C is treated with 0.33 ml of methylmagnesium bromide (3M in diethyl ether). The mixture is stirred at room temperature for 1 hour, quenched by the addition of sat. NH₄Cl, extracted with ethyl acetate, washed with brine, dried and concentrated. Flash chromatography (silica gel, hexane/ethyl acetate 75/25) affords the two stereoisomers of 5-O-*tert*-butyldimethylsilyl-4"-deoxy-4"-(*S*)-(2-hydroxypropyl)-avermectin B₁.

Step B: Each isomer of 5-O-*tert*-butyldimethylsilyl-4"-deoxy-4"-(*S*)-(2-hydroxypropyl)-avermectin B₁ is treated as described in Example P.1, step C, affording respectively the two corresponding stereoisomers of 4"-deoxy-4"-(*S*)-(2-hydroxy-propyl)-avermectin B₁ (compound 2.5).

4"-Deoxy-4"-(*S*)-(2-hydroxypropyl)-Avermectin B₁: C₅₁H₇₈O₁₄, MW: 914.5. LCMS: 1^{st} isomer *t_{RT}*, B₁ₐ: 12.36 min., 937.5 (M+Na); 2^{nd} isomer *t_{RT}*, B₁ₐ: 11.98 min., 937.5 (M+Na).

### Example P8: 4"-Deoxy-4"-(S)-(2-oxo-propyl)-Avermectin B₁

A suspension of 200 mg of 4"-deoxy-4"-(*S*)-allyl-avermectin B₁ in a mixture of N,N-dimethylacetamide/water (0.45ml, 7/1) is treated with 4 mg palladium dichloride and 9 mg copper (II) acetate hydrate. The mixture is placed under oxygen at 1 atm and stirred for 24 hours. Additional PdCl₂ (8 mg) and Cu(OAc)₂.H₂O (18mg) are added and after 48 hours the mixture is diluted with diethyl ether and filtered through celite. The filtrate is poured into water, extracted with diethyl ether, washed with brine, dried and concentrated *in vacuo.* Flash chromatography (silica gel, hexane/ethyl acetate 6/4) affords 4"-deoxy-4"-(S)-(2-oxopropyl)-avermectin B₁ (compound 4.7)

4"-Deoxy-4"-(*S*)-(2-oxo-propyl)-avermectin B₁ : C₅₁H₇₆O₁₄, MW: 913.2. LCMS: B₁ₐ: 10.56 min., 935.5 (M+Na). 1 H NMR (500 MHz, CDCl₃) selected data, δH (ppm): 3.44 (s, 3H, OCH₃), 3.27 (s, 3H, OCH₃), 2.05 (s, 3H, CH₃C=O).

### Example P9: 4"-Deoxy-4"-(S)-(2-Hydroxy-2-methyl-propyl)-Avermectin B₁

A solution of 4"-deoxy-4"-(*S*)-(2-oxo-propyl)-avermectin B₁ (108 mg) in tetrahydrofuran (5 ml) at -10°C is treated with methylmagnesium bromide (3M in diethyl ether, 0.13 ml). The mixture is stirred at room temperature for 1 hour, quenched by the addition of sat. NH₄Cl, extracted with ethyl acetate, washed with brine, dried and concentrated. Flash chromatography (silica gel, hexane/ethyl acetate 6/4) affords 4"-deoxy-4"-(*S*)-(2-hydroxy-2-methyl-propyl)-Avermectin B, (56%). (compound 2.17)

4"-Deoxy-4"-(*S*)-(2-hydroxy-2-methyl-propyl)-Avermectin B₁: C₅₂H₈₀O₁₄, MW: 928.6. LCMS: *t_{RT}*, B₁ₐ: 13.46 min., 951.5 (M+Na), 929.6 (M+H).

### Example P10: 4"-Deoxy-4"-(R)-(Hydroxymethyl)-Avermectin B₁

Step A: Diiodomethane (0.4 ml) is added to a rapidly stirred suspension of zinc dust (0.6 g) in tetrahydrofuran (10 ml). The mixture is stirred 45 min. at room temperature, then cooled to 0°C. Titanium tetrachloride (1 M in CH₂Cl₂, 1 ml) is added and the resulting dark brown solution stirred 30 min. at room temperature. 5-O-*tert*-butyldimethylsilyl-4"-oxo-avermectin B1 (985 mg) in tetrahydrofuran (5 ml) is added dropwise and the reaction mixture stirred for 10 min at room temperature, added via syringe to a stirred mixture of sat. NaHCO₃ (50 ml) and ethyl acetate (50 ml). The organic layer is washed with brine, dried over Na₂SO₄, and concentrated. Flash chromatography of the residue (silica gel, hexane/ethyl acetate 7/3) affords 5-O-*tert*-butyldimethylsilyl-4"-deoxy-4"-exo-methylene-avermectin B₁.

Step B: 300 mg of 5-O-*tert*-butyldimethylsilyl-4"-deoxy-4"-*exo*-methylene-avermectin B₁ is treated as described in Example P.5, Step A, affording after flash chromatography (silica gel, hexane/ethyl acetate 7/3) 5-O-*tert*-butyldimethylsilyl-4"-deoxy-4"-(*R*)-(hydroxymethyl)-Avermectin B₁.

Step C: 5-O-*tert*-butyldimethylsilyl-4"-deoxy-4"-(*R*)-(hydroxymethyl)-Avermectin B₁ obtained in step B is treated as described in Example P.1, Step C affording 4"-deoxy-4"-(*R*)-(hydroxymethyl)-avermectin B₁.

4"-Deoxy-4"-(*R*)-(hydroxymethyl)-avermectin B₁: C₄₉H₇₄O₁₄, MW: 886.5. LCMS: *t_{RT},* B₁ₐ: 8.85 min., 909 (M+Na); 1 H NMR (500 MHz, CDCl₃) selected data, δH (ppm): 2.15 (m, 1H, CH-4"), 3.03 (br, 1H, CH₂OH), 3.43 (s, 3H, OCH₃), 3.45 (s, 3H, OCH₃), 3.84 (m, 1H, CH₂OH), 4.06 (m, 1H, CH₂OH).

### Example P11: 4"-Deoxy-4"-(S)-Cyano-Avermectin B₁

Step A: 5-O-*ter*t-Butyldimethylsilyl-4"-deoxy-4"-(*R*)-trifluoromethanesulfonyloxy-avermectin B₁ (500 mg) is dissolved in N,N-Dimethylformamide (10 ml). The solution is cooled to -40°C then tetrabutylammonium cyanide (240 mg) is added in one portion. The orange solution is slowly warmed to room temperature and stirred at this temperature for 6h. The reaction mixture is poured into water, extracted with ethyl acetate, washed with water, sat. NH₄Cl, dried over Na₂SO₄, dried over Na₂SO₄ and concentrated providing 5-O-*tert-*butyldimethylsilyl-4"-deoxy-4"-(*S*)-cyano-Avermectin B₁.

Step B: Crude 5-O-*tert*-butyldimethylsilyl-4"-deoxy-4"-(*S*)-cyano-avermectin B₁ of step A is treated as described in Example P.1, Step C affording after flash-chromatography (silica gel, hexane/ethyl acetate 7/3) 4"-deoxy-4"-(*S*)-cyano-Avermectin B₁ (compound 3.26).

4"-Deoxy-4"-(*S*)-cyano-avermectin B₁: C₄₉H₇₁NO₁₃, MW: 881.5. LCMS: *t_{RT}*, B₁ₐ: 10.17 min., 904 (M+Na), 882.5 (M+H); 1 H NMR (500 MHz, CDCl₃) selected data, δH (ppm): 2.28 (m, 1 H, CH-4"), 3.22 (t, *J* = 8.5 Hz, 1H, CH-4'), 3.31 (dd, *J*= 2.2, 3.8 Hz, 1 H, CH-2), 3.44 (s, 3H, OCH₃), 3.52 (s, 3H, OCH₃), 3.75 (dq, *J* = 10.3, 6.2 Hz, 1H, CH-5"), 3.95 (s, 1H, CH-13), 3.98 (d, 1 H, *J* = 5.4 Hz, CH-6), 4.02 (s, 1H, 7-OH), 4.30 (m, 1H, CH-5), 5.43 (s, 1H, CH-3).

### Example P12: 4"-Deoxy-Avermectin B₁-4"-(S)-Dimethylmalonate

Step A: Dimethylmalonate (0.08 ml) is added to a suspension of sodium hydride (60% in oil, 22 mg) in N,N-dimethylformamide (10 ml) at 0°C, and the mixture is stirred at this temperature for 10 min. 5-O-tert-butyldimethylsilyl-4"-*ep*i-Avermectin B₁-trifluoromethanesulfonate (500 mg) in N,N-dimethylformamide (5 ml) is then added dropwise, and the resulting orange solution is stirred at 50°C for 1 hours. Water is added, and the mixture is extracted with CH₂Cl₂, dried over Na₂SO₄, and concentrated. Flash chromatography (silica gel, hexane/ethyl acetate 9/1) 5-O-*tert*-butyldimethylsilyl-4"-deoxy-avermectin B₁-4"-(*S*)-dimethylmalonate.

Step B: 5-O-*tert*-butyldimethylsilyl-4"-deoxy-Avermectin B₁-4"-(*S*)-dimethylmalonate is treated as described in Example P.1, Step C to give 4"-deoxy-Avermectin B₁-4"-(*S*)-dimethylmalonate (compound 3.1).

4"-Deoxy-avermectin B₁-4"-(*S*)-dimethylmalonate: C_{S3}H₇₈O₁₇, MW: 986.5. LCMS: *t_{RT}*, B₁ₐ: 9.89 min., 1009.4 (M+Na).

### Example P13: 4'-Deoxy-4'-(S)-Allyl-Avermectin B₁ Monosaccharide

Step A: To a solution of 5-O-*tert*-butyldimethylsilyl-avermectin B₁ monosaccharide (0.84 g) in N,N-dimethylformamide (5 ml) at room temprature is added 1,1'-thiocarbonyl diimidazole (0.53 g). The reaction mixture is stirred at 60°C for 4 hours and on cooling to room temperature it is diluted with ethyl acetate and poured into water. Extraction of the aqueous phase with ethyl acetate is followed by drying over MgSO₄, and concentration *in vacuo.* Flash chromatography (silica gel, hexanelethyl acetate 9/1) of the crude residue affords 4'-O-(imidazolethionocarbonyl)-5-O-*tert*-butyldimethylsilyl avermectin B₁ which is characterized by its mass and nmr spectra.

Step B: A degassed solution of the 4'-O-(imidazolethionocarbonyl)-5-O-*tert-*butyldimethylsilyl avermectin B₁ (953 mg, 1.0 mmol) in toluene (10 ml) is treated with allyltributyltin (1.24 ml, 4.0 mmol) and α,α'-azoisobutyronitrile (131 mg, 0.8 mmol) and the solution is stirred at 85°C for 1.5 hours. The reaction mixture is diluted with ethyl acetate and poured into water. Extraction of the aqueous phase with ethyl acetate is followed by drying over MgSO₄ and concentrating *in vacuo.* The crude residue is purified by flash chromatography (silica gel, hexane/ethyl acetate 9/1) affording 5- O-tert-butyldimethylsilyl-4"-deoxy-4"-(*S*)-allyl-avermectin B₁ monosaccharide which is characterized by its mass and nmr spectra.

Step C: To a solution of 5-O-*tert*-butyldimethylsilyi-4'-O-(*S*)-allyl-avermectin B₁ monosaccharide (231 mg) in methanol (5 ml) at 0 °C is added methanesulphonic acid (0.02 ml). The reaction mixture is stirred for 4 hours and is then poured into saturated sodium bicarbonate, extracted with ethyl acetate, dried over Mg₂SO₄, and concentrated *in vacuo.* Flash chromatography (silica gel, hexane/ethyl acetate 3/0) affords 4'-O-(*S*)-allyl-avermectin B₁ monosaccharide which is characterized by its mass and nmr spectra (compound 5,2).

4'-O-(*S*)-Allyl-avermectin B₁ monosaccharide: B₁ₐ C₄₄H₆₄O₁₀, MW: 752.5. LCMS: *t_{RT}*,: 10.45 min., 775.4 (M+Na); B_{1b} C₄₃H₆₂O₁₀, MW: 738.4. LCMS: *t_{RT}*,: 9.76 min., 961.4 (M+Na).

### Example P14: 4'-Deoxy-4'-(S)-(2-oxo-propyl)-Avermectin B₁

Step A: A suspension of 150 mg of 5-O-*tert*-butyldimethylsilyl-4'-deoxy-4'-(*S*)-allyl-avermectin B₁ in a mixture of N,N-dimethylacetamide/water (0.35ml, 7/1) is treated with 5 mg palladium dichloride and 7 mg copper (II) acetate hydrate. The mixture is placed under oxygen at 1 atm and stirred for 48 hours. The mixture is diluted with diethyl ether and filtered through celite. The filtrate is poured into water, extracted with diethyl ether, washed with brine, dried and concentrated *in vacuo.* Flash chromatography (silica gel, hexane/ethyl acetate 6/4) affords 5-O-*tert*-butyldimethylsilyl-4'-deoxy-4'-(*S*)-(2-oxo-propyl)-avermectin B₁ which is characterized by its mass and nmr spectra.

4'-deoxy-4'-(*S*)-(2-oxo-propyl)-avermectin B₁ : C₅₁H₇₆O₁₄, MW: 913.2. LCMS: B₁ₐ: 10.56 min., 935.5 (M+Na). 1 H NMR (500 MHz, CDCl₃) selected data, δH (ppm): 3.44 (s, 3H, OCH₃), 3.27 (s, 3H, OCH₃), 2.05 (s, 3H, CH₃C=O).

Step B: 5-O-*tert*-butyldimethylsilyl-4'-deoxy-4'-(*S*)-(2-oxo-propyl)-avermectin B₁ of step A is treated as described in Example P1, Step C affording after flash-chromatography (silica gel, hexane/ethyl acetate 3/2) 4'-deoxy-4'-(*S*)-(2-oxo-propyl)-avermectin B₁ which is characterized by its mass and nmr spectra (compound 4.8).

4'-Deoxy-4'-(*S*)-(2-oxo-propyl)-avermectin B₁ : C₄₄H₆₄O₁₁, MW: 768.4. LCMS: B₁ₐ: 8.48 min., 791.5 (M+Na); B_{1b} C₄₃H₆₂O₁₁, MW: 754.4. LCMS: t_{RT},: 7.84 min., 777.4 (M+Na).

### Example P15: 4'-Deoxy-4'-(S)-(3-methoxycarbonyl-allyl)-Avermectin B₁

Step A: 250 mg of 5-O-*tert*-butyldimethylsilyl-4"-deoxy-4"-(*S*)-allyl-Avermectin B₁ from Example P13, Step B, in dichloromethane is treated with 20 mg of [1,3-bis-(2,4,6-trimethylphenyl)-2-imidazolidinylidene]dichloro-(phenylmethylene)-(tricyclohexylphosphine)-ruthenium. After refluxing for 8 h, the reaction mixture is concentrated *in vacuo.* Flash chromatography (silica gel, hexane/ethyl acetate 4/1) affords 5-O-*tert*-butyldimethylsilyl-4'-deoxy-4'-(*S*)-(3-methoxycarbonyl-allyl)-avermectin B₁ which is characterized by its mass and nmr spectra.

Step B: 5-O-*tert*-butyldimethylsilyl-4'-deoxy-4'-(*S*)-(3-methoxycarbonyl-allyl)-avermectin B₁ of step A is treated as described in Example P1, Step C affording after flash-chromatography (silica gel, hexane/ethyl acetate 3/2) 4'-deoxy-4'-(*S*)-(3-methoxycarbonyl-allyl)-avermectin B₁ (compound 5c.1) which is characterized by its mass and nmr spectra.

4'-Deoxy-4'-(*S*)-(3-methoxycarbonyl-allyl)-avermectin B₁ : C₄₆H₆₆O₁₂, MW: 810.5. LCMS: B₁ₐ: 10.61 min., 833.4 (M+Na).

### Example P16: 4"-Deoxy-4"-(S)-(2-Methyl-vinyl) Avermectin B₁

Step A To a degassed solution of 5-O-*tert*-butyldimethylsilyl-4"-deoxy-4"-(*S*)-allyl-Avermectin B₁ (215 mg) in CH₂Cl₂ (17 ml) at room temprature is added trimethyl-vinyloxy-silane (300 µl) followed by [1,3-bis-(2,4,6-trimethylphenyl)-2-imidazolidinylidene]dichloro-(phenylmethylene)-(tricyclohexylphosphine)-ruthenium (9 mg). The reaction mixture is stirred at 40°C until ¹H-NMR showed no starting material present in the mixture. After cooling to room temperature the solvent is evaporatet under reduced pressure and the crude is purified by flash chromatography (silica gel, hexane/ethyl acetate, 5/1) yielding 5-O-*tert*-butyldimethylsilyl-4"-deoxy-4"-(S)-(2-metylvinyl) Avermectin B₁ which is characterized by its mass and nmr spectra.

Step B: 5-O-*tert*-butyldimethylsilyl-4"-deoxy-4"-(S)-(2-metylvinyl) Avermectin B₁ of step A is treated as described in Example P1, Step C affording after flash-chromatography (silica gel, hexane/ethyl acetate, 2/1) 4"-deoxy-4"-(S)-(2-metylvinyl) Avermectin B₁ which is characterized by its mass and nmr spectra (compound 5c.12).

4"-Deoxy-4"-(S)-(2-methyl-vinyl) Avermectin B₁ : B₁ₐ C₅₁H₇₆O₁₃, MW: 896.5. LCMS: *t_{RT}*,: 13.47min., 919.5 (M+Na); ¹H-NMR (400 MHz, COCl₃) selected data, δH (ppm): 5.2 (dd, 1H, CH₃C(H)=CH) 1.7 (d, *J* = 7.5 Hz, 3H, CH₃C(H)=CH).

### Example P17: 4"-Deoxy-4"-(R)-(Acetic acid ethyl ester) Avermectin B₁

Step A To a solution of triethylphosphono acetate (620 µl) in dry THF, under Argon atmoshere is added LiHMDS (3.1 ml of a 1 M solution in THF) at 0°C. After stirring during one hour, a solution of 5-O-*tert*-butyldimethylsilyl-7-O-trimethylsilyl-4"-oxo Avermectin B₁ (3.0 g) in dry THF (20 ml) is added *via cannula.* The mixture is stirred at the same temperature during 2 hours then quenched by the addition of sat. NH₄Cl, extracted with ethyl acetate, washed with brine, dried and concentrated. Flash chromatography of the crude product (silica gel, hexane/ethyl acetate, 5/1) provided pure 5-O-*tert*-butyldimethylsilyl-7-O-trimethylsilyl-4"-deoxy-4"-*exo*-(carboxyethyl methylene) Avermectin B₁ which is characterized by its mass and nmr spectra.

Step B: To a solution of 5-O-*tert*-butyldimethylsilyl-7-O-trimethylsilyl-4"-deoxy-4"-exo-(carboxyethyl methylene) Avermectin B₁ (8.0 g) of step A in ethyl acetate (170 ml), at 0°C, under argon atmosphere, is added NaBH₄ (1.1 g) and NiCl₂ (90 mg). Dry methanol (60 ml) is slowly added (gas evolution) and the black mixture is stirred until no starting material is observed (TLC monitoring). The reaction is quenched by the addition of iced water, extracted with ethyl acetate, washed with sat. NH₄Cl and brine, dried and concentrated. Flash chromatography of the crude product (silica gel, hexane/ethyl acetate, 7/1) provided pure 5-O-*tert*-butyldimethylsilyl-7-O-trimethylsilyl-4"-deoxy-4"-(R)-(acetic acid ethyl ester) Avermectin B₁ which is characterized by its mass and nmr spectra.

5-O-tert-butyldimethylsilyl-7-O-trimethylsilyl-4"-deoxy-4"-(R)-(acetic acid ethyl ester) Avermectin B₁ : B₁ₐ C₆₁H₁₀₀O₁₅Si₂, MW: 1128.7. LCMS: *t_{RT}*,: 16.09 min., 1151.6 (M+Na);

Step C: 5-O-*tert*-butyldimethylsilyl-7-O-trimethylsilyl-4"-deoxy-4"-(R)-(acetic acid ethyl ester) Avermectin B₁ of step B is treated as described in Example P1, Step C affording after flash-chromatography (silica gel, hexane/ethyl acetate, 3/2) 4"-deoxy-4"-(R)-(acetic acid ethyl ester) Avermectin B₁ which is characterized by its mass and nmr spectra spectra (compound 10a.28).

4"-deoxy-4"-(R)-(acetic acid ethyl ester) Avermectin B₁ : B₁ₐ C₅₂H₇₈O₁₅, MW: 942.5 LCMS: *t_{RT}*,: 12.39 min., 965.5 (M+Na);

### Example P18: 5-O-tert-Butyldimethylsilyl-7-O-trimethylsilyl-4"-Deoxy-4"-(S)-Formyl Avermectin B1 and 5-O-tert-Butyldimethylsilyl-7-O-trimethylsilyl-4"-Deoxy-4"-(R)-Formyl Avermectin B1

Step A To a solution of diethylisocyanomethyl phosphonate (1.5 ml) in dry diethyl ether (40 ml), under Argon atmoshere is added BuLi (5.85 ml of a 1.6M solution in hexane) at - 78°C. After stirring during one hour, a solution of 5-O-tert-butyldimethylsilyl-7-O-trimethylsilyl-4"-oxo Avermectin B₁ (3.0 g) in dry THF (20 ml) is added *via cannula* at -78°C. The mixture is stirred at-78°C during 2 hours and then the temperature is allowed to increase to 0°C. The reaction is quenched by the addition of brine and diluted with diethyl ether. The organic phase is separated and washed with brine, dried and concentrated. Flash chromatography of the crude product (silica gel, hexane/ethyl acetate, 7/1) provided pure 5-O-*tert*-butyldimethylsilyl-7-O-trimethylsilyl-4"-deoxy-4"-*exo*-(isocyano methylene) Avermectin B₁ as a mixture of isomers which are characterized by their mass and nmr spectra.

5-O-*tert*-butyldimethylsilyl-7-O-trimethylsilyl-4"-deoxy-4"-*exo*-(isocyanomethylene) Avermectin B₁ : B₁ₐ C₅₉H₃₉NO₁₃Si₂ MW: 1079.6 LCMS: isomer A *t_{RT}*,: 15.25 min., 1102.6 (M+Na); isomer B *t_{RT}*,: 15.47 min., 1102.6 (M+Na); ¹H-NMR (500 MHz, CDCl₃) selected data, δH (ppm): less polar isomer (Z) 3.82 (m, 1 H, CH-3"), 4.82 (q, 1 H, CH-5"), 5.72 (s, 1 H, C=CH(NC)); more polar isomer (E) 4.44 (m, 1 H, CH-3"), 4.45 (q, 1 H, CH-5"), 5.81 (s, 1 H, C=CH(NC)).

Step B: To a solution of pure 5-O-*tert*-butyldimethylsilyl-7-O-trimethylsilyl-4"-deoxy-4"-*exo*-(isocyano methylene) Avermectin B₁ (3.0 g) of step A in dry benzene (50 ml), at room temperature, under argon atmosphere,is added Pb(OAc)₄ (1.6 g). After stirring during 2 hours the mixture is diluted with diethyl ether and the reaction quenched with a sat aq. solution of NaHCO₃. After extraction, the organic phase is washed with brine, dried and the solvent evaporated under reduced pressure. The crude product is dissolved in CH₂Cl₂ (50 ml) and neutral alumine (activity grade II-III) is added. After stirring during 4 hours the mixture is filtrated over *celite* and concentrated. Purification of the crude product by flash chromatography (silica gel, hexane/ethyl acetate, 5/1) provided an inseparable mixture of 5-O-*tert*-butyldimethylsilyl-7-O-trimethylsilyl-4"-deoxy-4"-(S)-formyl Avermectin B1 and 5-O-*tert*-butyldimethylsilyl-7-O-trimethylsilyl-4"-deoxy-4"-(R)-formyl Avermectin B1 isomers which are characterized by their mass and nmr spectra.

5-O-*tert*-butyldimethylsilyl-7-O-trimethylsilyl-4"-Deoxy-4"-(*S*)-formyl Avermectin B1 and 5-O-*tert*-butyldimethylsilyl-7-O-trimethylsilyl-4"-Deoxy-4"-(*R*)-formyl Avermectin B1 : B₁ₐ C₅₈H₉₄O₁₄Si₂ MW: 1070.6 LCMS: *t_{RT}*,: 15.31 min., 1093.6 (M+Na); ¹H-NMR (500 MHz, CDCl₃) selected data, δH (ppm): (*S*) isomer 9.82 (d, 1H, C(=O)H); (*R*) isomer 9.95 (d, 1 H, C(=O)H);

### Example P19: 4"-Deoxy-4"-(S)-(Hydroxymethyl)-Avermectin B₁

Step A: The above described mixture of 5-O-*tert*-butyldimethylsilyl-7-O-trimethylsilyl-4"-deoxy-4"-(*S*)-formyl Avermectin B1 and 5-O-*tert*-butyldimethylsilyl-7-O-trimethylsilyl-4"-deoxy-4"-(*R*)-formyl Avermectin B1 (Example P17, step B) (375 mg) is dissolved in a mixture of MeOH (8 ml) and H₂O (0.8 ml). At 0°C NaBH₄ (25 mg) is added. After 30 min the reaction is quenched by addition of sat aq. NH₄Cl and the organic phase extracted with ethyl acetate, then washed with brine, dried and concentated. The crude product is purified by flash chromatography (silica gel, hexane/tertbutylmethyl ether, 3/2) yielding 5-O-*tert-*butyldimethylsilyl-7-O-trimethylsilyl-4"-deoxy-4"-(*S*)-(hydroxymethyl)-avermectin B₁ and 5-O-*tert*-butyldimethylsilyl-7-O-trimethylsilyl-4"-deoxy-4"-(*R*)-(hydroxymethyl)-avermectin B₁ which are characterized by their mass and nmr spectra.

Step B 5-O-*tert*-butyldimethylsilyl-7-O-trimethylsilyl-4"-deoxy-4"-(*S*)-(hydroxymethyl)-Avermectin B₁ (75 mg) of step A is treated as described in Example P1, Step C affording after flash-chromatography (silica gel, hexane/ethyl acetate, 1/1) 4"-deoxy-4"-(*R*)-(hydroxymethyl) Avermectin B₁ which is characterized by its mass and nmr spectra (compound 2a.14).

4"-deoxy-4"-(*S*)-(hydroxymethyl)-Avermectin B₁ : B₁ₐ C₄₉H₇₄O₁₄, MW: 886.5 LCMS: *t_{RT}*,: 8.53 min., 909.5 (M+Na). ¹H-NMR (500 MHz, CDCl₃) selected data, δH (ppm): 1.54 (m, 1 H, CH-4"), 2.95 (br d, 1 H, CH₂OH), 3.40 (s, 3H, OCH₃), 3.44 (s, 3H, OCH₃), 3.63 (m, 1 H, CH₂OH), 3.82 (m, 1 H, CH₂OH).

Similarly to the preparation examples above it is also possible to prepare the corresponding compounds listed in Tables 1 to 8. In addition, the remaining compounds listed in Tables 1 to 9 may be prepared by methods known to those skilled in the art.

Since in most cases the compounds are present as mixtures of the avermectin derivatives B1 a and B1 b, characterization by customary physical data such as melting point or refractive index makes little sense. For this reason, the compounds are characterized by the retention times which are determined in an analysis by HPLC (high performance liquid chromatography). Here, the term B1a refers to the main component in which R₁ is sec-butyl, with a content of usually more than 80%. B1b denotes the minor component in which R₁ is isopropyl. The compounds where two retention times are given both for the B1a and for the B1b derivative are mixtures of diastereomers which can be separated chromatographically. In the case of compounds where a retention time is given only in column B1 a or only in column B1 b, the pure B1 a or B1 b component, respectively, can be obtained during work-up. The correct structures of the B1a and B1b components are assigned by mass spectrometry.
The following method is used for HPLC analysis:

| HPLC gradient conditions | | | |
|---|---|---|---|
| Solvent A: | 0.01 % of trifluoroacetic acid in H₂O | | |
| Solvent B: | 0.01 % of trifluoroacetic acid in CH₃CN | | |

| Time [min] | A [%] | B [%] | Flow rate [µl/min] |
|---|---|---|---|
| 0 | 80 | 20 | 500 |
| 0.1 | 50 | 50 | 500 |
| 10 | 5 | 95 | 500 |
| 15 | 0 | 100 | 500 |
| 17 | 0 | 100 | 500 |
| 17.1 | 80 | 20 | 500 |
| 22 | 80 | 20 | 500 |
| Type of column | YMC-Pack ODS-AQ | | |
| Column length | 125 mm | | |
| Internal diameter of column: | 2 mm | | |
| Temperature | 40°C | | |

The YMC-Pack ODS-AQ column used for the chromatography of the compounds is manufactured by YMC, Alte Raesfelderstrasse 6, 46514 Schermbeck, Germany.

**Table 1: Compounds of the formula**

| | | | | | | | | |
|---|---|---|---|---|---|---|---|---|
| 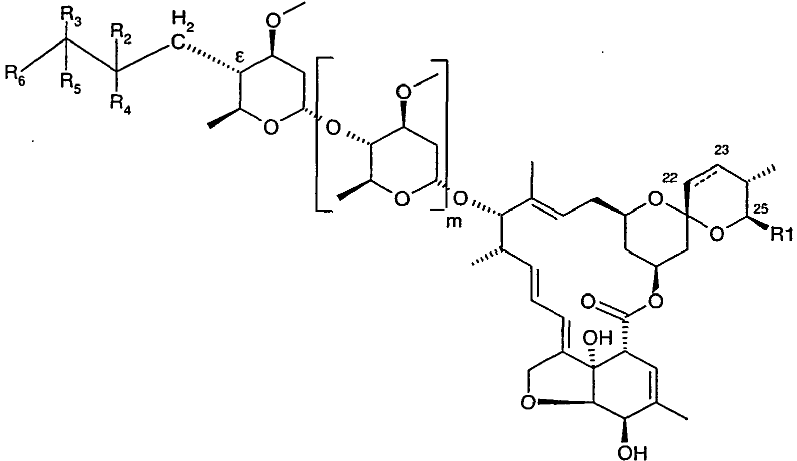 | | | | | | | | |
| in which R₁ is sec-butyl (B1a) or isopropyl (B1b), and the bond between C₂₂ and C₂₃ is a double bond | | | | | | | | |

| No. | m | R₂ | R₃ | R₄ | R₅ | R₆ | Retent. time (min) | |
|---|---|---|---|---|---|---|---|---|
| | | | | | | | B1a | B1b |
| 1.1 | 1 | H | H | H | H | OH | 8.64 | |
| 1.2 | 0 | H | H | H | H | OH | 7.68 | 6.99 |
| 1.3 | 1 | OH | H | H | H | OH | | |
| 1.4 | 0 | OH | H | H | H | OH | | |
| 1.5 | 1 | H | CH₃ | H | H | OH | | |
| 1.6 | 0 | H | CH₃ | H | H | OH | | |
| 1.7 | 1 | H | CF₃ | H | H | OH | | |
| 1.8 | 0 | H | CF₃ | H | H | OH | | |
| 1.9 | 1 | H | CF₃ | H | CH₃ | OH | | |
| 1.10 | 0 | H | CF₃ | H | CH₃ | OH | | |
| 1.11 | 1 | H | CH₃ | H | CH₃ | OH | | |
| 1.12 | 0 | H | CH₃ | H | CH₃ | OH | | |
| 1.13 | 0 | H | H | H | H | OC(=O)NH₂ | | |
| 1.14 | 1 | H | H | H | H | OC(=O)NHMe | | |
| 1.15 | 0 | H | H | H | H | OC(=O)NHMe | | |
| 1.16 | 1 | H | H | H | H | OC(=S)NHMe | | |
| 1.17 | 0 | H | H | H | H | OC(=S)NHMe | | |
| 1.18 | 1 | H | H | H | H | C(=O)CF₃ | | |
| 1.19 | 0 | H | H | H | H | C(=O)CF₃ | | |
| 1.20 | 1 | H | H | H | H | OC(=O)CH₃ | | |
| 1.21 | 0 | H | H | H | H | OC(=O)CH₃ | | |
| 1.22 | 1 | H | H | H | H | SC(=O)CH₃ | | |
| 1.23 | 0 | H | H | H | H | SC(=O)CH₃ | | |
| 1.24 | 1 | H | H | H | H | OCH₃ | | |
| 125 | 0 | H | H | H | H | OCH₃ | | |
| 126 | 1 | H | H | H | H | OCH₂OCH₃ | | |
| 127 | 0 | H | H | H | H | OCH₂OCH₃ | 11.09 | 10.24 |
| 128 | 1 | H | H | H | H | ONH₂ | | |
| 1.29 | 0 | H | H | H | H | ONH₂ | | |
| 1.30 | 1 | H | H | H | H | N₃ | | |
| 1.31 | 0 | H | H | H | H | N₃ | 11.79 | 11.15 |
| 1.32 | 1 | H | H | H | H | NHCHO | | |
| 1.33 | 0 | H | H | H | H | NHCHO | 8.37 | |
| 1.34 | 1 | H | H | H | H | NHC(=O)CH₃ | | |
| 1.35 | 0 | H | H | H | H | NHC(=O)CH₃ | 8.32 | |
| 1.36 | 1 | H | H | H | H | NHC(=O)CH₂OCH₃ | | |
| 1.37 | 0 | H | H | H | H | NHC(=O)CH₂OCH₃ | 9.07 | |
| 1.38 | 1 | H | H | H | H | NH₂ | | |
| 1.39 | 0 | H | H | H | H | NH₂ | 5.12 | |
| 1.40 | 1 | H | H | H | H | NHCH₂C(=O)CH₃ | | |
| 1.41 | 0 | H | H | H | H | NHCH₂C(=O)CH₃ | | |
| 1.42 | 1 | H | H | H | H | NHOH | | |
| 1.43 | 0 | H | H | H | H | NHOH | | |
| 1.44 | 1 | H | H | H | H | NHOCH₃ | | |
| 1.45 | 0 | H | H | H | H | NHOCH₃ | | |
| 1.46 | 1 | H | H | H | H | N(CH₃)OH | | |
| 1.47 | 0 | H | H | H | H | N(CH₃)OH | | |
| 1.48 | 1 | H | H | H | H | N(CH₃)OH | | |
| 1.49 | 0 | H | H | H | H | N(CH₃)OH | | |
| 1.50 | 1 | H | H | H | H | NHC(=O)OCH₃ | | |
| 1.51 | 0 | H | H | H | H | NHC(=O)OCH₃ | | |
| 1.52 | 1 | H | H | H | H | Cl | | |
| 1.53 | 0 | H | H | H | H | Cl | | |

**Table 2: Compounds of the formula**

| | | | | | | | |
|---|---|---|---|---|---|---|---|
| 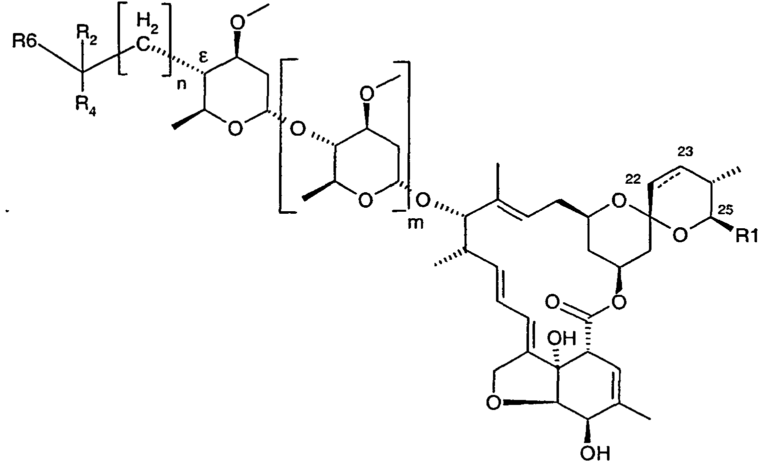 | | | | | | | |
| in which R₁ is sec-butyl (B1a) or isopropyl (B1b), and the bond between C₂₂ and C₂₃ is a double bond | | | | | | | |

| No. | m | n | R₂ | R₄ | R₆ | Retent. time (min) | |
|---|---|---|---|---|---|---|---|
| | | | | | | B1a | B1b |
| 2.1 | 1 | 1 | H | H | OH | 8.71 | |
| 2.2 | 0 | 1 | H | H | OH | | |
| 2.3 | 1 | 0 | H | H | OH | 8.55 | 7.84 |
| 2.4 | 0 | 0 | H | H | OH | | |
| 2.5 | 1 | 1 | H | CH₃ | OH | 12.36/ 11.98 | |
| 2.6 | 0 | 1 | H | CH₃ | OH | | |
| 2.7 | 1 | 0 | H | CH₃ | OH | 9.87 | |
| 2.8 | 0 | 0 | H | CH₃ | OH | | |
| 2.9 | 1 | 1 | H | CF₃ | OH | | |
| 2.10 | 0 | 1 | H | CF₃ | OH | | |
| 2.11 | 1 | 0 | H | CF₃ | OH | | |
| 2.12 | 0 | 0 | H | CF₃ | OH | | |
| 2.13 | 1 | 1 | CH₃ | CF₃ | OH | | |
| 2.14 | 0 | 1 | CH₃ | CF₃ | OH | | |
| 2.15 | 1 | 0 | CH₃ | CF₃ | OH | | |
| 2.16 | 0 | 0 | CH₃ | CF₃ | OH | | |
| 2.17 | 1 | 1 | CH₃ | CH₃ | OH | 13.46 | |
| 2.18 | 0 | 1 | CH₃ | CH₃ | OH | | |
| 2.19 | 1 | 0 | CH₃ | CH₃ | OH | | |
| 2.20 | 0 | 0 | CH₃ | CH₃ | OH | | |
| 2.21 | 1 | 0 | CO₂CH₃ | CO₂CH₃ | H | | |
| 2.22 | 0 | 0 | CO₂CH₃ | CO₂CH₃ | H | | |
| 2.23 | 1 | 0 | CH₂OH | CH₂OH | H | | |
| 2.24 | 0 | 0 | CH₂OH | CH₂OH | H | | |
| 2.25 | 1 | 0 | CH₃ | CH₃ | H | | |
| 2.26 | 0 | 0 | CH₃ | CH₃ | H | | |
| 2.27 | 1 | 1 | H | H | H | | |
| 2.28 | 0 | 1 | H | H | H | | |
| 2.29 | 1 | 1 | H | H | SH | | |
| 2.30 | 1 | 0 | H | H | SH | | |
| 2.31 | 0 | 1 | H | H | SH | | |
| 2.32 | 0 | 0 | H | H | SH | | |
| 2.33 | 1 | 1 | H | H | SCH₃ | | |
| 2.34 | 1 | 0 | H | H | SCH₃ | | |
| 2.35 | 0 | 1 | H | H | SCH₃ | | |
| 2.36 | 0 | 0 | H | H | SCH₃ | | |
| 2.37 | 1 | 1 | H | H | S(O)CH₃ | | |
| 2.38 | 1 | 0 | H | H | S(O)CH₃ | | |
| 2.39 | 0 | 1 | H | H | S(O)CH₃ | | |
| 2.40 | 0 | 0 | H | H | S(O)CH₃ | | |
| 2.41 | 1 | 1 | H | H | S(O)₂CH₃ | | |
| 2.42 | 1 | 0 | H | H | S(O)₂CH₃ | | |
| 2.43 | 0 | 1 | H | H | S(O)₂CH₃ | | |
| 2.44 | 0 | 0 | H | H | S(O)₂CH₃ | | |
| 2.45 | 1 | 1 | H | H | SC(=O)CH₃ | | |
| 2.46 | 1 | 0 | H | H | SC(=O)CH₃ | | |
| 2.47 | 0 | 1 | H | H | SC(=O)CH₃ | | |
| 2.48 | 0 | 0 | H | H | SC(=O)CH₃ | | |
| 2.49 | 1 | 1 | H | H | N₃ | | |
| 2.50 | 1 | 0 | H | H | N₃ | | |
| 2.51 | 0 | 1 | H | H | N₃ | | |
| 2.52 | 0 | 0 | H | H | N₃ | | |
| 2.53 | 1 | 1 | H | H | OC(=O)CH₃ | | |
| 2.54 | 1 | 0 | H | H | OC(=O)CH₃ | | |
| 2.55 | 0 | 1 | H | H | OC(=O)CH₃ | | |
| 2.56 | 0 | 0 | H | H | OC(=O)CH₃ | | |
| 2.57 | 1 | 1 | H | H | OCH₂OCH₃ | | |
| 2.58 | 1 | 0 | H | H | OCH₂OCH₃ | | |
| 2.59 | 0 | 1 | H | H | OCH₂OCH₃ | | |
| 2.60 | 0 | 0 | H | H | OCH₂OCH₃ | | |
| 2.61 | 1 | 1 | H | H | OS(O)₂NH₂ | | |
| 2.62 | 1 | 0 | H | H | OS(O)₂NH₂ | | |
| 2.63 | 0 | 1 | H | H | OS(O)₂NH₂ | | |
| 2.64 | 0 | 0 | H | H | OS(O)₂NH₂ | | |
| 2.65 | 1 | 1 | H | H | OC(=O)NH₂ | | |
| 2.66 | 1 | 0 | H | H | OC(=O)NH₂ | | |
| 2.67 | 0 | 1 | H | H | OC(=O)NH₂ | | |
| 2.68 | 0 | 0 | H | H | OC(=O)NH₂ | | |
| 2.69 | 1 | 1 | H | H | OC(=O)NHMe | | |
| 2.70 | 1 | 0 | H | H | OC(=O)NHMe | | |
| 2.71 | 0 | 1 | H | H | OC(=O)NHMe | | |
| 2.72 | 0 | 0 | H | H | OC(=O)NHMe | | |
| 2.73 | 1 | 1 | H | H | OC(=S)NHMe | | |
| 2.74 | 1 | 0 | H | H | OC(=S)NHMe | | |
| 2.75 | 1 | 1 | H | H | C(=O)OH | | |
| 2.76 | 1 | 0 | H | H | C(=O)OH | | |
| 2.77 | 0 | 0 | H | H | C(=O)OH | 8.38 | |
| 2.78 | 1 | 1 | H | H | C(=O)NH₂ | | |
| 2.79 | 1 | 0 | H | H | C(=O)NH₂ | | |
| 2.80 | 0 | 1 | H | H | C(=O)NH₂ | | |
| 2.81 | 0 | 0 | H | H | C(=O)NH₂ | | |
| 2.82 | 1 | 1 | H | H | N(CH₃)₂ | | |
| 2.83 | 1 | 0 | H | H | N(CH₃)₂ | 5.16 | |
| 2.84 | 0 | 1 | H | H | N(CH₃)₂ | | |
| 2.85 | 0 | 0 | H | H | N(CH₃)₂ | | |
| 2.86 | 1 | 1 | H | H | NHCH₃ | | |
| 2.87 | 1 | 0 | H | H | NHCH₃ | | |
| 2.88 | 0 | 1 | H | H | NHCH₃ | | |
| 2.89 | 0 | 0 | H | H | NHCH₃ | | |
| 2.90 | 1 | 1 | H | H | NHCHO | | |
| 2.91 | 1 | 0 | H | H | NHCHO | | |
| 2.92 | 0 | 1 | H | H | NHCHO | | |
| 2.93 | 0 | 0 | H | H | NHCHO | | |
| 2.94 | 1 | 1 | H | H | NHC(=O)CH₃ | | |
| 2.95 | 1 | 0 | H | H | NHC(=O)CH₃ | | |
| 2.96 | 0 | 1 | H | H | NHC(=O)CH₃ | | |
| 2.97 | 0 | 0 | H | H | NHC(=O)CH₃ | | |
| 2.98 | 1 | 1 | H | H | NHC(=O)CH₂OCH₃ | | |
| 2.99 | 1 | 0 | H | H | NHC(=O)CH₂OCH₃ | | |
| 2.100 | 0 | 1 | H | H | NHC(=O)CH₂OCH₃ | | |
| 2.101 | 0 | 0 | H | H | NHC(=O)CH₂OCH₃ | | |
| 2.102 | 1 | 1 | H | H | NH₂ | | |
| 2.103 | 1 | 0 | H | H | NH₂ | | |
| 2.104 | 0 | 1 | H | H | NH₂ | | |
| 2.105 | 0 | 0 | H | H | NH₂ | | |
| 2.106 | 1 | 1 | H | H | ONH₂ | | |
| 2.107 | 1 | 0 | H | H | ONH₂ | | |
| 2.108 | 0 | 1 | H | H | ONH₂ | | |
| 2.109 | 0 | 0 | H | H | ONH₂ | | |
| 2.110 | 1 | 1 | H | H | NHOH | | |
| 2.111 | 1 | 0 | H | H | NHOH | | |
| 2.112 | 0 | 1 | H | H | NHOH | | |
| 2.113 | 0 | 0 | H | H | NHOH | | |
| 2.114 | 1 | 1 | H | H | N(CH₃)OH | | |
| 2.115 | 1 | 0 | H | H | N(CH₃)OH | | |
| 2.116 | 0 | 1 | H | H | N(CH₃)OH | | |
| 2.117 | 0 | 0 | H | H | N(CH₃)OH | | |
| 2.118 | 1 | 1 | H | H | NHC(=O)OCH₃ | | |
| 2.119 | 1 | 0 | H | H | NHC(=O)OCH₃ | | |
| 2.120 | 0 | 1 | H | H | NHC(=O)OCH₃ | | |
| 2.121 | 0 | 0 | H | H | NHC(=O)OCH₃ | | |
| 2.122 | 1 | 1 | H | H | CN | | |
| 2.123 | 1 | 0 | H | H | CN | | |
| 2.124 | 0 | 1 | H | H | CN | | |
| 2.125 | 0 | 0 | H | H | CN | | |
| 2.126 | 1 | 1 | H | H | OCH₃ | | |
| 2.127 | 0 | 1 | H | H | OCH₃ | | |
| 2.128 | 1 | 0 | H | H | OCH₃ | | |
| 2.129 | 0 | 0 | H | H | OCH₃ | | |
| 2.130 | 1 | 1 | H | H | OCH₂SCH₃ | | |
| 2.131 | 0 | 1 | H | H | OCH₂SCH₃ | | |
| 2.132 | 1 | 0 | H | H | OCH₂SCH₃ | | |
| 2.133 | 0 | 0 | H | H | OCH₂SCH₃ | | |
| 2.134 | 1 | 1 | H | H | OCH₂OCH₂C₅H₆ | | |
| 2.135 | 0 | 1 | H | H | OCH₂OCH₂C₅H₆ | | |
| 2.136 | 1 | 0 | H | H | OCH₂OCH₂C₅H₆ | | |
| 2.137 | 0 | 0 | H | H | OCH₂OCH₂C₅H₆ | | |
| 2.138 | 1 | 1 | H | H | OCH₂O(CH₂)₂OCH₃ | | |
| 2.139 | 0 | 1 | H | H | OCH₂O(CH₂)₂OCH₃ | | |
| 2.140 | 1 | 0 | H | H | OCH₂O(CH₂)₂OCH₃ | | |
| 2.141 | 0 | 0 | H | H | OCH₂O(CH₂)₂OCH₃ | | |
| 2.142 | 1 | 1 | H | H | NHCH₂C(=O)CH₃ | | |
| 2.143 | 0 | 1 | H | H | NHCH₂C(=O)CH₃ | | |
| 2.144 | 1 | 0 | H | H | NHCH₂C(=O)CH₃ | | |
| 2.145 | 0 | 0 | H | H | NHCH₂C(=O)CH₃ | | |
| 2.146 | 1 | 1 | OCH₃ | OCH₃ | H | | |
| 2.147 | 1 | 0 | OCH₃ | OCH₃ | H | | |
| 2.148 | 0 | 1 | OCH₃ | OCH₃ | H | | |
| 2.149 | 0 | 0 | OCH₃ | OCH₃ | H | | |
| 2.150 | 0 | 1 | H | H | CHO | 9.97 | |
| 2.151 | 0 | 1 | H | H | CH(=NOH) | 8.96 | |
| 2.152 | 0 | 1 | H | H | CH(=NOCH₃) | 11.15 | 10.29 |
| 2.153 | 1 | 1 | CH₂CH₃ | Et | OH | | |
| 2.154 | 1 | 1 | CH₂CH=CH₂ | H | OH | | |
| 2.155 | 1 | 0 | H | H | CH(=NOH) | | |
| 2.156 | 1 | 0 | H | H | CH(=NOCH₃) | | |
| 2.157 | 1 | 1 | H | H | OC(=O)NHCH₃ | | |
| 2.158 | 1 | 1 | H | H | 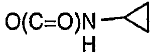 | | |
| 2.159 | 1 | 1 | H | H | NHC(=O)CH(CH₃)₂ | | |
| 2.160 | 1 | 1 | H | =N NHC(=O)OCH₃ | | | |
| 2.161 | 1 | 1 | H | =N NHS(=O)₂CH₃ | | | |
| 2.162 | 1 | 0 | H | H | NHC(=O)OC(CH₃)₃ | | |
| 2.163 | 1 | 1 | H | H | OCH₂O(CH₂)₃CH₃ | | |
| 2.164 | 1 | 1 | H | H | OC(=O)OCH₃ | | |
| 2.165 | 1 | 1 | H | H | O(=O)CH₂OCH₃ | | |
| 2.166 | 1 | 1 | H | H | O(=O)OCH₂C≡CH | | |
| 2.167 | 1 | 0 | H | H | C(=O)H | | |
| 2.168 | 1 | 0 | H | H | C(=O)OCH₂CH₃ | | |

**Table 3: Compounds of the formula**

| | | | | | | | | |
|---|---|---|---|---|---|---|---|---|
| 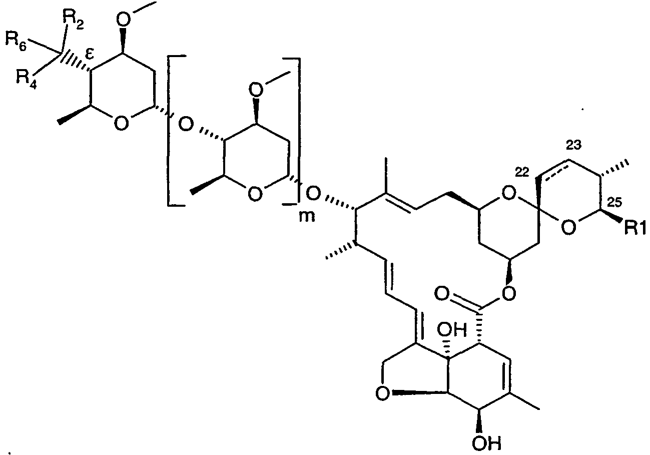 | | | | | | | | |
| in which R₁ is sec-butyl (B1a) or isopropyl (B1b), and the bond between C₂₂ and C₂₃ is a double bond | | | | | | | | |

| No. | m | R₂ | R₄ | R₆ | | | | |
|---|---|---|---|---|---|---|---|---|
| 3.1 | 1 | CO₂CH₃ | CO₂CH₃ | H | | | | |
| 3.2 | 1 | CO₂C₆H₅ | CO₂C₆H₅ | H | | | | |
| 3.3 | 1 | CO₂CH₂CH₃ | CO₂CH₂CH₃ | H | | | | |
| 3.4 | 1 | SO₂C₆H₅ | SO₂C₆H₅ | H | | | | |
| 3.5 | 1 | CN | CN | H | | | | |
| 3.6 | 1 | C(=O)CH₃ | C(=O)CH₃ | H | | | | |
| 3.7 | 1 | C(=O)Ph | C(=O)c₆H₅ | H | | | | |
| 3.8 | 1 | C(=O)CH₃ | CO₂CH₃ | H | | | | |
| 3.9 | 1 | C(=O)CH₃ | CO₂CH₂CH₃ | H | | | | |
| 3.10 | 1 | C(=O)CH₃ | CONHC₆H₅ | H | | | | |
| 3.11 | 1 | C(=O)CH₃ | CONHC(CH₃)₃ | H | | | | |
| 3.12 | 1 | C(=O)CH₂CH₃ | CO₂CH₃ | H | | | | |
| 3.13 | 1 | C(=O)CH₂CH₃ | CONHC₆H₅ | H | | | | |
| 3.14 | 1 | C(=O)CH₂CH₃ | CONHC(CH₃)₃ | H | | | | |
| 3.15 | 1 | CN | CO₂CH₃ | H | | | | |
| 3.16 | 1 | CN | CO₂CH₂CH₃ | H | | | | |
| 3.17 | 1 | CN | CONHC₆H₅ | H | | | | |
| 3.18 | 1 | NO₂ | H | H | | | | |
| 3.19 | 1 | C(=O)CH₃ | SC₆H₅ | H | | | | |
| 3.20 | 1 | C(=O)CH₃ | SC₆H₅ | H | | | | |
| 3.21 | 1 | C(=O)SCH₂CH₃ | C(=O)SCH₂CH₃ | H | | | | |
| 3.22 | 1 | (O=)COC(CH₃)₂OC(=O) | | H | | | | |
| 3.23 | 1 | CN | SO₂C₆H₅ | H | | | | |
| 3.24 | 1 | C(=O)CF₃ | CO₂CH₂CH₃ | H | | | | |
| 3.25 | 1 | CO₂CH₂CH₃ | C(=O)CH₂OCH₃ | H | | | | |
| 3.26 | 1 | ≡N | | | | | | |
| 3.27 | 0 | CO₂CH₃ | CO₂CH₃ | H | | | | |
| 3.28 | 0 | CO₂C₆H₅ | CO₂C₆H₅ | H | | | | |
| 3.29 | 0 | CO₂CH₂CH₃ | CO₂CH₂CH₃ | H | | | | |
| 3.30 | 0 | SO₂C₆H₅ | SO₂C₆H₅ | H | | | | |
| 3.31 | 0 | CN | CN | H | | | | |
| 3.32 | 0 | C(=O)CH₃ | C(=O)CH₃ | H | | | | |
| 3.33 | 0 | C(=O)Ph | C(=O)C₆H₅ | H | | | | |
| 3.34 | 0 | C(=O)CH₃ | CO₂CH₃ | H | | | | |
| 3.35 | 0 | C(=O)CH₃ | CO₂CH₂CH₃ | H | | | | |
| 3.36 | 0 | C(=O)CH₃ | CONHC₆H₅ | H | | | | |
| 3.37 | 0 | C(=O)CH₃ | CONHC(CH₃)₃ | H | | | | |
| 3.38 | 0 | C(=O)CH₂CH₃ | CO₂CH₃ | H | | | | |
| 3.39 | 0 | C(=O)CH₂CH₃ | CONHC₆H₅ | H | | | | |
| 3.40 | 0 | C(=O)CH₂CH₃ | CONHC(CH₃)₃ | H | | | | |
| 3.41 | 0 | CN | CO₂CH₃ | H | | | | |
| 3.42 | 0 | CN | CO₂CH₂CH₃ | H | | | | |
| 3.43 | 0 | CN | CONHC₆H₅ | H | | | | |
| 3.44 | 0 | NO₂ | H | H | | | | |
| 3.45 | 0 | C(=O)CH₃ | SC₆H₅ | H | | | | |
| 3.46 | 0 | C(=O)CH₃ | SC₆H₅ | H | | | | |

| No. | m | R₂ | | R₄ | | | R₆ | |
|---|---|---|---|---|---|---|---|---|
| 3.47 | 0 | C(=O)SCH₂CH₃ | | C(=O)SCH₂CH₃ | | | H | |
| 3.48 | 0 | (O=)COC(CH₃)_{2O}C(=O) | | | | | H | |
| 3.49 | 0 | CN | | SO₂C₆H₅ | | | H | |
| 3.50 | 0 | C(=O)CF₃ | | CO₂CH₂CH₃ | | | H | |
| 3.51 | 0 | CO₂CH₂CH₃ | | C(=O)CH₂OCH₃ | | | H | |
| 3.52 | 0 | | | ≡N | | | | |
| 3.53 | 1 | OH | | H | | | H | |
| 3.54 | 1 | OH | | CH₃ | | | H | |
| 3.55 | 1 | =NNHC(=O)OCH₃ | | | | | H | |
| 3.56 | 1 | =O | | | | | H | |
| | | | | | | | | |

| No. | m | R₂ | R₆ | | R₄ | Retention time (min) | | |
|---|---|---|---|---|---|---|---|---|
| | | | | | | B1a | | B1b |
| 3.2 | 1 | CO₂C₆H₅ | CO₂C₆H₅ | | H | 9.89 | | |
| 3.26 | 1 | ≡N | | | | 10.17 | | |
| 3.55 | 1 | =NNHC(=O)OCH₃ | | | H | 9.63 | | |

**Table 4: Compounds of the formula**

| | | | | | |
|---|---|---|---|---|---|
| 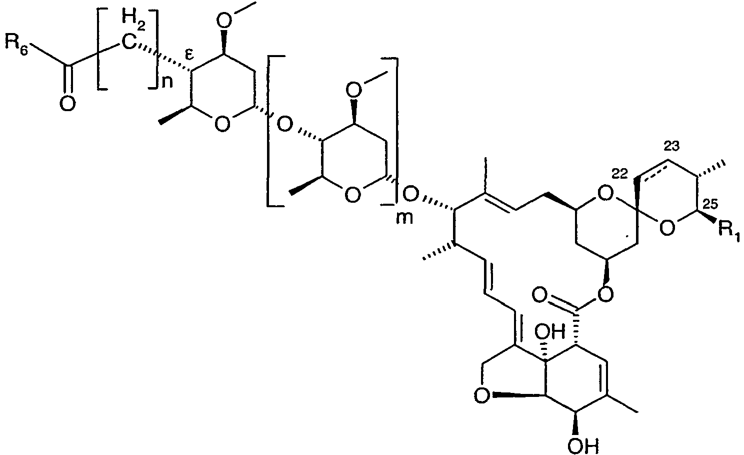 | | | | | |
| in which R₁ is sec-butyl (B1a) or isopropyl (B1b), and the bond between C₂₂ and C₂₃ is a double bond | | | | | |

| No. | m | n | R₆ | Retention time (min) | |
|---|---|---|---|---|---|
| | | | | B1a | B1b |
| 4.1 | 1 | 1 | H | 9.81 | |
| 4.2 | 0 | 1 | H | | |
| 4.3 | 1 | 0 | H | | |
| 4.4 | 0 | 0 | H | | |
| 4.5 | 1 | 2 | H | | |
| 4.6 | 0 | 2 | H | | |
| 4.7 | 1 | 1 | CH₃ | 10.56 | |
| 4.8 | 0 | 1 | CH₃ | 8.55 | 7.84 |
| 4.9 | 1 | 0 | CH₃ | 11.42 | 10.67 |
| 4.10 | 0 | 0 | CH₃ | | |
| 4.11 | 1 | 2 | CH₃ | | |
| 4.12 | 0 | 2 | CH₃ | | |
| 4.13 | 1 | 1 | CH₂CH₃ | | |
| 4.14 | 0 | 1 | CH₂CH₃ | | |
| 4.15 | 1 | 0 | CH₂CH₃ | | |
| 4.16 | 0 | 0 | CH₂CH₃ | | |
| 4.17 | 1 | 2 | CH₂CH₃ | | |
| 4.18 | 0 | 2 | CH₂CH₃ | | |
| 4.19 | 1 | 0 | OH | | |
| 4.20 | 0 | 0 | OH | | |
| 4.21 | 1 | 1 | OH | | |
| 4.22 | 0 | 1 | OH | | |
| 4.23 | 1 | 2 | OH | | |
| 4.24 | 0 | 2 | OH | | |
| 4.25 | 1 | 0 | NH₂ | | |
| 4.26 | 1 | 1 | NH₂ | | |
| 4.27 | 0 | 0 | NH₂ | | |
| 4.28 | 0 | 1 | NH₂ | | |
| 4.29 | 1 | 2 | NH₂ | | |
| 4.30 | 0 | 2 | NH₂ | | |
| 4.31 | 1 | 0 | OCH₃ | | |
| 4.32 | 0 | 0 | OCH₃ | | |
| 4.33 | 1 | 1 | OCH₃ | | |
| 4.34 | 0 | 1 | OCH₃ | | |
| 4.35 | 1 | 2 | OCH₃ | | |
| 4.36 | 0 | 2 | OCH₃ | | |
| 4.37 | 1 | 0 | NHCH₃ | | |
| 4.38 | 0 | 0 | NHCH₃ | | |
| 4.39 | 1 | 1 | NHCH₃ | | |
| 4.40 | 0 | 1 | NHCH₃ | | |
| 4.41 | 1 | 2 | NHCH₃ | | |
| 4.42 | 0 | 2 | NHCH₃ | | |
| 4.43 | 1 | 0 | CH₂CH=CH₂ | | |
| 4.44 | 0 | 0 | CH₂CH=CH₂ | | |
| 4.45 | 1 | 1 | CH₂CH=CH₂ | | |
| 4.46 | 0 | 1 | CH₂CH=CH₂ | | |
| 4.47 | 1 | 2 | CH₂CH=CH₂ | | |
| 4.48 | 0 | 2 | CH₂CH=CH₂ | | |
| 4.49 | 1 | 0 | OCH₂CH₂OCH₃ | | |
| 4.50 | 0 | 0 | OCH₂CH₂OCH₃ | | |
| 4.51 | 1 | 1 | OCH₂CH₂OCH₃ | | |
| 4.52 | 0 | 1 | OCH₂CH₂OCH₃ | | |
| 4.53 | 1 | 0 | OC₆H₅ | | |
| 4.54 | 0 | 0 | OC₆H₅ | | |
| 4.55 | 1 | 1 | OC₆H₅ | | |
| 4.56 | 0 | 1 | OC₆H₅ | | |
| 4.57 | 1 | 0 | OC₆H₅ | | |
| 4.58 | 0 | 0 | OC₆H₅ | | |
| 4.59 | 1 | 1 | OC₆H₅ | | |
| 4.60 | 0 | 1 | OC₆H₅ | | |
| 4.61 | 1 | 0 | 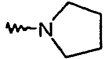 | | |
| 4.62 | 0 | 0 | 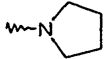 | | |
| 4.63 | 1 | 1 | 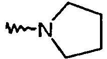 | | |
| 4.64 | 0 | 1 | 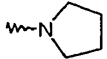 | | |
| 4.65 | 1 | 0 | 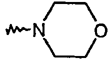 | | |
| 4.66 | 0 | 0 | 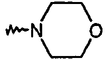 | | |
| 4.67 | 1 | 1 | 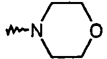 | | |
| 4.68 | 0 | 1 | 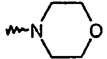 | | |
| 4.69 | 1 | 0 | F | | |
| 4.70 | 0 | 0 | F | | |
| 4.71 | 1 | 1 | F | | |
| 4.72 | 0 | 1 | F | | |
| 4.73 | 1 | 2 | F | | |
| 4.74 | 0 | 2 | F | | |
| 4.75 | 1 | 0 | Cl | | |
| 4.76 | 0 | 0 | Cl | | |
| 4.77 | 1 | 1 | Cl | | |
| 4.78 | 0 | 1 | Cl | | |
| 4.79 | 1 | 2 | Cl | | |
| 4.80 | 0 | 2 | Cl | | |
| 4.81 | 1 | 0 | CF₃ | | |
| 4.82 | 0 | 0 | CF₃ | | |
| 4.83 | 1 | 1 | CF₃ | | |
| 4.84 | 0 | 1 | CF₃ | | |
| 4.85 | 1 | 2 | CF₃ | | |
| 4.86 | 0 | 2 | CF₃ | | |

**Table 5A: Compounds of the formula**

| | | | | | |
|---|---|---|---|---|---|
| 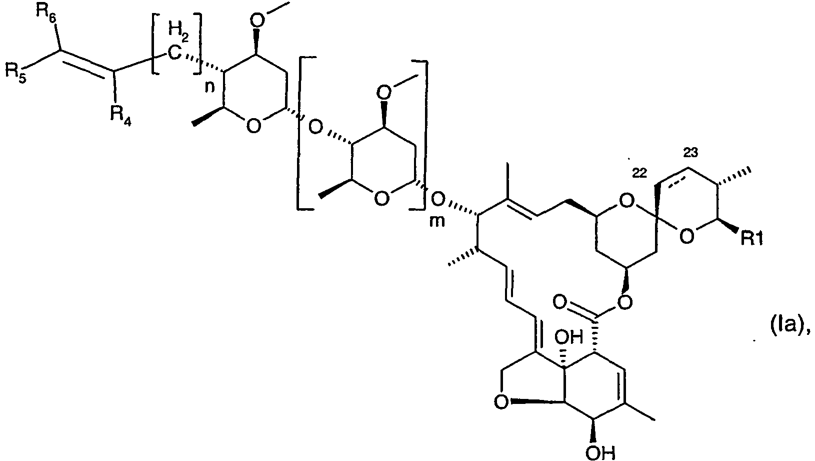 | | | | | |
| in which R₁ is sec-butyl (B1a) or isopropyl (B1b), and the bond between C₂₂ and C₂₃ is a double bond. | | | | | |

| No. | m | n | R₄ | R₅ | R₆ |
|---|---|---|---|---|---|
| 5A.1 | 1 | 1 | H | H | H |
| 5A.2 | 0 | 1 | H | H | H |
| 5A.3 | 1 | 2 | H | H | H |
| 5A.4 | 0 | 2 | H | H | H |
| 5A.5 | 1 | 0 | H | H | C₆H₅ |
| 5A.6 | 0 | 0 | H | H | C₆H₅ |
| 5A.7 | 1 | 1 | H | H | CH₂OH |
| 5A.8 | 0 | 1 | H | H | CH₂OH |
| 5A.9 | 1 | 1 | H | H | CH₂OCH₂OCH₃ |
| 5A.10 | 0 | 1 | H | H | CH₂OCH₂OCH₃ |
| 5A.11 | 1 | 1 | H | H | CO₂CH₃ |
| 5A.12 | 0 | 1 | H | H | CO₂CH₃ |
| 5A.13 | 1 | 1 | H | H | CO₂H |
| 5A.14 | 0 | 1 | H | H | CO₂H |
| 5A.15 | 1 | 1 | H | H | CHO |
| 5A.16 | 0 | 1 | H | H | CHO |
| 5A.17 | 1 | 1 | H | H | CH₂NHCH₃ |
| 5A.18 | 0 | 1 | H | H | CH₂NHCH₃ |
| 5A.19 | 1 | 1 | H | H | CN |
| 5A.20 | 0 | 1 | H | H | CN |
| 5A.21 | 1 | 1 | H | H | C(=O)CH₃ |
| 5A.22 | 0 | 1 | H | H | C(=O)CH₃ |
| 5A.23 | 1 | 1 | H | H | C(=O)C₆H₅ |
| 5A.24 | 1 | 1 | H | H | CH₂Oac |
| 5A.25 | 0 | 1 | H | H | CH₂Oac |
| 5A.26 | 1 | 1 | H | H | 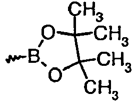 |
| 5A.27 | 0 | 1 | H | H | 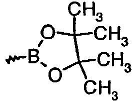 |
| 5A.28 | 1 | 1 | H | H | Si(OEt)₃ |
| 5A.29 | 0 | 1 | H | H | Si(OEt)₃ |
| 5A.30 | 1 | 1 | H | H | C(=O)C₆H₅ |
| 5A.31 | 0 | 1 | H | H | C(=O)C₆H₅ |
| 5A.32 | 1 | 1 | H | H | CH₂Cl |
| 5A.33 | 0 | 1 | H | H | CH₂Cl |
| 5A.34 | 1 | 1 | H | H | P(O)(OEt)₂ |
| 5A.35 | 0 | 1 | H | H | P(O)(OEt)₂ |
| 5A.36 | 1 | 1 | H | H | CH₂P(O)(OEt)₂ |
| 5A.37 | 0 | 1 | H | H | CH₂P(O)(OEt)₂ |
| 5A.38 | 1 | 1 | H | H | S(O)₂C₆H₅ |
| 5A.39 | 0 | 1 | H | H | S(O)₂C₆H₅ |
| 5A.40 | 1 | 1 | H | H | 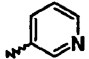 |
| 5A.41 | 1 | 1 | H | H | 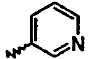 |
| 5A.42 | 0 | 1 | H | H | 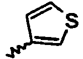 |
| 5A.43 | 0 | 1 | H | H | 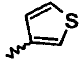 |
| 5A.44 | 1 | 1 | H | Cl | Cl |
| 5A.45 | 0 | 1 | H | Cl | Cl |
| 5A.46 | 1 | 1 | H | Br | Br |
| 5A.47 | 0 | 1 | H | Br | Br |
| 5A.48 | 1 | 1 | H | F | F |
| 5A.49 | 0 | 1 | H | F | F |
| 5A.50 | 0 | 1 | H | H | 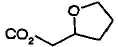 |
| 5A.51 | 0 | 1 | H | H | C(=O)CH₂CH₃ |
| 5A.52 | 0 | 1 | H | H | CH₂SO₂C₆H₅ |
| 5A.53 | 0 | 1 | H | H | CH₂O(C=O)OCH₃ |
| 5A.54 | 1 | 0 | H | H | CH₃ |

**Table 5B: Compounds of the formula**

| | | | | | | | |
|---|---|---|---|---|---|---|---|
| 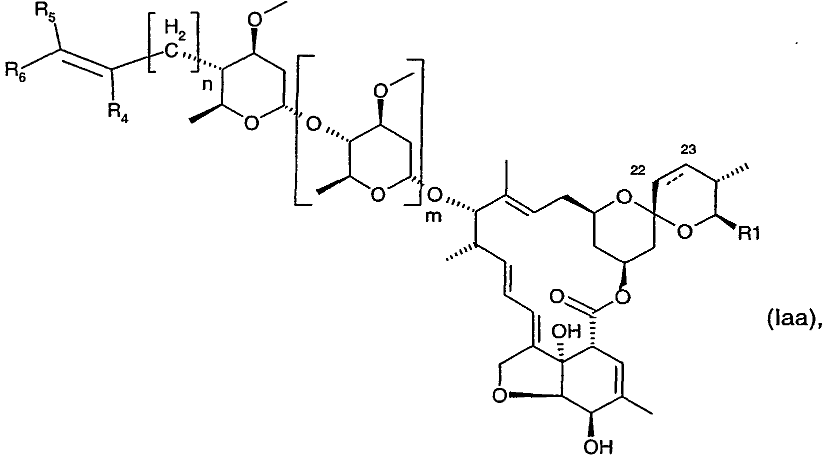 | | | | | | | |
| in which R₁ is sec-butyl (B1a) or isopropyl (B1b), and the bond between C₂₂ and C₂₃ is a double bond and the combination of the substituents R₄, R₅ and R₆ for each compound corresponds to a line 5.1 to 5.54 Table 5A. | | | | | | | |

| No. | m | n | R₄ | R₅ | R₆ | Retention time (min) | |
|---|---|---|---|---|---|---|---|
| | | | | | | B1a | B1b |
| 5B.1 | 0 | 1 | H | H | CO₂CH₃ | 10.61 | |
| 5B.2 | 0 | 1 | H | H | 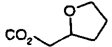 | 10.99 | |
| 5B.3 | 0 | 1 | H | H | SO₂C₆H₅ | 10.33 | |
| 5B.4 | 0 | 1 | H | H | (C=O)H | 9.76 | 9.02 |
| 5B.5 | 0 | 1 | H | H | CN | 9.88 | 9.13 |
| 5B.6 | 0 | 1 | H | H | C(=O)CH₂CH₃ | 10.40 | 9.66 |
| 5B.7 | 0 | 1 | H | H | (C=O)CH₃ | 9.66 | |
| 5B.8 | 0 | 1 | H | H | CH₂SO₂C₆H₅ | 10.36 | 9.61 |
| 5B.9 | 0 | 1 | H | H | CH₂O(C=O)OCH₃ | 10.52 | 9.77 |
| 5B.10 | 0 | 1 | H | H | H | 10.45 | 9.76 |
| 5B.11 | 1 | 1 | H | H | H | 11.99 | |
| 5B.12 | 1 | 0 | H | H | CH₃ | 13.50 | |
| 5B.13 | 1 | 0 | H | H | C₆H₅ | 12.19 | |

**Table 6A: Compounds of the formula**

| |
|---|
| 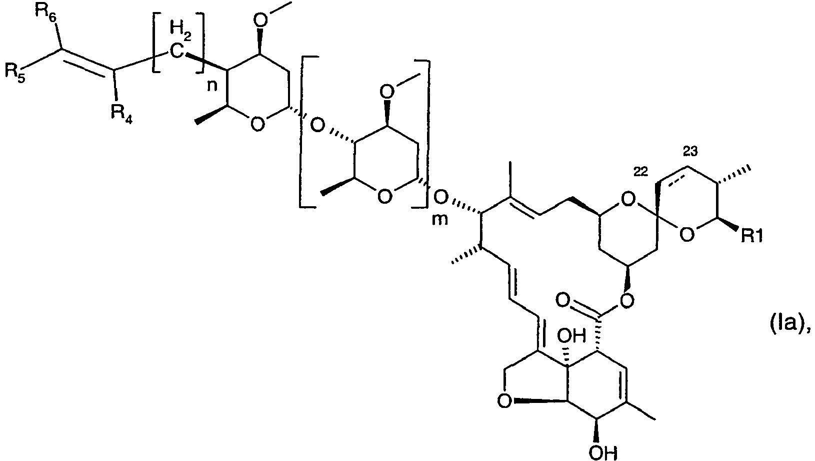 |
| in which R₁ is sec-butyl (B1a) or isopropyl (B1b), the bond between C₂₂ and C₂₃ is a double bond, and wherein the combination of m, n, R₄, R₅ and R₆ for each compound corresponds to a line 5.1 to 5.54 of table 5. |

**Table 6B: Compounds of the formula**

| |
|---|
| 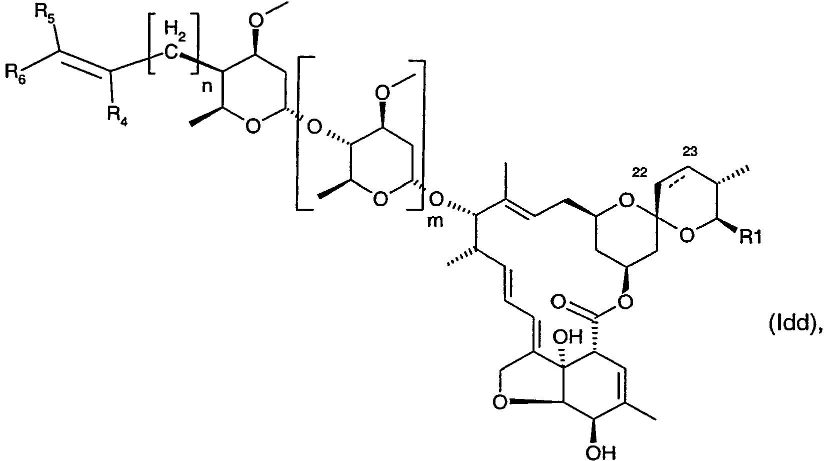 |
| in which R₁ is sec-butyl (B1a) or isopropyl (B1b), the bond between C₂₂ and C₂₃ is a double bond, and wherein the combination of m, n, R₄, R₅ and R₆ for each compound corresponds toaline 5.1 to 5.54 of table 5. |

**Table 7: Compounds of the formula**

| | | | |
|---|---|---|---|
| 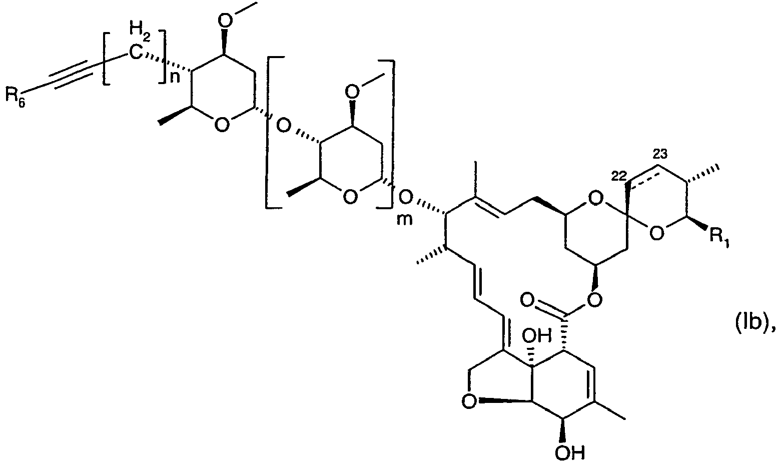 | | | |
| in which R₁ is sec-butyl (B1a) or isopropyl (B1b), and the bond between C₂₂ and C₂₃ is a double bond | | | |

| No. | m | n | R₆ |
|---|---|---|---|
| 7.1 | 1 | 0 | H |
| 7.2 | 0 | 0 | H |
| 7.3 | 1 | 1 | H |
| 7.4 | 0 | 1 | H |
| 7.5 | 1 | 2 | H |
| 7.6 | 0 | 2 | H |
| 7.7 | 1 | 0 | C₆H₅ |
| 7.8 | 0 | 0 | C₆H₅ |
| 7.9 | 1 | 1 | C₆H₅ |
| 7.10 | 0 | 1 | C₆H₅ |
| 7.11 | 1 | 0 | C(=O)CH₃ |
| 7.12 | 0 | 0 | C(=O)CH₃ |
| 7.13 | 1 | 1 | C(=O)CH₃ |
| 7.14 | 0 | 1 | C(=O)CH₃ |
| 7.15 | 1 | 0 | CH₂OH |
| 7.16 | 0 | 0 | CH₂OH |
| 7.17 | 1 | 1 | CH₂OH |
| 7.18 | 0 | 1 | CH₂OH |
| 7.19 | 1 | 0 | C(=O)NHCH₃ |
| 7.20 | 0 | 0 | C(=O)NHCH₃ |
| 7.21 | 1 | 1 | C(=O)NHCH₃ |
| 7.22 | 0 | 1 | C(=O)NHCH₃ |
| 7.23 | 1 | 0 | 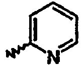 |
| 7.24 | 0 | 0 | 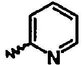 |
| 7.25 | 1 | 1 | 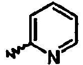 |
| 7.26 | 0 | 1 | 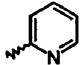 |
| 7.27 | 1 | 0 | 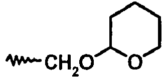 |
| 7.28 | 0 | 0 | 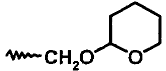 |
| 7.29 | 1 | 1 | 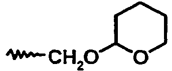 |
| 7.30 | 0 | 1 | 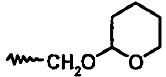 |
| 7.31 | 1 | 0 | 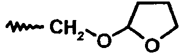 |
| 7.32 | 0 | 0 | 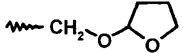 |
| 7.33 | 1 | 1 | 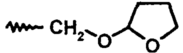 |
| 7.34 | 0 | 1 | 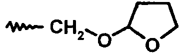 |

**Table 8: Compounds of the formula**

| |
|---|
| 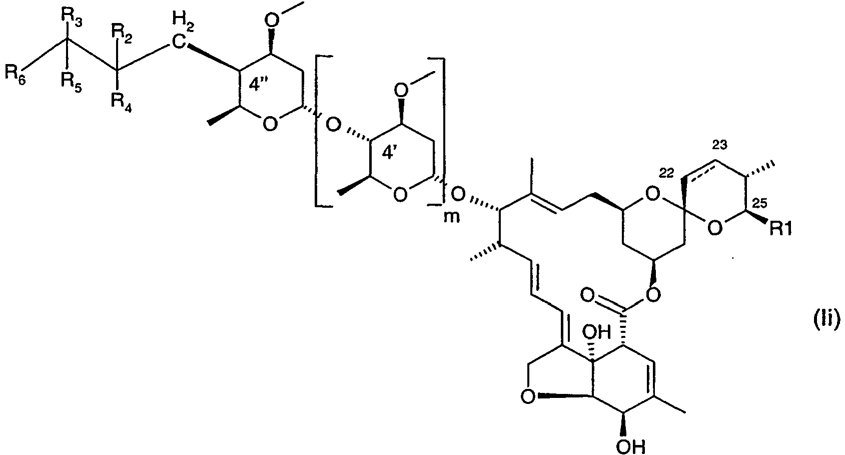 |
| in which R₁ is sec-butyl (B1a) or isopropyl (B1b), and the bond between C₂₂ and C₂₃ is a double bond and wherein the combination of m, n, R₂, R₃, R₄, R₅ and R₆ for each compound corresponds to a line 1.1 to 1.53 of table 1. |

**Table 9: Compounds of the formula**

| | | | | | | |
|---|---|---|---|---|---|---|
| 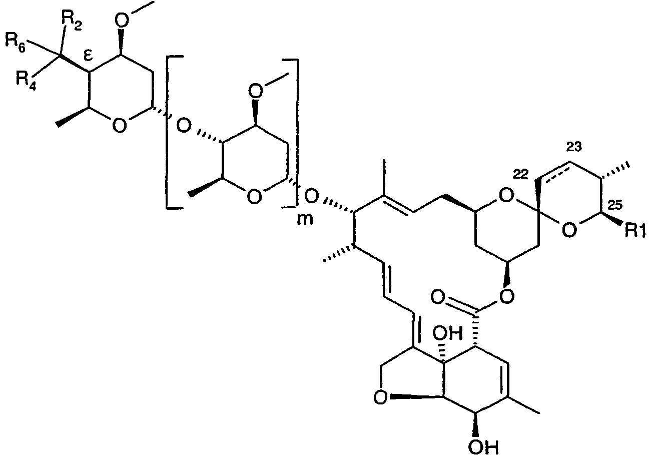 | | | | | | |
| in which R₁ is sec-butyl (B1a) or isopropyl (B1b), and the bond between C₂₂ and C₂₃ is a double bond and wherein the combination of m, R₂, R₄ and R₆ for each compound corresponds to a line 3.1 to 3.56 of table 3. | | | | | | |

| No. | m | R₂ | R₄ | R₆ | Retention time (min) | |
|---|---|---|---|---|---|---|
| | | | | | B1a | B1b |
| 9.53 | 1 | OH | H | H | 8.85 | |
| 9.54 | 1 | OH | CH₃ | H | 10.67 | 10.19 |
| | | | | | 10.92 | 10.35 |
| 9.55 | 1 | =NNHC(=O)OCH₃ | | H | 9.34 | |
| 9.56 | 1 | =O | | H | 10.68 | 9.91 |

**Table 10: Compounds of the formula**

| | | | | | | | |
|---|---|---|---|---|---|---|---|
| 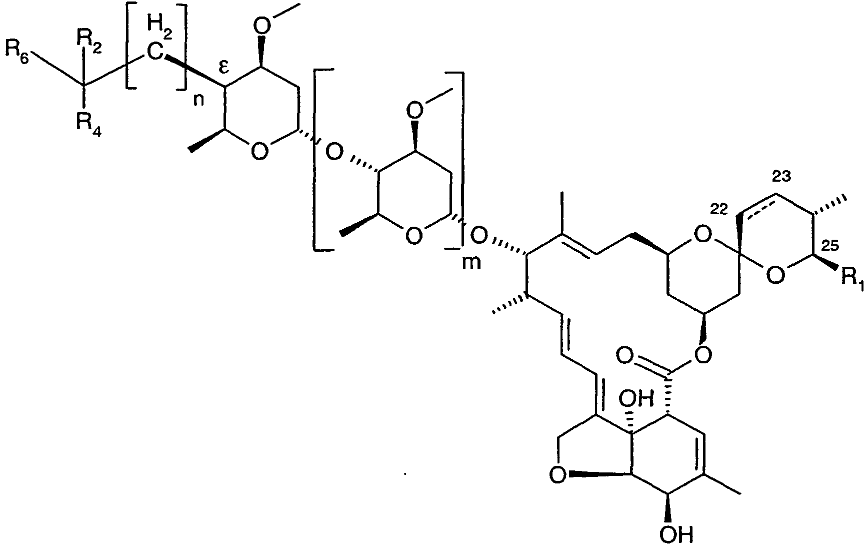 | | | | | | | |
| in which R₁ is sec-butyl (B1a) or isopropyl (B1b), the bond between C₂₂ and C₂₃ is a double bond and the and wherein the combination of m, n, R₂, R₄ and R₆ for each compound corresponds to a line 2.1 to 2.168 of table 2. | | | | | | | |

| No. | m | n | R₂ | R₄ | R₆ | Retention time (min) | |
|---|---|---|---|---|---|---|---|
| | | | | | | B1a | B1b |
| 10.5 | 1 | 1 | CH₃ | H | OH | 9.68 | |
| | | | | | | 10.30 | |
| 10.17 | 1 | 1 | CH₃ | CH₃ | OH | 11.69 | |
| 10.53 | 1 | 1 | H | H | OC(=O)CH₃ | 10.95 | 10.26 |
| 10.57 | 1 | 1 | H | H | OCH₂OCH₃ | 11.40 | |
| 10.61 | 1 | 1 | H | H | OS(=O)₂NH₂ | 8.81 | 8.19 |
| 10.83 | 1 | 1 | H | H | N(CH₃)₂ | 4.69 | |
| 10.87 | 1 | 1 | H | H | NHCH₃ | | |
| 10.91 | 1 | 1 | H | H | NHC(=O)H | 11.31 | |
| 10.99 | 1 | 1 | H | H | NHC(=O)CH₂OCH₃ | 11.30 | |
| 10.135 | 1 | 1 | H | H | OCH₂OCH₂C₆H₅ | 12.67 | |
| 10.139 | 1 | 1 | H | H | OCH₂O(CH₂)₂OCH₃ | 11.10 | |
| 10.153 | 1 | 1 | CH₂CH₃ | Et | OH | 13.01 | |
| 10.154 | 1 | 1 | CH₂CH=CH₂ | H | OH | 10.73 | |
| | | | | | | 11.24 | |
| 10.155 | 1 | 0 | H | H | CH(=NOH) | 11.29 | 10.62 |
| 10.156 | 1 | 0 | H | H | CH(=NOCH₃) | 12.87 | 12.45 |
| 10.157 | 1 | 1 | H | H | OC(=O)NHCH₃ | 11.82 | |
| 10.158 | 1 | 1 | H | H | 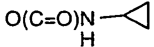 | 12.30 | |
| 10.159 | 1 | 1 | H | H | NHC(=O)CH(CH₃)₂ | 12.80 | |
| 10.160 | 1 | 1 | H | | =NNHC(=O)OCH₃ | 11.42 | |
| 10.161 | 1 | 1 | H | | =NNHS(=O)₂CH₃ | 11.56 | |
| 10.162 | 1 | 0 | H | H | NHC(=O)OC(CH₃)₃ | 13.54 | |
| 10.163 | 1 | 1 | H | H | OCH₂O(CH₂)₃CH₃ | 13.26 | |
| 10.164 | 1 | 1 | H | H | OC(=O)OCH₃ | 10.93 | 10.27 |
| 10.165 | 1 | 1 | H | H | O(=O)CH₂OCH₃ | 10.49 | 9.81 |
| 10.166 | 1 | 1 | H | H | 0(=O)OCH2C=CH | 10.89 | 10.27 |
| 10.167 | 1 | 0 | H | H | C(=O)H | 10.60 | |
| 10.168 | 1 | 0 | H | H | C(=O)OCH₂CH₃ | 12.41 | |

**Table 11: Compounds of the formula**

| |
|---|
| 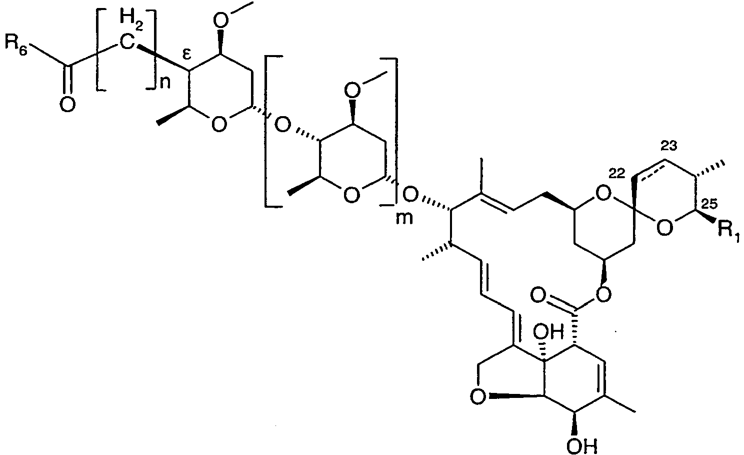 |
| in which R₁ is sec-butyl (B1a) or isopropyl (B1b), and the bond between C₂₂ and C₂₃ is a double bond and m, n and R₆ for each compound corresponds to a line 4.1 to 4.86 of table 4. |

**Table 12: Compounds of the formula**

| |
|---|
| 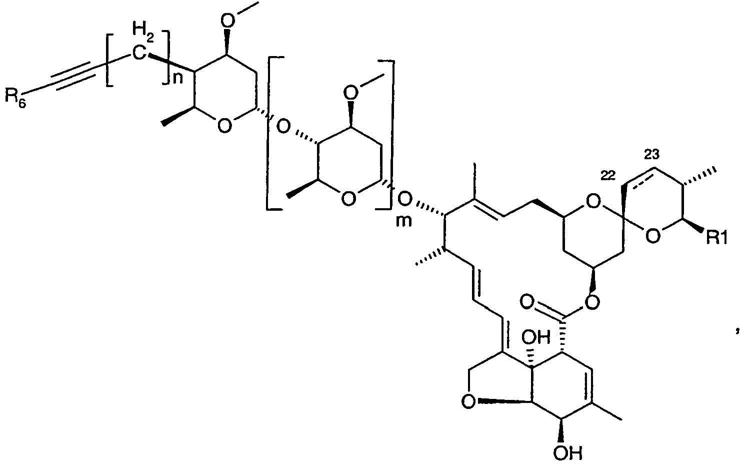 |
| in which R₁ is sec-butyl (B1a) or isopropyl (B1b), the bond between C₂₂ and C₂₃ is a double bond, m and n and R₆ for each compound corresponds to a line 7.1 to 7.34 of table 7. |

Table 13: Compounds of the formula as in table 1 in which R₁ is cyclohexyl, the bond between C₂₂ and C₂₃ is a double bond, and the combination of m, R₂, R₃, R₄, R₅ and R₆ for each compound corresponds to a line 1.1 to 1.53 of table 1.
Table 14: Compounds of the formul a as in table 1 in which R₁ is 1-methyl-butyl, the bond between C₂₂ and C₂₃ is a double bond, and the combination of m, R₂, R₃, R₄, R₅ and R₆ for each compound corresponds to a line 1.1 to 1.53 of table 1.
Table 15: Compounds of the formula as in table 1 in which R₁ is sec-butyl (B1a) or isopropyl (B1b), the bond between C₂₂ and C₂₃ is a single bond, and the combination of m, R₂, R₃, R₄, R₅ and R₆ for each compound corresponds to a line 1.1 to 1.53 of table 1.
Table 16: Compounds of the formula as in table 1 in which R₁ is cyclohexyl, the bond between C₂₂ and C₂₃ is a single bond, and the combination of m, R₂, R₃, R₄, R₅ and R₆ for each compound corresponds to a line 1.1 to 1.53 of table 1.
Table 17: Compounds of the formula as in table 1 in which R₁ is 1-methyl-butyl, the bond between C₂₂ and C₂₃ is a single bond, and the combination of m, R₂, R₃, R₄, R₅ and R₆ for each compound corresponds to a line 1.1 to 1.53 of table 1.
Table 18: Compounds of the formula as in table 2 in which R₁ is cyclohexyl, the bond between C₂₂ and C₂₃ is a double bond, and the combination of m, n, R₂, R₄, and R₆ for each compound corresponds to a line 2.1 to 2.168 of table 2.
Table 19: Compounds of the formula as in table 2 in which R₁ is 1-methyl-butyl, the bond between C₂₂ and C₂₃ is a double bond, and the combination of m, n, R₂, R₄, and R₆ for each compound corresponds to a line 2.1 to 2.168 of table 2.
Table 20: Compounds of the formula as in table 2 in which R₁ is sec-butyl (B1a) or isopropyl (B1b), the bond between C₂₂ and C₂₃ is a single bond, and the combination of m, n, R₂, R₄, and R₆ for each compound corresponds to a line 2.1 to 2.168 of table 2.
Table 21: Compounds of the formula as in table 2 in which R₁ is cyclohexyl, the bond between C₂₂ and C₂₃ is a single bond, and the combination of m, n, R₂, R₄, and R₆ for each compound corresponds to a line 2.1 to 2.168 of table 2.
Table 22: Compounds of the formula as in table 2 in which R₁ is 1-methyl-butyl, the bond between C₂₂ and C₂₃ is a single bond, and the combination of m, n, R₂, R₄, and R₆ for each compound corresponds to a line 2.1 to 2.168 of table 2.
Table 23: Compounds of the formula as in table 3 in which R₁ is cyclohexyl, the bond between C₂₂ and C₂₃ is a double bond, and the combination of m, R₂, R₄, and R₆ for each compound corresponds to a line 3.1 to 3.56 of table 3.
Table 24: Compounds of the formula as in table 3 in which R₁ is 1-methyf-butyl, the bond between C₂₂ and C₂₃ is a double bond, and the combination of m, R₂, R₄, and R₆ for each compound corresponds to a line 3.1 to 3.56 of table 3.
Table 25: Compounds of the formula as in table 3 in which R₁ is sec-butyl (B1a) or isopropyl (B1b), the bond between C₂₂ and C₂₃ is a single bond, and the combination of m, R₂, R₄, and R₆ for each compound corresponds to a line 3.1 to 3.56 of table 3.
Table 26: Compounds of the formula as in table 3 in which R₁ is cyclohexyl, the bond between C₂₂ and C₂₃ is a single bond, and the combination of m, R₂, R₄, and R₆ for each compound corresponds to a line 3.1 to 3.56 of table 3.
Table 27: Compounds of the formula as in table 3 in which R₁ is 1-methyl-butyl, the bond between C₂₂ and C₂₃ is a single bond, and the combination of m, R₂, R₄, and R₆ for each compound corresponds to a line 3.1 to 3.56 of table 3.
Table 28: Compounds of the formula as in table 4 in which R₁ is cyclohexyl, the bond between C₂₂ and C₂₃ is a double bond, and the combination of m, n and R₆ for each compound corresponds to a line 4.1 to 4.86 of table 4.
Table 29: Compounds of the formula as in table 4 in which R₁ is 1-methyl-butyl, the bond between C₂₂ and C₂₃ is a double bond, and the combination of m, n and R₆ for each compound corresponds to a line 4.1 to 4.86 of table 4.
Table 30: Compounds of the formula as in table 4 in which R₁ is sec-butyl (B1a) or isopropyl (B1b), the bond between C₂₂ and C₂₃ is a single bond, and the combination of m, n and R₆ for each compound corresponds to a line 4.1 to 4.86 of table 4.
Table 31: Compounds of the formula as in table 4 in which R₁ is cyclohexyl, the bond between C₂₂ and C₂₃ is a single bond, and the combination of m, n and R₆ for each compound corresponds to a line 4.1 to 4.86 of table 4.
Table 32: Compounds of the formula as in table 4 in which R₁ is 1-methyl-butyl, the bond between C₂₂ and C₂₃ is a single bond, and the combination of m, n and R₆ for each compound corresponds to a line 4.1 to 4.86 of table 4.
Table 33: Compounds of the formula as in table 5 in which R₁ is cyclohexyl, the bond between C₂₂ and C₂₃ is a double bond, and the combination of m, n, R₄, R₅ and R₆ for each compound corresponds to a line 5.1 to 5.54 of table 5.
Table 34: Compounds of the formula as in table 5 in which R₁ is 1-methyl-butyl, the bond between C₂₂ and C₂₃ is a double bond, and the combination of m, n, R₄, R₅ and R₆ for each compound corresponds to a line 5.1 to 5.54 of table 5.
Table 35: Compounds of the formula as in table 5 in which R₁ is sec-butyl (B1a) or isopropyl (B1b), the bond between C₂₂ and C₂₃ is a single bond, and the combination of m, n, R₄, R₅ and R₆ for each compound corresponds to a line 5.1 to 5.54 of table 5.
Table 36: Compounds of the formula as in table 5 in which R₁ is cyclohexyl, the bond between C₂₂ and C₂₃ is a single bond, and the combination of m, n, R₄, R₅ and R₆ for each compound corresponds to a line 5.1 to 5.54 of table 5.
Table 37: Compounds of the formula as in table 5 in which R₁ is 1-methyl-butyl, the bond between C₂₂ and C₂₃ is a single bond, and the combination of m, n, R₄, R₅ and R₆ for each compound corresponds to a line 5.1 to 5.54 of table 5.
Table 38: Compounds of the formula as in table 6 in which R₁ is cyclohexyl, the bond between C₂₂ and C₂₃ is a double bond, and the combination of m, n, R₄, R₅ and R₆ for each compound corresponds to a line 5.1 to 5.54 of table 5.
Table 39: Compounds of the formula as in table 6 in which R₁ is 1-methyl-butyl, the bond between C₂₂ and C₂₃ is a double bond, and the combination of m, n, R₄, R₅ and R₆ for each compound corresponds to a line 5.1 to 5.54 of table 5.
Table 40: Compounds of the formula as in table 6 in which R₁ is sec-butyl (B1a) or isopropyl (B1b), the bond between C₂₂ and C₂₃ is a single bond, and the combination of m, n, R₄, R₅ and R₆ for each compound corresponds to a line 5.1 to 5.54 of table 5.
Table 41: Compounds of the formula as in table 6 in which R₁ is cyclohexyl, the bond between C₂₂ and C₂₃ is a single bond, and the combination of m, n, R₄, R₅ and R₆ for each compound corresponds to a line 5.1 to 5.54 of table 5.
Table 42: Compounds of the formula as in table 6 in which R₁ is 1-methyl-butyl, the bond between C₂₂ and C₂₃ is a single bond, and the combination of m, n, R₄, R₅ and R₆ for each compound corresponds to a line 5.1 to 5.54 of table 5.
Table 43: Compounds of the formula as in table 7 in which R₁ is cyclohexyl, the bond between C₂₂ and C₂₃ is a double bond, and the combination of m, n and R₆ for each compound corresponds to a line 7.1 to 7.34 of table 7.
Table 44: Compounds of the formula as in table 7 in which R₁ is 1-methyl-butyl, the bond between C₂₂ and C₂₃ is a double bond, and the combination of m, n and R₆ for each compound corresponds to a line 7.1 to 7.34 of table 7.
Table 45: Compounds of the formula as in table 7 in which R₁ is sec-butyl (B1a) or isopropyl (B1b), the bond between C₂₂ and C₂₃ is a single bond, and the combination of m, n and R₆ for each compound corresponds to a line 7.1 to 7.34 of table 7.
Table 46: Compounds of the formula as in table 7 in which R₁ is cyclohexyl, the bond between C₂₂ and C₂₃ is a single bond, and the combination of m, n and R₆ for each compound corresponds to a line 7.1 to 7.34 of table 7.
Table 47: Compounds of the formula as in table 7 in which R₁ is 1-methyl-butyl, the bond between C₂₂ and C₂₃ is a single bond, and the combination of m, n and R₆ for each compound corresponds to a line 7.1 to 7.34 of table 7.
Table 48: Compounds of the formula as in table 8 in which R₁ is cyclohexyl, the bond between C₂₂ and C₂₃ is a double bond, and the combination of m, R₂, R₃, R₄, R₅ and R₆ for each compound corresponds to a line 1.1 to 1.53 of table 1.
Table 49: Compounds of the formula as in table 8 in which R₁ is 1-methyl-butyl, the bond between C₂₂ and C₂₃ is a double bond, and the combination of m, R₂, R₃, R₄, R₅ and R₆ for each compound corresponds to a line 1.1 to 1.53 of table 1.
Table 50: Compounds of the formula as in table 8 in which R₁ is sec-butyl (B1a) or isopropyl (B1b), the bond between C₂₂ and C₂₃ is a single bond, and the combination of m, R₂, R₃, R₄, R₅ and R₆ for each compound corresponds to a line 1.1 to 1.53 of table 1.
Table 51: Compounds of the formula as in table 8 in which R₁ is cyclohexyl, the bond between C₂₂ and C₂₃ is a single bond, and the combination of m, R₂, R₃, R₄, R₅ and R₆ for each compound corresponds to a line 1.1 to 1.53 of table 1.
Table 52: Compounds of the formula as in table 8 in which R₁ is 1-methyl-butyf, the bond between C₂₂ and C₂₃ is a single bond, and the combination of m, R₂, R₃, R₄, R₅ and R₆ for each compound corresponds to a line 1.1 to 1.53 of table 1.
Table 53: Compounds of the formula as in table 9 in which R₁ is cyclohexyl, the bond between C₂₂ and C₂₃ is a double bond, and the combination of m, R₂, R₄, and R₆ for each compound corresponds to a line 3.1 to 3.56 of table 3.
Table 54: Compounds of the formula as in table 9 in which R₁ is 1-methyl-butyl, the bond between C₂₂ and C₂₃ is a double bond, and the combination of m, R₂, R₄, and R₆ for each compound corresponds to a line 3.1 to 3.56 of table 3.
Table 55: Compounds of the formula as in table 9 in which R₁ is sec-butyl (B1a) or isopropyl (B1b), the bond between C₂₂ and C₂₃ is a single bond, and the combination of m, R₂, R₄, and R₆ for each compound corresponds to a line 3.1 to 3.56 of table 3.
Table 56: Compounds of the formula as in table 9 in which R₁ is cyclohexyl, the bond between C₂₂ and C₂₃ is a single bond, and the combination of m, R₂, R₄, and R₆ for each compound corresponds to a line 3.1 to 3.56 of table 3.
Table 57: Compounds of the formula as in table 9 in which R₁ is 1-methyl-butyl, the bond between C₂₂ and C₂₃ is a single bond, and the combination of m, R₂, R₄, and R₆ for each compound corresponds to a line 3.1 to 3.56 of table 3.
Table 58: Compounds of the formula as in table 10 in which R₁ is cyclohexyl, the bond between C₂₂ and C₂₃ is a double bond, and the combination of m, n, R₂, R₄, and R₆ for each compound corresponds to a line 2.1 to 2.168 of table 2.
Table 59: Compounds of the formula as in table 10 in which R₁ is 1-methyl-butyl, the bond between C₂₂ and C₂₃ is a double bond, and the combination of m, n, R₂, R₄, and R₆ for each compound corresponds to a line 2.1 to 2.168 of table 2.
Table 60: Compounds of the formula as in table 10 in which R₁ is sec-butyl (B1a) or isopropyl (B1b), the bond between C₂₂ and C₂₃ is a single bond, and the combination of m, n, R₂, R₄, and R₆ for each compound corresponds to a line 2.1 to 2.168 of table 2.
Table 61: Compounds of the formula as in table 10 in which R₁ is cyclohexyl, the bond between C₂₂ and C₂₃ is a single bond, and the combination of m, n, R₂, R₄, and R₆ for each compound corresponds to a line 2.1 to 2.168 of table 2.
Table 62: Compounds of the formula as in table 10 in which R₁ is 1-methyl-butyl, the bond between C₂₂ and C₂₃ is a single bond, and the combination of m, n, R₂, R₄, and R₆ for each compound corresponds to a line 2.1 to 2.168 of table 2.
Table 63: Compounds of the formula as in table 11 in which R₁ is cyclohexyl, the bond between C₂₂ and C₂₃ is a double bond, and the combination of m, n and R₆ for each compound corresponds to a line 4.1 to 4.86 of table 4.
Table 64: Compounds of the formula as in table 11 in which R₁ is 1-methyl-butyl, the bond between C₂₂ and C₂₃ is a double bond, and the combination of m, n and R₆ for each compound corresponds to a line 4.1 to 4.86 of table 4.
Table 65: Compounds of the formula as in table 11 in which R₁ is sec-butyl (B1a) or isopropyl (B1b), the bond between C₂₂ and C₂₃ is a single bond, and the combination of m, n and R₆ for each compound corresponds to a line 4.1 to 4.86 of table 4.
Table 66: Compounds of the formula as in table 11 in which R₁ is cyclohexyl, the bond between C₂₂ and C₂₃ is a single bond, and the combination of m, n and R₆ for each compound corresponds to a line 4.1 to 4.86 of table 4.
Table 67: Compounds of the formula as in table 11 in which R₁ is 1-methyl-butyl, the bond between C₂₂ and C₂₃ is a single bond, and the combination of m, n and R₆ for each compound corresponds to a line 4.1 to 4.86 of table 4.
Table 68: Compounds of the formula as in table 12 in which R₁ is cyclohexyl, the bond between C₂₂ and C₂₃ is a double bond, and the combination of m, n and R₆ for each compound corresponds to a line 7.1 to 7.34 of table 7.
Table 69: Compounds of the formula as in table 12 in which R₁ is 1-methyl-butyl, the bond between C₂₂ and C₂₃ is a double bond, and the combination of m, n and R₆ for each compound corresponds to a line 7.1 to 7.34 of table 7.
Table 70: Compounds of the formula as in table 12 in which R₁ is sec-butyl (B1a) or isopropyl (B1b), the bond between C₂₂ and C₂₃ is a single bond, and the combination of m, n and R₆ for each compound corresponds to a line 7.1 to 7.34 of table 7.
Table 71: Compounds of the formula as in table 12 in which R₁ is cyclohexyl, the bond between C₂₂ and C₂₃ is a single bond, and the combination of m, n and R₆ for each compound corresponds to a line 7.1 to 7.34 of table 7.
Table 72: Compounds of the formula as in table 12 in which R₁ is 1-methyl-butyl, the bond between C₂₂ and C₂₃ is a single bond, and the combination of m, n and R₆ for each compound corresponds to a line 7.1 to 7.34 of table 7.

### Formulation examples for use in crop protection (% = per cent by weight)

| Example F1: Emulsion concentrates | a) | b) | c) |
|---|---|---|---|
| Active compound25% 40% 50% | | | |
| Calcium dodecylbenzenesulphonate | 5% | 8% | 6% |
| Castor oil polyethylene glycol ether (36 mol of EO) | 5% | - | - |
| Tributylphenol polyethylene glycol ether (30 mol of EO) | - | 12% | 4% |
| Cyclohexanone | - | 15% | 20% |
| Xylene mixture | 65% | 25% | 20% |

Mixing of finely ground active compound and additives gives an emulsion concentrate which, by dilution with water, affords emulsions of the desired concentration.

| Example F2: Solutions | a) | b) | c) | d) |
|---|---|---|---|---|
| active ingredient | 80% | 10% | 5% | 95% |
| ethylene glycol monomethyl ether | - | 20% | - | - |
| polyethylene glycol (MW 400) | - | 70% | - | - |
| N-methylpyrrolid-2-one | 20% | - | - | - |
| epoxidised coconut oil | - | - | 1 % | 5% |
| petroleum ether (boiling range: 160-190°) | - | - | 94% | - |

Mixing of finely ground active compound and additives gives a solution suitable for use in the form of microdrops.

| Example F3: Granules | a) | b) | c) | d) |
|---|---|---|---|---|
| Active compound | 5% | 10% | 8% | 21 % |
| Kaolin | 94% | - | 79% | 54% |
| Finely divided silicic acid | 1% | - | 13% | 7% |
| Attapulgite | - | 90% | - | 18% |

The active compound is dissolved in dichloromethane, the solution is sprayed onto the mixture of carriers and the solvent is evaporated under reduced pressure.

| Example F4: Wettable powder | a) | b) | c) |
|---|---|---|---|
| Active compound | 25% | 50% | 75% |
| Sodium lignosulphonate | 5% | 5% | - |
| Sodium lauryl sulphate | 3% | - | 5% |
| Sodium diisobutylnaphthalene sulphonate | - | 6% | 10% |
| Octylphenol polyethylene glycol ether (7-8 mol of EO) | - | 2% | - |
| Finely divided silicic acid | 5% | 10% | 10% |
| Kaolin | 62% | 27% | - |

Active compound and additives are mixed and the mixture is ground in a suitable mill. This gives wettable powders which can be diluted with water to give suspensions of the desired concentration.

### Example F5: Emulsion concentrate

| | |
|---|---|
| Active compound | 10% |
| Octylphenol polyethylene glycol ether (4-5 mol of EO) | 3% |
| Calcium dodecylbenzenesulphonate | 3% |
| Castor oil polyethylene glycol ether (36 mol of EO) | 4% |
| Cyclohexanone | 30% |
| Xylene mixture | 50% |

Mixing of finely ground active compound and additives gives an emulsion concentrate which, by dilution with water, affords emulsions of the desired concentration.

### Example F6: Extruder granules

| | |
|---|---|
| Active compound | 10% |
| Sodium lignosulphonate | 2% |
| Carboxymethylcellulose | 1% |
| Kaolin | 87% |

Active compound and additives are mixed, the mixture is ground, moistened with water, extruded and granulated, and the granules are dried in a stream of air.

### Example 7: Coated granules

| | |
|---|---|
| Active compound | 3% |
| Polyethylene glycol (MW 200) | 3% |
| Kaolin | 94% |

In a mixer, the finely ground active compound is applied uniformly to the kaolin which has been moistened with polyethylene glycol. This gives dust-free coated granules.

### Example F8: Suspension concentrate

| | |
|---|---|
| Active compounds | 40% |
| Ethylene glycol | 10% |
| Nonylphenol polyethylene glycol ether (15 mol of EO) | 6% |
| Sodium lignosulphonate | 10% |
| Carboxymethylcellulose | 1 % |
| Aqueous formaldehyde solution (37%) | 0.2% |
| Aqueous silicone oil emulsion (75%) | 0.8% |
| Water | 32% |

Mixing of finely ground active compound and additives gives a suspension concentrate which, by dilution with water, affords suspensions of the desired concentration.

### Biological examples:

### Example B1: Activity against Spodoptera littoralis

Young soya bean plants are sprayed with an aqueous emulsion spray liquor which comprises 12.5 ppm of active compound, and, after the spray coating has dried on, populated with 10 caterpillars of the first stage of Spodoptera littoralis and introduced into a plastic container. 3 days later, the reduction in the population in per cent and the reduction in the feeding damage in per cent (% activity) are determined by comparing the number of dead caterpillars and the feeding damage between the treated and the untreated plants.

In this test, the compounds of the tables show good activity. Thus, in particular the compounds 2.1, 2.2, 2.3, 2.4, 5.1 and 5.2 effect a reduction in the pest population by more than 80%.

### Example B2: Activity agiainst Spodoptera littoralis, systemic:

Maize seedlings are placed into the test solution which comprises 12.5 ppm of active compound. After 6 days, the leaves are cut off, placed onto moist filter paper in a Petri dish and populated with 12 to 15 Spodoptera littoralis larvae of the L₁ stage. 4 days later, the reduction of the population in per cent (% activity) is determined by comparing the number of dead caterpillars between the treated and the untreated plants.

In this test, the compounds of the tables show good activity.

### Example B3: Activity against Heliothis virescens

35 0- to 24-hour-old eggs of Heliothis virescens are placed onto filter paper in a Petri dish on a layer of synthetic feed. 0.8 ml of the test solution which comprises 12.5 ppm of active compound, is then pipetted onto the filter papers. Evaluation is carried out after 6 days. The reduction in the population in per cent (% activity) is determined by comparing the number of dead eggs and larvae on the treated and the untreated filter papers.

In this test, the compounds of the tables show good activity. Thus, in particular the compounds 2.1, 2.2, 2.3, 2.4, 5.1 and 5.2 effect a reduction in the pest population by more than 80%.

### Example B4: Activity against Plutella xylostella caterpillars

Young cabbage plants are sprayed with an aqueous emulsion spray liquor which comprises 12.5 ppm of the active compound. After the spray coating has dried on, the cabbage plants are populated with 10 caterpillars of the first stage of Plutella xylostella and introduced into a plastic container. Evaluation is carried out after 3 days. The reduction in the population in per cent and the reduction in the feeding damage in per cent (% activity) are determined by comparing the number of dead caterpillars and the feeding damage on the treated and the untreated plants.

In this test, the compounds of the tables show good activity against Plutella xylostella. Thus, in particular the compounds 2.1, 2.2, 2.3, 2.4, 5.1 and 5.2 effect a reduction in the pest population by more than 80%.

### Example B5: Activity against Frankliniella occidentalis

In Petri dishes, discs of the leaves of beans are placed onto agar and sprayed with test solution which comprises 12.5 ppm of active compound, in a spraying chamber. The leaves are then populated with a mixed population of Frankliniella occidentalis. Evaluation is carried out after 10 days. The reduction in per cent (% activity) is determined by comparing the population on the treated leaves with that of the untreated leaves.

In particular the compounds 2.1, 2.2, 2.3, 2.4, 5.1 and 5.2 effect a reduction in the pest population by more than 80%.

### Example B6: Activity against Diabrotica balteata

Maize seedlings are sprayed with an aqueous emulsion spray liquor which comprises 12.5 ppm of active compound and, after the spray coating has dried on, populated with 10 larvae of the second stage of Diabrotica balteata and then introduced into a plastic container. After 6 days, the reduction in the population in per cent (% activity) is determined by comparing the dead larvae between the treated and the untreated plants.

In this test, the compounds of the tables show good activity. Thus, in particular the compounds 2.1, 2.2, 2.3, 2.4, 5.1 and 5.2 effect a reduction in the pest population by more than 80%.

### Example B7: Activity against Tetranychus urticae

Young bean plants are populated with a mixed population of Tetranychus urticae and, after 1 day, sprayed with an aqueous emulsion spray liquor which comprises 12.5 ppm of active compound, incubated at 25°C for 6 days and then evaluated. The reduction in the population in per cent (% activity) is determined by comparing the number of dead eggs, larvae and adults on the treated and on the untreated plants.

In this test, the compounds of the tables show good activity. Thus, in particular the compounds 2.1, 2.2, 2.3, 2.4, 5.1 and 5.2 effect a reduction in the pest population by more than 80%.

## Claims

1. A compound of the formula 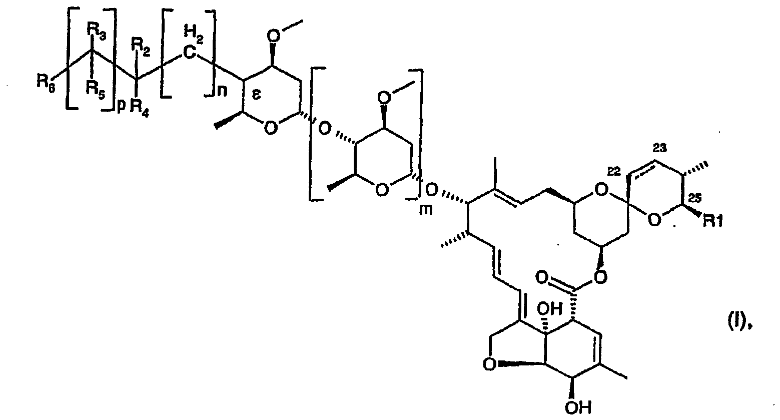 wherein the bond of atoms C₂₂ and C₂₉ is a single or double bond;
m is 0ⱼ
n is 0, 1 or 2;
p is 0 or 1;
R₁ is C₁-C₁₂-alkyl, C₃-C₈-cycloalkyl or C₂-C₁₂-alkanyl;
R₂ is H, C₁-C₁₂-alkyl, C₁-C₁₂-haloalkyl, C₁-C₁₂-hydroxyalkyl, OH, halogen, -N₃, SCN, NO₂, CN, C₃-C₈cycloalkyl unsubstituted or substituted by from one to three methyl groups, C₃-C₈halocycloalkyl, C₁-C₁₂alkoxy, C₁-C₆alkoxy-C₁-C₆alkyl, C₁-C₆alkoxy-C₁-C₆alkoxy, C₁-C₈alkoxy-C₁-C₆alkoxy-C₁-C₆alkyl, C₂-C₁₂alkenyl, C₂-C₁₂haloalkenyl, C₂-C₁₂haloalkenyloxy, C₂-C₁₂alkynyl, C₂-C₁₂haloalkynyl, C₃-C₁₂alkynyloxy, C₃-C₁₂haloalkynyloxy, -P(=O)(OC₁-C₆alkyl)₂, -Si(C₁-C₆alkyl)₃, -(CH₂)-Si(C₁-C₆alkyl)₃, -Si(OC₁-C₆alkyl)₃, -N(R₉)₂, -(CH₂)-N(R₉)₂, wherein the two substituents R₉ are independent of each other, -C(=X)-R₇, -(CH₂)-C(=X)-R7, -O-C(=X)-R₇, -(CH₂)-O-C(=X)-R₇, -S-C(=X)-R₇, -(CH₂)-S-C(=X)-R₇, -NR₉C(=X)R₇, -(CH₃)-NR₈C(=X)R₇, -NR₉NHC(=X)-R₇, -NR₉-OR₁₀, -(CH₂)-NR₉-OR₁₀, -SP₉, -S(=O)R₁₁, -S(=O)₂R₁₁, aryl, heterocyclyl, aryloxy or heterocyclyloxy; wherein the aryl, heterocyclyl, aryloxy and heterocyclyloxy radicals are unsubstituted or, depending upon the possibilities of substitution at the ring, mono- to penta-substituted by substituents selected from the group consisting of OH, halogen, CN, NO₂, SCN, -N₃, C₁-C₁₂alkyl, C₃-C₈cycloalkyl, C₁-G₁₂haloalkyl, C₁-C₁₂alkoxy, C₁-C₁₂haloalkoxy, C₁-C₁₂alkylthio, C₁-C₁₂haloalkylthio, C₁-C₆alkoxy-C₁-C₆alkyl, C₂-C₈alkenyl, C₂-C₈alkynyl, C₂-O₁₂haloalkenyl, C₁-C₁₂haloalkenyloxy, C₂-C₁₂haloalkynyl, C₃-C₁₂alkynyloxy, C₃-C₁₂haloalkynyloxy and phenoxy;
or, when p is 1, R₂ together with R₃ is a bond;
or R₂ together with R₄ is =O or =S;
or R₂ together with R₄ form with the carbon to which they are bound a three- to seven-membered ring, which may be monocyclic or bicyclic, and may be saturated or unsaturated, and that may contain one or two hetero atoms selected from the group consisting of N, O and 5, and which is either unsubstituted or independently of one another mono- to penta-substituted with substituents selected from OH, =O, SH, =S, halogen, CN, -N₃, SCN, NO₂, aryl, C₁-C₁₂alkyl, C₃-C₈cycloalkyl, C₁-C₁₂haloalkyl, C₁-C₁₂alkoxy, C₁-C₁₂haloalkoxy, C₁-C₁₂alkylthlo, C₁-C₁₂haloalkylthio, C₁-C₆alkoxy-C₁-C₆alkyl, C₂-C₆alkenyl, C₂-C₈alkynyl, C₂-C₁₂haloalkenyl, C₂-C₁₂haloalkenyloxy, C₂-C₁₂haloalkynyl, C₃-C₁₂alkynyloxy, C₃-C₁₂haloalkynyloxy, phenoxy, phenyl-C₁-C₈alkyl, -N(R₉)₂ wherein the two R₉ are independent of each other, C₁-C₆alkylsulfinyl, C₃-C₈cycloalkylsulfinyl, C₁-C₆haloalkylsulfinyl, C₃-C₈halocycloalkylsulfinyl, C₁-C₆alkylsulfonyl, C₃-C₈cycloalkylsulfonyl, C₁-C₆haloalkylsulfonyl and C₃-C₈halocycloalkylsulfonyl; or
R₂ together with R₄ is =NN(R₁₂)₂, wherein the two substituents R₉ are independent of each other;
or, when p is 0, R₂ together with R₄ and R₆ is ≡N;
or when p is 0, R₂ together with R₈ is =NOR₁₂ or =NN(A₁₂)₂, wherein the two substituents R₉ are independent of each other;
R₃ is H, C₁-C₁₂-alkyl, halogen, halo-C₁-C₂alkyl, CN, -N₃, SCN, NO₂, C₃-C₈cycloalkyl unsubstituted or substituted by from one to three methyl groups, C₃-C₈halocycloalkyl, C₁-C₁₂a,lkoxy, C₁-C₆alkoxy-C₁-C₆alkyl, C₁-C₆alkoxy-C₁-C₆alkoxy-C₁-C₆alkoxyl, C₃-C₈cycloalkoxy, C₁-C₁₂haloalkoxy, C₁-C₁₂alkylthio, C₃-C₈cycloalkylthio, C₁-C₁₂haloalkylthio, C₁-C₁₂alkylsulfinyl, C₃-C₈cycloalkylsulfinyl, C₁-C₁₂haloalkylsulfinyl, C₃-C₈halocycloalkylsulfinyl, C₁-C₁₂alkylsulfonyl, C₃-C₈cycloalkylsulfonyl, C₁-C₁₂haloalkylsulfonyl, C₃-C₈halocycloalkylsulfonyl, C₂-C₈-alkenyl, C₂-C₈alkynyl, C₂-C₁₂haloalkenyl, C₂-C₁₂haloalkenyloxy, C₂-C₁₂haloalkynyl, C₃-C₁₂haloalkynyloxy, -N(R₉)₂, wherein the two substituents R₉ are independent of each other, aryl, heterocyclyl, aryloxy or heterocyclyloxy; wherein the aryl, heterocyclyl, aryloxy and heterocyclyloxy radicals are unsubstituted or, depending upon the possibilities of substitution at the ring, mono- to penta-substituted by substituents selected from the group consisting of halogen, CN, NO₂, C₁-C₁₂alkyl, C₃-C₈cycloalkyl, C₁-G₁₂haloalkyl, (C₁-C₁₂alkoxy, C₁-C₁₂haloalkoxy, C₁-C₁₂alkylthio, C₁-C₁₂haloalkylthio, C₁-C₆alkoxy-C₁-C₆alkyl, C₂-C₈alkenyl, C₂-C₈-alkynyl, C₂-C₁₂haloalkenyl, C₂-C₁₂haloalkenyloxy, C₂-C₁₂haloalkynyl and C₃-C₁₂haloalkynyloxy;
or when p is 1, R₃ together with R₂ is a bond;
R₄ is H, C₁-C₁₂-alkyl, C₁-C₁₂-haloalkyl, C₁-C₁₂-hydroxyalkyl, OH, halogen, NO₂, CN, C₃-C₈cydoalkyl unsubstituted or substituted by from one to three methyl groups, C₃-C₈halocycloalkyl, C₁-C₁₂alkoxy, C₁-C₆alkoxy-C₁-C₆alkyl, C₁-C₆alkoxy-C₁-C₆alkoxy, C₁-C₆alkoxy-C₁-C₆lkoxy-C₁-C₆alkyl, C₂-C₁₂alkenyl, C₂-C₁₂haloalkanyl, C₂-C₁₂haloalkenyloxy, C₂-C₁₂alkynyl, C₂-C₁₂haloalkynyl, C₃-C₁₂haloalkynyloxy, -P(=O)(OC₁-C₆alkyl)₂, -Si(C₁-C₆alkyl)₃, -(CH₂)-Si(C₁-C₆alkyl)₃, -Si(OC₁-C₆alkyl)₃, -N(R₉)₂, -(CH₂)-N(R₉)₂, wherein the two substituents R₉ are independent of each other, -C(=X)-R₇, -(CH₂)-O(=X)-R₇, -O-C(=X)-R₇, -(CH₂-O-C(=X)-R₇, -S-C(=X)-R₇, -(CH₂)-S-C(=X)-R₇, NR₉C(=X)R₇, -(CH₂)-NR₈C(=X)R₇, -NR₉NHC(=X)-R₇, -NR₉-OR₁₀, -(CH₂)-NR₉-OR₁₀, -SR₉, -S(=O)R₁₁, -S(=O)₂R₁₁, aryl, heterocyclyl, aryloxy or heterocyclyloxy; wherein the aryl, heterocyclyl, aryloxy and heterocyclyloxy radicals are unsubstituted or, depending upon the possibilities of substitution at the ring, mono- to penta-substituted by substituents selected from the group consisting of OH, halogen, CN, NO₂, C₁-C₁₂alkyl, C₃-C₈cycloalkyl, C₁-C₁₂haloalkyl, C₁-C₁₂alkoxy, C₁-C₁₂haloalkoxy, C₁-C₁₂alkylthio, C₁-C₁₂haloalkylthio, C₁-C₆alkoxy-C₁-C₆alkyl, C₂-C₈alkenyl, C₂-C₈alkynyl, C₂C₁₂haloalkenyl, C₂-C₁₂haloalkenyloxy, C₂-C₁₂haloalkynyl, C₃-C₁₂haloalkynyloxy and phenoxy;
or R₄ together with R₂ forms =O or =S;
or when p is 1, R₄ together with R₅ is a bond;
or, when p is 0, together with R₂ and R₈ is ≡N;
R₅ and R₈ Independently of each other are H, C₁-C₁₂-alkyl, -N₃, CN, NO₂, OH, SH, halogen, halo-C₁-C₂alkyl, hydroxy-C₁-C₂alkyl, C₃-C₈cycloalkyl that is unsubstituted or substituted by from one to two methyl groups, C₃-C₈halocycloalkyl, C₁-C₁₂alkoxy, C₁-C₆alkoxy-C₁-C₆alkyl, C₁-C₆alkoxy-C₁-C₆alkoxy, C₁-C₆alkoxy-C₁-C₆alkoxy-C₁-C₆alkyl, C₃-C₈cycloalkoxy, C₁-C₁₂haloalkoxy, G₁-C₁₂haloalkylthio, C₂-C₈alkenyl, C2-C₈alkynyl, C₂-C₁₂haloalkenyl, C₂-C₁₂haloalkenyloxy, C₂-C₁₂haloalkynyl, C₃-C₁₂haloalkynyloxy, -P(=O)(OC₁-C₆alkyl)₂, -CH₂-P(=O)(OC₁-C₆alkyl)₂, -Si(OC₁-C₆alkyl)₃, -N(R₉)₂, -O-N(R₉)₂, wherein the two substituents R₉ are independent of each other, -C(=X)-R₇, -CH-NOH, -CH=NOC₁-C₆alkyl, -O-C(=X)-R₇, -S-C(=X)-R₇, -NR₉C(=X)R₇, -NR₉NHC(=X)-R₇, -NR₉-OR₁₀, -SR₉, -S(=O)R₁₁, -S(=O)₂R₁₁, -CH₂-S(=O)₂R₁₁, aryl, aryloxy, benzyloxy, -NR₉-aryl, heterocyclyl, heterocyclyloxy, -NR₉-heterocyclyl, -CH₂-aryl, -CH₂-O-aryl, -CH₂-NR₉-aryl, -CH₂-NR₉-C₁-C₂alkyl, -CH₂-heterocyclyl, -CH₂-O-heterocyclyl and -CH₂-NR₉-heteroeyolyl; wherein the aryl, aryloxy, benzyloxy, -NR₉-aryl, heterocyclyl, heterocyclyloxy and -NR₉-heterocyclyl radicals are unsubstituted or, depending upon the possibilities of substitution at the ring, mono- to penta-substituted by substituents selected from the group consisting of OH, =O, SH, =S, halogen, CN, NO₂, C₁-C₁₂alkyl, C₃-C₈cycloalkyl, C₁-C₁₂haloalkyl, C₁-C₁₂alkoxy, C₁-C₁₂haloalkoxy, C₁-C₁₂alkylthio, C₁-C₁₂haloalkylthio, C₁-C₆alkoxy-C₁-C₆alkyl, C₂-C₈alkenyl, C₂-C₈alkynyl, C₂-C₁₂haloalkenyl, C₂-C₁₂haloalkenyloxy, C₂-C₁₂haloalkynyl, C₃-C₁₂haloalkynyloxy, phenoxy, methylenedioxy, NH₂, NH(C₁₋C₁₂alkyl), N(C₁-C₁₂alkyl)₂ and C₁-C₈alkylsulfinyl; or
R₅ and R₆ are, together with the carbon atom to which they are bound, a five- to seven-membered ring, which may be saturated or unsaturated, and which may contain one or two members selected from the group consisting of O, NR₈ and S; and which is optionally substituted with one to three substituents selected from C₁-C₁₂-alkyJ, CN, NO₂, OH, halogen, halo-C₁-C₂alkyl, C₃-C₈cycloalkyl C₁-C₈halocycloalkyl, C₁-C₁₂alkoxy, C₁-C₆alkoxy-C₁-C₆alkyl, C₁-C₆alkoxy-C₁-C₆alkoxy-C₁-C₆alkyl, C₃-C₈cycloalkoxy, C₁-C₁₂haloalkoxy, C₁-C₁₂alkylthio, C₃-C₈cycloalkylthio, C₁-C₁₂haloalkylthio, C₂-C₁₂alkenyl, C₂-C₁₂haloalkenyl, C₂-C₁₂haloalkenyloxy, C₂-C₁₂alkynyl, C₂-C₁₂haloalkynyl and C₃-C₁₂haloalkynyloxy;
or when p is 1, R₅ together with R₄ is a bond;
or, when p is 0, R₆ together with R₂ and R₄ is ≡N;
R₇ is H, OH, C₁-C₁₂alkyl, C₁-C₁₂haloalkyl, C₂-C₁₂alkenyl, C₂-C₁₂alkynyl, C₂-C₁₂haloalkenyloxy, C₂-C₁₂haloalkynyl, C₃-C₁₂haloalkynyloxy, C₁-C₁₂alkoxy, C₁-C₁₂haloalkoxy, C₁-C₆-alkoxy-C₁-C₆alkyl, C₁-C₆alkoxy-C₁-C₆alkoxy, C₂-C₈alkenyloxy, C₃-C₈alkinyloxy, -N(R₈)₂ wherein the two R₈ are independent of each other, aryl, aryloxy, benzyloxy, heterocyclyl, heterocyclyloxy or heterocyclylmethoxy; and wherein the aryl, aryloxy, benzyloxy, heterocyclyl and heterocyclyloxy radicals are unsubstituted or, depending upon the possibilities of substitution at the ring, mono- to penta-substituted by substituents selected from the group consisting of halogen, CN, NO₂, C₁-C₁₂alkyl, C₃-C₈cycloalkyl, C₁-C₁₂haloalkyl, C₁-C₁₂alkoxy, C₁-C₁₂haJoalkoxy, C₁-C₁₂alkylthio, C₁-C₁₂haloalkylthio, C₁-C₆alkoxy-C₁-C₆alkyl, C₂-C₈alkenyl, C₂-C₁₂haloalkenyl, C₂-C₁₂haloalkenyloxy, C₂-C₈alkynyl, C₂-C₁₂haloalkynyl and C₃-C₁₂haloalkynyloxy;
R₈ is H, C₁-C₆alkyl that is optionally substituted with one to five substituents selected from the group consisting of halogen, C₁-C₆alkoxy, C₁-C₆alkoxy-C₁-C₆alkoxy, C₂-C₁₂alkenyl, C₂-C₁₂haloalkenyl, C₂-C₁₂haloalkenyloxy, C₂-C₁₂alkynyl, C₂-C₁₂haloalkynyl, C₃-C₁₂haloalkynyloxy, hydroxy and cyano, C₃-C₈-cycloalkyl, aryl, benzyl or heteroaryl; wherein the aryl, benzyl and heteroaryl radicals are unsubstituted or, depending on the possibilities of substitution on the ring, mono- to trisubstituted by substituents selected from the group consisting of OH, halogen, CN, NO₂, C₁-C₁₂alkyl, C₁-C₁₂haloalkyl, C₁-C₁₂alkoxy, C₁-C₁₂haloalkoxy, C₁-C₁₂alkylthlo, C₂-C₁₂alkenyl, C₂-C₁₂haloalkenyl, C₂-C₁₂haloalkenyloxy, C₂-C₁₂alkynyl, C₂-C₁₂haloalkynyl, C₃-C₁₂haloalkynyloxy and C₁-C₁₂haloalkylthio;
R₉ is H, C₁-C₆alkyl, C₁-C₆cycloalkyl, C₁-C₆alkoxy-C₁-C₆alkyl, C₁-C₆alkoxy-C₁-C₆alkoxy-C₁-C₆alkyl, C₂-C₁₂alkenyl, C₂-C₁₂alkynyl, benzyl, aryl or heteroaryl;
R₁₀ H, C₁-C₆alkyl that is optionally substituted with one to five substituents selected from the group consisting of halogen, C₁-C₆alkoxy, NO₂, hydroxy and cyano, C₁-C₁₂haloalkyl, C₂-C₁₂alkenyl, C₂-C₁₂haloalkynyl, C₂-C₁₂haloalkenyl, C₂-C₁₂alkynyl, C₃-C₈-cycloalkyl, aryl, benzyl or heteroaryl; wherein the aryl, benzyl and heteroaryl radicals are unsubstituted or, depending on the possibilities of substitution on the ring, mono- to trisubstituted by substituents selected from the group consisting of OH, halogen, CN, NO₂, C₁-C₁₂alkyl, C₁-C₁₂haloalkyl, C₁-C₁₂alkoxy, C₁-C₁₂haloaikoxy, C₁-C₁₂alkylthio, C₁-C₁₂haloalkylthio, C₂-C₁₂alkenyl, C₂-C₁₂haloalkenyl, C₂-C₁₂haloalkenyloxy, C₂-C₁₂alkynyl, C₆-C₁₂haloalkynyl and C₃-C₁₂haloalkynyloxy;
R₁₁ is H, C₁-C₆alkyl that is optionally substituted with one to five substituents selected from the group consisting of halogen, C₁-C₆alkoxy, hydroxy and cyano, -N(R₉)₂ wherein the two substituents R₉ are independent of each other, C₃-C₈cycloalkyl, C₃-C₈halocycloalkyl, C₂-C₁₂alkenyl, C₂-C₁₂haloalkenyl, C₂-C₁₂haloalkenyloxy, C₂-C₁₂alkynyl, C₃-C₁₂haloalkynyl, C₃-C₁₂haloalkynyloxy, aryl, benzyl or heteroaryl; wherein the aryl, benzyl and heteroaryl radicals are unsubstituted or, depending on the possibilities of substitution on the ring, mono- to trisubstituted by substituents selected from the group consisting of OH, halogen, CN, NO₂, C₁-C₁₂alkyl, C₁-C₁₂haloalkyl, C₁-C₁₂alkoxy, C₁-C₁₂haloalkoxy, C₁-C₁₂alkylthio, C₁-C₁₂haloalkylthio, C₂-C₁₂alkenyl, C₂-C₁₂haloalkenyl, C₂-C₁₂haloalkenyloxy, C₂-C₁₂alkynyl, C₂-C₁₂haloalkynyl and C₃-C₁₂haloalkynyloxy;
R₁₂ is H, C₁-C₆alkyl, C₁-C₆cycloalkyl, C₁-C₆alkoxy-C₁-C₆alkyl, C₁-C₆alkoxy-C₁-C₆alkoxy-C₁-C₆alkyl, C₂-C₁₂alkenyl, C₂-C₁₂alkynyl, -C(=O)C₁-C₆alkyl, -C(=O)OC₁-C₆alkyl, -SO₂C₁-C₆alkyl, benzyl, aryl, heteroaryl;
X is O or S;
or, if appropriate, an E/Z isomer, E/Z isomer mixture and/or tautomer thereof, in each case in free form or in salt form;

2. A pesticide which contains at least one compound of the formula (I) as described In claim 1 as active compound and at least one auxiliary.

3. A composition as described in claim 2 for use in a method for controlling pests wherein the composition is applied to the pests or their habitat.

4. A process for preparing a composition as described in claim 2 which contains at least one auxiliary, wherein the active compound is mixed intimately and/or ground with the auxiliary(s).

5. The use of a compound of the formula (I) as described in claim 1 for preparing a composition as described in claim 2.

6. The composition as described in claim 2 for use in controlling pests.

7. A method according to claim 3 for protecting plant propagation material, wherein the propagation material or the location where the propagation material is planted is treated.

8. Plant propagation material treated in accordance with the method described in claim 7.

9. A compound of formula (I) according to claim 1 for use in controlling pests.

10. A compound of formula (I) according to claim 1 for use in protecting domestic animals from infestation by fleas, ticks and nematodes.

## Patentansprüche

1. Eine Verbindung der Formel 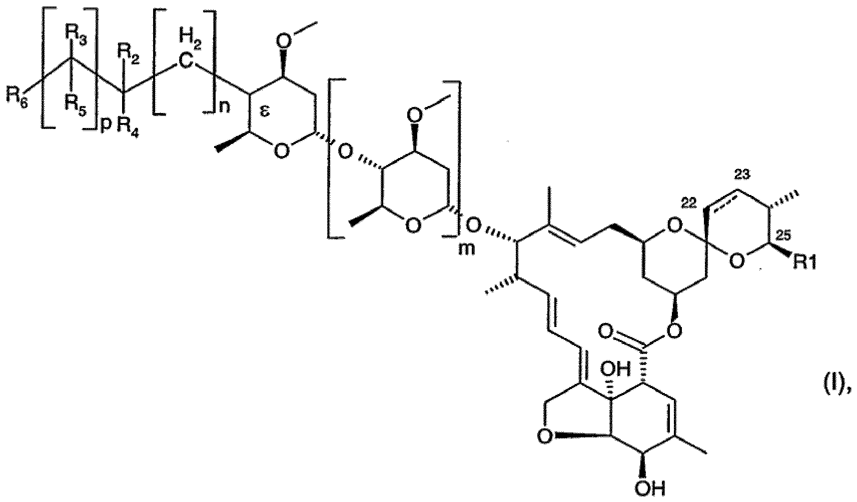 wobei die Bindung der Atome C₂₂ und C₂₃ eine Einfachbindung oder Doppelbindung ist;
m 0 ist;
n 0, 1 oder 2 ist;
p 0 oder 1 ist;
R₁ C₁-C₁₂-Alkyl, C₃-C₈-Cycloalkyl oder C₂-C₁₂-Alkenyl ist;
R₂ H, C₁-C₁₂-Alkyl, C₁-C₁₂-Halogenalkyl, C₁-C₁₂-Hydroxyalkyl, OH, Halogen, -N₃,
SCN, NO₂, CN, C₃-C₈-Cycloalkyl, unsubstituiert oder mit einer bis drei Methylgruppen substituiert, C₃-C₈-Halogencycloalkyl, C₁-C₁₂-Alkoxy, C₁-C₆-Alkoxy-C₁-C₆-alkyl, C₁-C₆-Alkoxy-C₁-C₆-alkoxy, C₁-C₆-Alkoxy-C₁-C₆-alkoxy-C₁-C₆-alkyl, C₂-C₁₂-Alkenyl, C₂-C₁₂-Halogenalkenyl, C₂-C₁₂-Halogenalkenyloxy, C₂-C₁₂-Alkinyl, C₂-C₁₂-Halogenalkinyl, C₃-C₁₂-Alkinyloxy, C₃-C₁₂-Halogenalkinyloxy, -P(=O)(OC₁-C₆-Alkyl)₂, -Si(C₁-C₆-Alkyl)₃, -(CH₂)-Si(C₁-C₆-Alkyl)₃, -Si(OC₁-C₆-Alkyl)₃, -N(R₉)₂, -(CH₂)-N(R₉)₂, wobei die beiden Substituenten R₉ unabhängig voneinander sind, -C(=X)-R₇, -(CH₂)-C(=X)-R₇, -O-C(=X)-R₇, -(CH₂)-O-C(=X)-R₇, -S-C(=X)-R₇, -(CH₂)-S-C(=X)-R₇, -NR₉C(=X)R₇, -(CH₂)-NR₉C(=X)R₇, -NR₉NHC(=X)-R₇, -NR₉-OR₁₀, -(CH₂)-NR₉-OR₁₀, -SR₉, -S(=O)R₁₁, -S(=O)₂R₁₁, Aryl, Heterocyclyl, Aryloxy oder Heterocyclyloxy ist; wobei die Aryl-, Heterocyclyl-, Aryloxy- und Heterocyclyloxyreste unsubstituiert sind oder, abhängig von den Substitutionsmöglichkeiten am Ring, mono- bis penta-substituiert sind mit Substituenten, ausgewählt aus der Gruppe bestehend aus OH, Halogen, CN, NO₂, SCN, -N₃, C₁-C₁₂-Alkyl, C₃-C₈-Cycloalkyl, C₁-C₁₂-Halogenalkyl, C₁-C₁₂-Alkoxy, C₁-C₁₂-Halogenalkoxy, C₁-C₁₂-Alkylthio, C₁-C₁₂-Halogenalkylthio, C₁-C₆-Alkoxy-C₁-C₆-alkyl, C₂-C₈-Alkenyl, C₂-C₈-Alkinyl, C₂-C₁₂-Halogenalkenyl, C₂-C₁₂-Halogenalkenyloxy, C₂-C₁₂-Halogenalkinyl, C₃-C₁₂-Alkinyloxy, C₃-C₁₂-Halogenalkinyloxy und Phenoxy;
oder, wenn p gleich 1 ist, R₂ zusammen mit R₃ eine Bindung ist;
oder R₂ zusammen mit R₄ gleich =O oder =S ist;
oder R₂ zusammen mit R₄ mit dem Kohlenstoff, an den sie gebunden sind, einen drei- bis siebengliedrigen Ring bilden, der monocyclisch oder bicyclisch sein kann und gesättigt oder ungesättigt sein kann und der ein oder zwei Heteroatome, ausgewählt aus der Gruppe bestehend aus N, O und S, enthalten kann und der entweder unsubstituiert ist oder unabhängig voneinander mono- bis penta-substituiert ist mit Substituenten, ausgewählt aus OH, =O, SH, =S, Halogen, CN, -N₃, SCN, NO₂, Aryl, C₁-C₁₂-Alkyl, C₃-C₈-Cycloalkyl, C₁-C₁₂-Halogenalkyl, C₁-C₁₂-Alkoxy, C₁-C₁₂-Halogenalkoxy, C₁-C₁₂-Alkylthio, C₁-C₁₂-Halogenalkylthio, C₁-C₆-Alkoxy-C₁-C₆-alkyl, C₂-C₈-Alkenyl, C₂-C₈-Alkinyl, C₂-C₁₂-Halogenalkenyl, C₂-C₁₂-Halogenalkenyloxy, C₂-C₁₂-Halogenalkinyl, C₃-C₁₂-Alkinyloxy, C₃-C₁₂-Halogenalkinyloxy, Phenoxy, Phenyl-C₁-C₈-Alkyl, -N(R₉)₂, wobei die beiden Reste R₉ unabhängig voneinander sind, C₁-C₆-Alkylsulfinyl, C₃-C₈-Cycloalkylsulfinyl, C₁-C₆-Halogenalkylsulfmyl, C₃-C₈-Halogencycloalkylsulfinyl, C₁-C₈-Alkylsulfonyl, C₃-C₈-Cycloalkylsulfonyl, C₁-C₆-Halogenalkylsulfonyl und C₃-C₈-Halogencycloalkylsulfonyl; oder
R₂ zusammen mit R₄ gleich =NN(R₁₂)₂ ist, wobei die beiden Substituenten R₉ voneinander unabhängig sind;
oder, wenn p gleich 0 ist, R₂ zusammen mit R₄ und R₆ gleich ≡N ist;
oder, wenn p gleich 0 ist, R₂ zusammen mit R₈ gleich =NOR₁₂ oder =NN(R₁₂)₂ ist, wobei die beiden Substituenten R₉ voneinander unabhängig sind;
R₃ H, C₁-C₁₂-Alkyl, Halogen, Halogen-C₁-C₂-alkyl, CN, -N₃, SCN, NO₂, C₃-C₈-Cycloalkyl, unsubstituiert oder mit einer bis drei Methylgruppen substituiert, C₃-C₈-Halogencycloalkyl, C₁-C₁₂-Alkoxy, C₁-C₆-Alkoxy-C₁-C₆-alkyl, C₁-C₆-Alkoxy-C₁-C₆-alkoxy-C₁-C₆-alkyl, C₃-C₈-Cycloalkoxy, C₁-C₁₂-Halogenalkoxy, C₁-C₁₂-Alkylthio, C₃-C₈-Cycloalkylthio, C₁-C₁₂-Halogenalkylthio, C₁-C₁₂-Alkylsulfonyl, C₃-C₈-Cycloalkylsulfmyl, C₁-C₁₂-Halogenalkylsulfinyl, C₃-C₈-Halogencycloalkylsulfinyl, C₁-C₁₂-Alkylsulfonyl, C₃-C₈-Cycloalkylsulfonyl, C₁-C₁₂-Halogenalkylsulfonyl, C₃-C₈-Halogencycloalkylsulfonyl, C₂-C₈-Alkenyl, C₂-C₈-Alkinyl, C₂-C₁₂-Halogenalkenyl, C₂-C₁₂-Halogenalkenyloxy, C₂-C₁₂-Halogenalkinyl, C₃-C₁₂-Halogenalkinyloxy, -N(R₉)₂, wobei die beiden Substituenten R₉ voneinander unabhängig sind, Aryl, Heterocyclyl, Aryloxy oder Heterocyclyloxy ist; wobei die Aryl-, Heterocyclyl-, Aryloxy- und Heterocyclyloxyreste unsubstituiert sind oder, abhängig von den Substitutionsmöglichkeiten am Ring, mono- bis penta-substituiert sind mit Substituenten, ausgewählt aus der Gruppe bestehend aus Halogen, CN, NO₂, C₁-C₁₂-Alkyl, C₃-C₈-Cycloalkyl, C₁-C₁₂-Halogenalkyl, C₁-C₁₂-Alkoxy, C₁-C₁₂-Halogenalkoxy, C₁-C₁₂-Alkylthio, C₁-C₁₂-Halogenalkylthio, C₁-C₆-Alkoxy-C₁-C₆-alkyl, C₂-C₈-Alkenyl, C₂-C₈-Alkinyl, C₂-C₁₂-Halogenalkenyl, C₂-C₁₂-Halogenalkenyloxy, C₂-C₁₂-Halogenalkinyl und C₃-C₁₂-Halogenalkinyloxy;
oder, wenn p gleich 1 ist, R₃ zusammen mit R₂ eine Bindung ist;
R₄ H, C₁-C₁₂-Alkyl, C₁-C₁₂-Halogenalkyl, C₁-C₁₂-Hydroxyalkyl, OH, Halogen, NO₂, CN, C₃-C₈-Cycloalkyl, unsubstituiert oder mit einer bis drei Methylgruppen substituiert, C₃-C₈-Halogencycloalkyl, C₁-C₁₂-Alkoxy, C₁-C₆-Alkoxy-C₁-C₆-alkyl, C₁-C₆-Alkoxy-C₁-C₆-alkoxy, C₁-C₆-Alkoxy-C₁-C₆-alkoxy-C₁-C₆-alkyl, C₂-C₁₂-Alkenyl, C₂-C₁₂-Halogenalkenyl, C₂-C₁₂-Halogenalkenyloxy, C₂-C₁₂-Alkinyl, C₂-C₁₂-Halogenalkinyl, C₃-C₁₂-Halogenalkinyloxy, -P(=O)(OC₁-C₆-Alkyl)₂, -Si(C₁-C₆-Alkyl)₃, -(CH₂)-Si(C₁-C₆-Alkyl)₃, -Si(OC₁-C₆-Alkyl)₃, -N(R₉)₂, -(CH₂)-N(R₉)₂, wobei die beiden Substituenten R₉ unabhängig voneinander sind, -C(=X)-R₇, -(CH₂)-C(=X)-R₇, -O-C(=X)-R₇, -(CH₂)-O-C(=X)-R₇, -S-C(=X)-R₇, -(CH₂)-S-C(=X)-R₇, -NR₉C(=X)R₇, -(CH₂)-NR₉C(=X)R₇, -NR₉NHC(=X)-R₇, -NR₉-OR₁₀, -(CH₂)-NR₉-OR₁₀, -SR₉, -S(=O)R₁₁,
-S(=O)₂R₁₁, Aryl, Heterocyclyl, Aryloxy oder Heterocyclyloxy ist; wobei die Aryl-, Heterocyclyl-, Aryloxy- und Heterocyclyloxyreste unsubstituiert sind oder, abhängig von den Substitutionsmöglichkeiten am Ring, mono- bis penta-substituiert sind mit Substituenten, ausgewählt aus der Gruppe bestehend aus OH, Halogen, CN, NO₂, C₁-C₁₂-Alkyl, C₃-C₈-Cycloalkyl, C₁-C₁₂-Halogenalkyl, C₁-C₁₂-Alkoxy, C₁-C₁₂-Halogenalkoxy, C₁-C₁₂-Alkylthio, C₁-C₁₂-Halogenalkylthio, C₁-C₆-Alkoxy-C₁-C₆-alkyl, C₂-C₈-Alkenyl, C₂-C₈-Alkinyl, C₂-C₁₂-Halogenalkenyl, C₂-C₁₂-Halogenalkenyloxy, C₂-C₁₂-Halogenalkinyl, C₃-C₁₂-Halogenalkinyloxy und Phenoxy;
oder R₄ zusammen mit R₂ =O oder =S bildet;
oder, wenn p gleich 1 ist, R₄ zusammen mit R₅ eine Bindung ist;
oder, wenn p gleich 0 ist, zusammen mit R₂ und R₆ gleich ≡N ist;
R₅ und R₆ unabhängig voneinander H, C₁-C₁₂-Alkyl, -N₃, CH, NO₂, OH, SH, Halogen, Halogen-C₁-C₂-alkyl, Hydroxy-C₁-C₂-alkyl, C₃-C₈-Cycloalkyl, unsubstituiert oder mit einer bis drei Methylgruppen substituiert, C₃-C₈-Halogencycloalkyl, C₁-C₁₂-Alkoxy, C₁-C₆-Alkoxy-C₁-C₆-alkyl, C₁-C₆-Alkoxy-C₁-C₆-alkoxy, C₁-C₆-Alkoxy-C₁-C₆-alkoxy-C₁-C₆-alkyl, C₃-C₈-Cycloalkoxy, C₁-C₁₂-Halogenalkoxy, C₁-C₁₂-Halogenalkylthio, C₂-C₈-Alkenyl, C₂-C₈-Alkinyl, C₂-C₁₂-Halogenalkenyl, C₂-C₁₂-Halogenalkenyloxy, C₂-C₁₂-Halogenalkinyl, C₃-C₁₂-Halogenalkinyloxy, -P(=O)(OC₁-C₆-Alkyl)₂, -CH₂-P(=O)(OC₁-C₆-Alkyl)₂, -Si(OC₁-C₆-Alkyl)₃, -N(R₉)₂, -O-N(R₉)₂, wobei die beiden Substituenten R₉ unabhängig voneinander sind, -C(=X)-R₇, -CH=NOH, -CH=NOC₁-C₆-Alkyl, -O-C(=X)-R₇, -S-C(=X)-R₇, -NR₉C(=X)R₇, -NR₉NHC(=X)-R₇, -NR₉-OR₁₀, -SR₉, -S(=O)R₁₁, -S(=O)₂R₁₁, -CH₂-S(=O)₂R₁₁, Aryl, Aryloxy, Benzyloxy, -NR₉-Aryl, Heterocyclyl, Heterocyclyloxy, -NR₉-Heterocyclyl, -CH₂-Aryl, -CH₂-O-Aryl, -CH₂-NR₉-Aryl, -CH₂-NR₉-C₁-C₂-Alkyl, -CH₂-Heterocyclyl, -CH₂-O-Heterocyclyl und -CH₂-NR₉-Heterocyclyl sind; wobei die Aryl-, Aryloxy-, Benzyloxy-, -NR₉-Aryl-, Heterocyclyl-, Heterocyclyloxy- und -NR₉-Heterocyclylreste unsubstituiert sind oder, abhängig von den Substitutionsmöglichkeiten am Ring, mono- bis penta-substituiert sind mit Substituenten, ausgewählt aus der Gruppe bestehend aus OH, =O, SH, =S, Halogen, CN, NO₂, C₁-C₁₂-Alkyl, C₃-C₈-Cycloalkyl, C₁-C₁₂-Halogenalkyl, C₁-C₁₂-Alkoxy, C₁-C₁₂-Halogenalkoxy, C₁-C₁₂-Alkylthio, C₁-C₁₂-Halogenalkylthio, C₁-C₆-Alkoxy-C₁-C₆-alkyl, C₂-C₈-Alkenyl, C₂-C₈-Alkinyl, C₂-C₁₂-Halogenalkenyl, C₂-C₁₂-Halogenalkenyloxy, C₂-C₁₂-Halogenalkinyl, C₃-C₁₂-Halogenalkinyloxy, Phenoxy, Methylendioxy, NH₂, NH(C₁-C₁₂-Alkyl), N(C₁-C₁₂-Alkyl)₂ und C₁-C₈-Alkylsulfonyl, oder
R₅ und R₆, zusammen mit dem Kohlenstoffatom, an das sie gebunden sind, ein fünf- bis siebengliedriger Ring sind, der gesättigt oder ungesättigt sein kann und der ein oder zwei Glieder, ausgewählt aus der Gruppe bestehend aus O, NR₈ und S, enthalten kann; und der gegebenenfalls mit einem bis drei Substituenten, ausgewählt aus C₁-C₁₂-Alkyl, CN, NO₂, OH, Halogen, Halogen-C₁-C₂-alkyl, C₃-C₈-Cycloalkyl, C₃-C₈-Halogencycloalkyl, C₁-C₁₂-Alkoxy, C₁-C₆-Alkoxy-C₁-C₆-alkyl, C₁-C₆-Alkoxy-C₁-C₆-alkoxy-Cₗ-C₆-alkyl, C₃-C₈-Cycloalkoxy, C₁-C₁₂-Halogenalkoxy, C₁-C₁₂-Alkylthio, C₃-C₈-Cycloalkylthio, C₁-C₁₂-Halogenalkylthio, C₂-C₁₂-Alkenyl, C₂-C₁₂-Halogenalkenyl, C₂-C₁₂-Halogenalkenyloxy, C₂-C₁₂-Alkinyl, C₂-C₁₂₋Halogenalkinyl und C₃-C₁₂-Halogenalkinyloxy substituiert ist;
oder, wenn p gleich 1 ist, R₅ zusammen mit R₄ eine Bindung ist;
oder, wenn p gleich 0 ist, R₆ zusammen mit R₂ und R₄ gleich ≡N ist;
R₇ H, OH, C₁-C₁₂-Alkyl, C₁-C₁₂-Halogenalkyl, C₂-C₁₂-Alkenyl, C₂-C₁₂-Alkinyl, C₂-C₁₂-Halogenalkenyloxy, C₂-C₁₂-Halogenalkinyl, C₃-C₁₂-Halogenalkinyloxy, C₁-C₁₂-Alkoxy, C₁-C₁₂-Halogenalkoxy, C₁-C₆-Alkoxy-C₁-C₆-alkyl, C₁-C₆-Alkoxy-C₁-C₆-alkoxy, C₂-C₈-Alkenyloxy, C₃-C₈-Alkinyloxy, -N(R₈)₂ ist, wobei die beiden Reste R₈ unabhängig voneinander sind, Aryl, Aryloxy, Benzyloxy, Heterocyclyl, Heterocyclyloxy oder Heterocyclylmethoxy ist; und wobei die Aryl-, Aryloxy-, Benzyloxy-, Heterocyclyl-, und Heterocyclyloxyreste unsubstituiert sind oder, abhängig von den Substitutionsmöglichkeiten am Ring, mono- bis penta-substituiert sind mit Substituenten, ausgewählt aus der Gruppe bestehend aus Halogen, CN, NO₂, C₁-C₁₂-Alkyl, C₃-C₈-Cycloalkyl, C₁-C₁₂-Halogenalkyl, C₁-C₁₂-Alkoxy, C₁-C₁₂-Halogenalkoxy, C₁-C₁₂-Alkylthio, C₁-C₁₂-Halogenalkylthio, C₁-C₆-Alkoxy-C₁-C₆-alkyl, C₂-C₈-Alkenyl, C₂-C₁₂-Halogenalkenyl, C₂-C₁₂-Halogenalkenyloxy, C₂-C₈-Alkinyl, C₂-C₁₂-Halogenalkinyl und C₃-C₁₂-Halogenalkinyloxy;
R₈ H, C₁-C₆-Alkyl, gegebenenfalls substituiert mit einem bis fünf Substituenten, ausgewählt aus der Gruppe bestehend aus Halogen, C₁-C₆-Alkoxy, C₁-C₆-Alkoxy-C₁-C₆-alkoxy, C₂-C₁₂-Alkenyl, C₂-C₁₂-Halogenalkenyl, C₂-C₁₂-Halogenalkenyloxy, C₂-C₁₂-Alkinyl, C₂-C₁₂-Halogenalkinyl, C₃-C₁₂-Halogenalkinyloxy, Hydroxy und Cyano, C₃-C₈-Cycloalkyl, Aryl, Benzyl oder Heteroaryl, ist; wobei die Aryl-, Benzyl- und Heteroarylreste unsubstituiert sind oder, abhängig von den Substitutionsmöglichkeiten am Ring, mono- bis trisubstituiert sind mit Substituenten, ausgewählt aus der Gruppe bestehend aus OH, Halogen, CN, NO₂, C₁-C₁₂-Alkyl, C₁-C₁₂-Halogenalkyl, C₁-C₁₂-Alkoxy, C₁-C₁₂-Halogenalkoxy, C₁-C₁₂-Alkylthio, C₂-C₁₂-Alkenyl, C₂-C₁₂-Halogenalkenyl, C₂-C₁₂-Halogenalkenyloxy, C₂-C₁₂-Alkinyl, C₂-C₁₂-Halogenalkinyl, C₃-C₁₂-Halogenalkinyloxy und C₁-C₁₂-Halogenalkylthio;
R₉ H, C₁-C₆-Alkyl, C₁-C₆-Cycloalkyl, C₁-C₆-Alkoxy-C₁-C₆-alkyl, C₁-C₆-Alkoxy-C₁-C₆-alkoxy-C₁-C₆-alkyl, C₂-C₁₂-Alkenyl, C₂-C₁₂-Alkinyl, Benzyl, Aryl oder Heteroaryl ist;
R₁₀ H, C₁-C₆-Alkyl, gegebenenfalls substituiert mit einem bis fünf Substituenten, ausgewählt aus der Gruppe bestehend aus Halogen, C₁-C₆-Alkoxy, NO₂, Hydroxy und Cyano, C₁-C₁₂-Halogenalkyl, C₂-C₁₂-Alkenyl, C₂-C₁₂-Halogenalkinyl, C₂-C₁₂-Halogenalkenyl, C₂-C₁₂-Alkinyl, C₃-C₈-Cycloalkyl, Aryl, Benzyl oder Heteroaryl, ist; wobei die Aryl-, Benzyl- und Heteroarylreste unsubstituiert sind oder, abhängig von den Substitutionsmöglichkeiten am Ring, mono- bis trisubstituiert sind mit Substituenten, ausgewählt aus der Gruppe bestehend aus OH, Halogen, CN, NO₂, C₁-C₁₂-Alkyl, C₁-C₁₂-Halogenalkyl, C₁-C₁₂-Alkoxy, C₁-C₁₂-Halogenalkoxy, C₁-C₁₂-Alkylthio, C₁-C₁₂-Halogenalkylthio, C₂-C₁₂-Alkenyl, C₂-C₁₂-Halogenalkenyl, C₂-C₁₂-Halogenalkenyloxy, C₂-C₁₂-Alkinyl, C₃-C₁₂-Halogenalkinyl und C₃-C₁₂-Halogenalkinyloxy;
R₁₁ H, C₁-C₆-Alkyl, gegebenenfalls substituiert mit einem bis fünf Substituenten, ausgewählt aus der Gruppe bestehend aus Halogen, C₁-C₆-Alkoxy, Hydroxy und Cyano, -N(R₉)₂, wobei die beiden Substituenten R₉ unabhängig voneinander sind, C₃-C₈-Cycloalkyl, C₃-C₈-Halogencycloalkyl, C₂-C₁₂-Alkenyl, C₂-C₁₂-Halogenalkenyl, C₂-C₁₂-Halogenalkenyloxy, C₂-C₁₂-Alkinyl, C₃-C₁₂-Halogenalkinyl, C₃-C₁₂-Halogenalkinyloxy, Aryl, Benzyl oder Heteroaryl ist; wobei die Aryl-, Benzyl- und Heteroarylreste unsubstituiert sind oder, abhängig von den Substitutionsmöglichkeiten am Ring, mono- bis trisubstituiert sind mit Substituenten, ausgewählt aus der Gruppe bestehend aus OH, Halogen, CN, NO₂, C₁-C₁₂-Alkyl, C₁-C₁₂-Halogenalkyl, C₁-C₁₂-Alkoxy, C₁-C₁₂-Halogenalkoxy, C₁-C₁₂-Alkylthio, C₁-C₁₂-Halogenalkylthio, C₂-C₁₂-Alkenyl, C₂-C₁₂-Halogenalkenyl, C₂-C₁₂-Halogenalkenyloxy, C₂-C₁₂-Alkinyl, C₂-C₁₂-Halogenalkinyl und C₃-C₁₂-Halogenalkinyloxy;
R₁₂ H, C₁-C₆-Alkyl, C₁-C₆-Cycloalkyl, C₁-C₆-Alkoxy-C₁-C₆-alkyl, C₁-C₆-Alkoxy-C₁-C₆-alkoxy-C₁-C₆-alkyl, C₂-C₁₂-Alkenyl, C₂-C₁₂-Alkinyl, -C(=O)-C₁-C₆-Alkyl, -C(=O)-OC₁-C₆-Alkyl, -SO₂-C₁-C₆-Alkyl, Benzyl, Aryl, Heteroaryl ist;
X O oder S ist;
oder, wenn angebracht, ein E/Z-Isomer, E/Z-Isomerengemisch und/oder Tautomer davon, jeweils in freier Form oder in Form eines Salzes.

2. Ein Pestizid, das mindestens eine Verbindung der Formel (I) wie in Anspruch 1 beschrieben als Wirkstoff und mindestens einen Hilfsstoff enthält.

3. Eine Zusammensetzung wie in Anspruch 2 beschrieben zur Verwendung bei einem Verfahren zur Schädlingsbekämpfung, wobei die Zusammensetzung auf die Schädlinge oder deren Lebensraum aufgebracht wird.

4. Ein Verfahren zur Herstellung einer Zusammensetzung wie in Anspruch 2 beschrieben, die mindestens einen Hilfsstoff enthält, wobei der Wirkstoff mit dem/-n Hilfsstoff(en) gründlich vermischt und/oder vermahlen wird.

5. Die Verwendung einer Verbindung der Formel (I) wie in Anspruch 1 beschrieben zur Herstellung einer Zusammensetzung wie in Anspruch 2 beschrieben.

6. Die Zusammensetzung wie in Anspruch 2 beschrieben zur Verwendung bei der Schädlingsbekämpfung.

7. Ein Verfahren gemäß Anspruch 3 zum Schutz von Pflanzenvermehrungsmaterial, wobei das Vermehrungsmaterial oder der Ort, an dem das Vermehrungsmaterial gepflanzt wird, behandelt wird.

8. Gemäß dem in Anspruch 7 beschriebenen Verfahren behandeltes Pflanzenvermehrungsmaterial.

9. Eine Verbindung der Formel (I) gemäß Anspruch 1 zur Verwendung bei der Schädlingsbekämpfung.

10. Eine Verbindung der Formel (I) gemäß Anspruch 1 zur Verwendung beim Schutz von Haustieren vor einem Befall mit Flöhen, Zecken und Nematoden.

## Revendications

1. Composé de formule 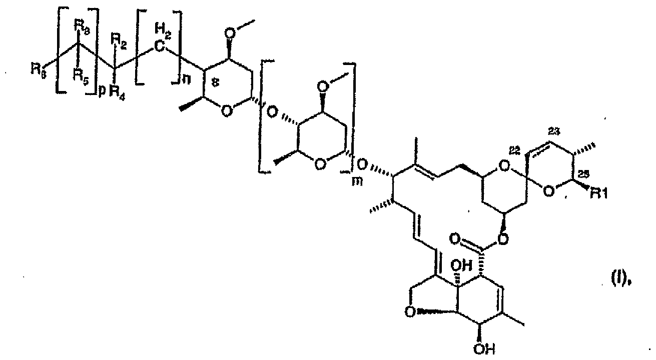 dans laquelle la liaison des atomes C₂₂ et C₂₃ est une liaison simple ou une double liaison ;
m vaut 0;
n vaut 0, 1 ou 2 ;
p vaut 0 ou 1 ;
R₁ est un groupe alkyle en C₁-C₁₂, cycloalkyle en C₃-C₈ ou alcényle en C₂-C₁₂ ;
R₂ est H, un groupe alkyle en C₁-C₁₂, halogénoalkyle en C₁-C₁₂, hydroxyalkyle en C₁-C₁₂, OH, halogéno, -N₃, SCN, NO₂, CN, cycloalkyle en C₃-C₈ non substitué ou substitué par un à trois groupes méthyle, halogénocycloalkyle en C₃-C₈, alcoxy en C₁-C₁₂, (alcoxy en C₁-C₆)-alkyle en C₁-C₆, (alcoxy en C₁-C₆)-alcoxy en C₁-C₆, (alcoxy en C₁-C₆)-(alcoxy en C₁-C₆)-alkyle en C₁-C₆, alcényle en C₂-C₁₂, halogénoalcényle en C₂-C₁₂, halogénoalcényloxy en C₂-C₁₂, alcynyle en C₂-C₁₂, halogénoalcynyle en C₂-C₁₂, alcynyloxy en C₃-C₁₂, halogénoalcynyloxy en C₃-C₁₂, -P(=O)(O-alkyle en C₁-C₆)₂, -Si(alkyle en C₁-C₆)₃, -(CH₂)-Si(alkyle en C₁-C₆)₃, -Si(O-allcyle en C₁-C₆)₃, -N(R₉)₂, -(CH₂)-N(R₉)₂, où les deux substituants R₉ sont indépendants l'un de l'autre, -C(=X)-R₇, -(CH₂)-C(=X)-R₇, -O-C(=X)-R₇, -(CH₂)-O-C(=X)-R₇, -S-C(=X)-R₇, -(CH₂)-S-C(=X)-R₇, -NR₉C(=X)-R₇, -(CH₂)-NR₉C(=X)-R₇, -NR₉NHC(=X)-R₇, -NR₉-OR₁₀, -(CH₂)-NR₉-OR₁₀, -SR₉, S(=O)R₁₁, -S(=O)₂R₁₁, aryle, hétérocyclyle, aryloxy ou hétéroaryloxy ; où les radicaux aryle, hétércyclyle, aryloxy et hétérocyclyloxy sont non substitués ou, en fonction des possibilités de substitution sur le cycle, ils sont mono- à penta-substitués par des substituants choisis dans le groupe constitué de OH, halogéno, CN, NO₂, SCN, -N₃, alkyle en C₁-C₁₂, cycloalkyle en C₃-C₆, halogénoalkyle en C₁-C₁₂, alcoxy en C₁-C₁₂, halogénoalcoxy en C₁-C₁₂, alkylthio en C₁-C₁₂, halogénoalkylthio en C₁-C₁₂, (alcoxy en C₁-C₆)-alkyle en C₁-C₆, alcényle en C₂-C₈, alcynyle en C₂-C₈, halogénoacényle en C₂-C₁₂, halogénoacényloxy en C₂-C₁₂, halogénoalcynyle en C₂-C₁₂, alcynyloxy en C₃-C₁₂, halogénoalcynyloxy en C₃-C₁₂ et phénoxy ;
ou, lorsque p vaut 1, R₂ conjointement à R₃ est une liaison ;
ou R₂ conjointement à R₄ est =O ou =S ;
ou R₂ conjointement à R₄ forme, avec l'atome de carbone auquel ils sont liés, un cycle de trois à sept chaînons, qui peut être monocyclique ou bicyclique, et qui peut être saturé ou insaturé, et qui peut contenir un ou deux hétéroatomes choisis dans le groupe constitué de N, O et S, et qui est non substitué ou indépendamment l'un de l'autre mono- à penta-substitué par des substituants choisis parmi OH, =O, SH, =S, halogéno, CN, -N₃, SCN, NO₂, aryle, alkyle en C₁-C₁₂, cycloalkyle en C₃-C₈, halogénoalkyle en C₁-C₁₂, alcoxy en C₁-C₁₂, halogénoalcoxy en C₁-C₁₂, alkylthio en C₁-C₁₂, halogénoalkylthio en C₁-C₁₂, (alcoxy en C₁-C₆)-alkyle en C₁-C₆, alcényle en C₂-C₈, alcynyle en C₂-C₈, halogénoalcényle en C₂-C₁₂, halogénoalcényloxy en C₂-C₁₂, halogénoalcynyle en C₂-C₁₂, alcynyloxy en C₃-C₁₂, halogénoalcynyloxy en C₃-C₁₂, phénoxy, phényl-alkyle en C₁-C₈, -N(R₉)₂, où les deux substituants R₉ sont indépendants l'un de l'autre, alkylsulfinyle en C₁-C₆, cycloalkylsulfinyle en C₃-C₈, halogénoalkylsulfinyle en C₁-C₆, halogénocycloalkylsulfinyle en C₃-C₈, alkylsulfonyle en C₁-C₆, cycloalkylsulfonyle en C₃-C₈, halogénoalkylsulfonyle en C₁-C₆ et halogénocycloalkylsulfonyle en C₃-C₈ ; ou
R₂ conjointement à R₄ est =NN(R₁₂)₂, où les deux substituants R₉ sont indépendants l'un de l'autre ;
ou, lorsque p vaut 0, R₂ conjointement à R₄ et R₈ est ≡N ;
ou, lorsque p vaut 0, R₂ conjointement à R₈ est =NOR₁₂ ou =NN(R₁₂)₂, où les deux substituants R₉ sont indépendants l'un de l'autre ;
R₃ est H, un groupe alkyle en C₁-C₁₂, halogéno, halogénoalkyle en C₁-C₂, CN, -N₃, SCN, NO₂, cycloalkyle en C₃-C₈ non substitué ou substitué par un à trois groupes méthyle, halogénocycloalkyle en C₃-C₈, alcoxy en C₁-C₁₂, (alcoxy en C₁-C₆)-alkyle en C₁-C₆, (alcoxy en C₁-C₆)-(alcoxy en C₁-C₆)-alkyle en C₁-C₆, cycloalcoxy en C₃-C₈, halogénoalcoxy en C₁-C₁₂, alkylthio en C₁-C₁₂, cycloalkylthio en C₃-C₈, halogénoalkylthio en C₁-C₁₂, alkylsulfinyle en C₁-C₁₂, cycloalkylsulfinyle en C₃-C₈, halogénoalkylsulfinyle en C₁-C₁₂, halogénocycloalkylsulfinyle en C₃-C₈, alkylsulfonyle en C₁-C₁₂, cycloalkylsulfonyle en C₃-C₈, halogénoalkylsulfonyle en C₁-C₁₂, halogénocycloalkylsulfonyle en C₃-C₈, alcényle en C₂-C₈, alcynyle en C₂-C₈, halogénoalcényle en C₂-C₁₂, halogénoalcényloxy en C₂-C₁₂, halogénoalcynyle en C₂-C₁₂, halogénoalcynyloxy en C₃-C₁₂, -N(R₉)₂, où les deux substituants R₉ sont indépendants l'un de l'autre, aryle, hétérocyclyle, aryloxy ou hétérocyclyloxy ; où les radicaux aryle, hétérocyclyle, aryloxy et hétérocyclyloxy sont non substitués ou, en fonctions des possibilités de substitution sur le cycle, ils sont mono- à penta-substitués par des substituants choisis dans le groupe constitué de halogéno, CN, NO₂, alkyle en C₁-C₁₂, cycloalkyle en C₃-C₆, halogénoalkyle en C₁-C₁₂, alcoxy en C₁-C₁₂, halogénoalcoxy en C₁-C₁₂, alkylthio en C₁-C₁₂, halogénoalkylthio en C₁-C₁₂, (alcoxy en C₁-C₆)-alkyle en C₁-C₆, alcényle en C₂-C₈, alcynyle en C₂-C₈, halogénoalcényle en C₂-C₁₂, halogénoalcényloxy en C₂-C₁₂, halogénoalcynyle en C₂-C₁₂ et halogénoalcynyloxy en C₃-C₁₂ ;
ou lorsque p vaut 1, R₃ conjointement à R₂ est une liaison ;
R₄ est H, un groupe alkyle en C₁-C₁₂, halogénoalkyle en C₁-C₁₂, hydroxyalkyle en C₁-C₁₂, OH, halogéno, NO₂, CN, cycloalkyle en C₃-C₈ non substitué ou substitué par un à trois groupes méthyle, halogénocycloalkyle en C₃-C₈, alcoxy en C₁-C₁₂, (alcoxy en C₁-C₆)-alkyle en C₁-C₆, (alcoxy en C₁-C₆)-alcoxy en C₁-C₆, (alcoxy en C₁-C₆)-(alcoxy en C₁-C₆)-alkyle en C₁-C₆, alcényle en C₂-C₁₂, halogénoalcényle en C₂-C₁₂, halogénoalcényloxy en C₂-C₁₂, alcynyle en C₂-C₁₂, halogénoalcynyle en C₂-C₁₂, halogénoalcynyloxy en C₃-C₁₂, -P(=O)(O-alkyle en C₁-C₆)₂, -Si(alkyle en C₁-C₆)₃, -(CH₂)-Si(alkyle en C₁-C₆)₃, -Si(O-alkyle en C₁-C₆)₃, -N(R₉)₂, -(CH₂)-N(R₉)₂, où les deux substituants R₉ sont indépendants l'un de l'autre, -C(=X)-R₇, -(CH₂)-C(=X)-R₇, -O-C(=X)-R₇, -(CH₂)-O-C(=X)-R₇, -S-C(=X)-R₇, -(CH₂)-S-C(=X)-R₇, -NR₉C(=X)-R₇, -(CH₂)-NR₉C(=X)-R₇, -NR₉NHC(=X)-R₇, -NR₉-OR₁₀, -(CH₂)-NR₉-OR₁₀, -SR₉, S(=O)R₁₁, -S(=O)₂R₁₁, aryle, hétérocyelyle, aryloxy ou hétérocyclyloxy ; où les radicaux aryle, hétércyclyle, aryloxy et hétérocyclyloxy sont non substitués ou, en fonction des possibilités de substitution sur le cycle, ils sont mono- à penta-substitués par des substituants choisis dans le groupe constitué de OH, halogéno, CN, NO₂, alkyle en C₁-C₁₂, cycloalkyle en C₃-C₈, halogénoalkyle en C₁-C₁₂, alcoxy en C₁-C₁₂, halogénoalcoxy en C₁-C₁₂, alkylthio en C₁-C₁₂, halogénoalkylthio en C₁-C₁₂, (alcoxy en C₁-C₆)-alkyle en C₁-C₆, alcényle en C₂-C₈, alcynyle en C₂-C₈, halogénoacényle en C₂-C₁₂, halogénoacényloxy en C₂-C₁₂, halogénoalcynyle en C₂-C₁₂, halogénoalcynyle en C₂-C₁₂, halogénoalcynyloxy en C₃-C₁₂ et phénoxy ;
ou R₄ conjointement à R₂ forme =O ou =S ;
ou, lorsque p vaut 1, R₄ conjointement à R₅ est une liaison ;
ou, lorsque p vaut 0, conjointement avec R₂ et R₈ est ≡N ;
R₅ et R₆ sont indépendamment l'un de l'autre H, un groupe alkyle en C₁-C₁₂, -N₃, CN, NO₂, OH, SH, halogéno, halogénoallsyle en C₁-C₂, hydroxyalkyle en C₁-C₂, cycloalkyle en C₃-C₈ qui est non substitué ou substitué par un à deux groupes méthyle, halogénocycloalkyle en C₃-C₈, alcoxy en C₁-C₁₂, (alcoxy en C₁-C₆)-alkyle en C₁-C₆, (alcoxy en C₁-C₆)-alcoxy en C₁-C₆, (alcoxy en C₁-C₆)-(alcoxy en C₁-C₆)-alkyle en C₁-C₆, cycloalcoxy en C₃-C₈, halogénoalcoxy en C₁-C₁₂, halogénoalkylthio en C₁-C₁₂, alcényle en C₂-C₈, alcynyle en C₂-C₈, halogénoalcényle en C₂-C₁₂, halogénoalcényloxy en C₂-C₁₂, halogénoalcynyle en C₂-C₁₂, halogénoalcynyloxy en C₂-C₁₂, -P(=O)(O-alkyle en C₁-C₆)₂, -(CH₂)-P(=O)(O-alkyle en C₁-C₆)₂, -Si(O-alkyle en C₁-C₆)₃, -N(R₉)₂, -O-N(R₉)₂, où les deux substituants R₉ sont indépendants l'un de l'autre, -C(=X)-R₇, -CH=NOH, -CH=NO-alkyle en C₁-C₈, -O-C(=X)-R₇, -S-C(=X)-R₇, -NR₉C(=X)-R₇, -NR₉NHC(=X)-R₇, -NR₉-OR₁₀, -SR₉, -S(=O)R₁₁, -S(=O)₂R₁₁, - (CH₂)-S(=O)₂R₁₁, aryle, aryloxy, benzyloxy, -NR₉-aryle, hétérocyclyle, hétérocyclyloxy, -NR₉-hétérocyclyle, -(CH₂)-aryle, -(CH₂)-O-aryle, -(CH₂)-NR₉-aryle, -(CH₂)-NR₉-alkyle en C₁-C₂, -(CH₂)-hétérocyclyle, -(CH₂)-O-hétérocylyle et -(CH₂)-NR₉-hétérocyclyle ; où les radicaux aryle, aryloxy, benzyloxy, -NR₉-aryle, hétérocyclyle, hétérocyclyloxy et -NR₉-hétérocyclyle sont non substitués ou, en fonction des possibilités de substitution sur le cycle, ils sont mono- à penta-substitués par des substituants choisis dans le groupe constitué de OH, =O, SH, =S, halogéno, CN, NO₂, alkyle en C₁-C₁₂, cycloalkyle en C₃-C₈, halogénoalkyle en C₁-C₁₂, alcoxy en C₁-C₁₂, halogénoalcoxy en C₁-C₁₂, alkylthio en C₁-C₁₂, halogénoalkylthio en C₁-C₁₂, (alcoxy en C₁-C₆)-alkyle en C₁-C₆, alcényle en C₂-C₆, alcynyle en C₂-C₈, halogénoacényle en C₂-C₁₂, halogénoacényloxy en C₂-C₁₂, halogénoalcynyle en C₂-C₁₂, halogénoalcynyloxy en C₃-C₁₂, phénoxy, méthylènedioxy, NH₂, NH-alkyle en C₁-C₁₂, N(alkyle en C₁-C₁₂)₂ et alkylsulfinyle en C₁-C₆ ; ou
ou R₅ et R₆ forment, avec l'atome de carbone auquel ils sont liés, un cycle de cinq à sept chaînons, qui peut saturé ou insaturé, et qui peut contenir un ou deux éléments choisis dans le groupe constitué de O, NR₈ et S ; et qui est éventuellement substitué par un à trois substituants choisis parmi un groupe alkyle en C₁-C₁₂, CN, NO₂, OH, halogéno, halogénoalkyle en C₁-C₂, cycloalkyle en C₃-C₈, halogénocycloalkyle en C₃-C₈, alcoxy en C₁-C₁₂, (alcoxy en C₁-C₆)-alkyle en C₁-C₆, (alcoxy en C₁-C₆)-(alcoxy en C₁-C₆)-alkyle en C₁-C₆, cycloalcoxy en C₃-C₈, halogénoalcoxy en C₁-C₁₂, alkylthio en C₁-C₁₂, cycloalkylthio en C₃-C₈, halogénoalkylthio en C₁-C₁₂, alcényle en C₂-C₁₂, halogénoalcényle en C₂-C₁₂, halogénoalcényloxy en C₂-C₁₂, alcynyle en C₂-C₁₂, halogénoalcynyle en C₂-C₁₂ et halogénoalcynyloxy en C₃-C₁₂ ;
ou, lorsque p vaut 1, R₅ conjointement à R₄ est une liaison ;
ou, lorsque p vaut 0, R₅ conjointement à R₂ et R₄ est ≡N ;
R₇ est H, OH, un groupe alkyle en C₁-C₁₂, halogénoalkyle en C₁-C₁₂, alcényle en C₂-C₁₂, alcynyle en C₂-C₁₂, halogénoalcényloxy en C₂-C₁₂, halogénoalcynyle en C₂-C₁₂, halogénoalcynyloxy en C₃-C₁₂, alcoxy en C₁-C₁₂, halogénoalcoxy en C₁-C₁₂, (alcoxy en C₁-C₆)-alkyle en C₁-C₆, (alcoxy en C₁-C₆)-alcoxy en C₁-C₆, alcényloxy en C₂-C₈, alcynyloxy en C₃-C₈, -N(R₈)₂, où les deux substituants R₈ sont indépendants l'un de l'autre, aryle, aryloxy, benzyloxy, hétérocyclyle, hétérocyclyloxy ou hétérocyclylméthoxy ; et les radicaux aryle, aryloxy, benzyloxy, hétérocyclyle et hétérocyclyloxy sont non substitués ou, en fonction des possibilités de substitution sur le cycle, ils sont mono- à penta-substitués par des substituants choisis dans le groupe constitué de halogéno, CN, NO₂, alkyle en C₁-C₁₂, cycloalkyle en C₃-C₈, halogénoalkyle en C₁-C₁₂, alcoxy en C₁-C₁₂, halogénoalcoxy en C₁-C₁₂, alkylthio en C₁-C₁₂, halogénoalkylthio en C₁-C₁₂, (alcoxy en C₁-C₆)-alkyle en C₁-C₆, alcényle en C₂-C₈, halogénoacényle en C₂-C₁₂, halogénoacényloxy en C₂-C₁₂, alcynyle en C₂-C₈, halogénoalcynyle en C₂-C₁₂ et halogénoalcynyloxy en C₃-C₁₂ ;
R₈ est H, un groupe alkyle en C₁-C₆, qui est éventuellement substitué par un à cinq substituants choisis dans le groupe constitué de halogéno, alcoxy en C₁-C₆, (alcoxy en C₁-C₆)-alcoxy en C₁-C₆, alcényle en C₂-C₁₂, halogénoacényle en C₂-C₁₂, halogénoacényloxy en C₂-C₁₂, alcynyle en C₂-C₁₂, halogénoalcynyle en C₂-C₁₂, halogénoalcynyloxy en C₃-C₁₂, hydroxy et cyano, cycloalkyle en C₃-C₈, aryle, benzyle ou hétéroaryle ; où les radicaux aryle, benzyle et hétéroaryle sont non substitués ou, en fonction des possibilités de substitution sur le cycle, ils sont mono- à tri-substitués par des substituants choisis dans le groupe constitué de OH, halogéno, CN, NO₂, alkyle en C₁-C₁₂, halogénoallcyle en C₁-C₁₂, alcoxy en C₁-C₁₂, halogénoalcoxy en C₁-C₁₂, alkylthio en C₁-C₁₂, alcényle en C₂-C₁₂, halogénoacényle en C₂-C₁₂, halogénoacényloxy en C₂-C₁₂, alcynyle en C₂-C₁₂, halogénoalcynyle en C₂-C₁₂, halogénoalcynyloxy en C₃-C₁₂ et halogénoalkylthio en C₁-C₁₂ ;
R₉ est H, un groupe alkyle en C₁-C₆, cycloalkyle en C₁-C₆, (alcoxy en C₁-C₆)-alkyle en C₁-C₆, (alcoxy en C₁-C₆)-(alcoxy en C₁-C₆)-allcyle en C₁-C₆, alcényle en C₂-C₁₂, alcynyle en C₂-C₁₂, benzyle, aryle ou hétéroaryle ;
R₁₀ est H, un groupe alkyle en C₁-C₆, qui est éventuellement substitué par un à cinq substituants choisis dans le groupe constitué de halogéno, alcoxy en C₁-C₆, NO₂, hydroxy et cyano, halogénoalkyle en C₁-C₁₂, alcényle en C₂-C₁₂, halogénoalcynyle en C₂-C₁₂, halogénoacényle en C₂-C₁₂, alcynyle en C₂-C₁₂, cycloalkyle en C₃-C₈, aryle, benzyle ou hétéroaryle ; où les radicaux aryle, benzyle et hétéroaryle sont non substitués ou, en fonction des possibilités de substitution sur le cycle, ils sont mono- à tri-substitués par des substituants choisis dans le groupe constitué de OH, halogéno, CN, NO₂, alkyle en C₁-C₁₂, halogénoalkyle en C₁-C₁₂, alcoxy en C₁-C₁₂, halogénoalcoxy en C₁-C₁₂, alkylthio en C₁-C₁₂, halogénoalkylthio en C₁-C₁₂, alcényle en C₂-C₁₂, halogénoacényle en C₂-C₁₂, halogénoacényloxy en C₂-C₁₂, alcynyle en C₂-C₁₂, halogénoalcynyle en C₃-C₁₂ et halogénoalcynyloxy en C₃-C₁₂ ; R₁₁ est H, un groupe alkyle en C₁-C₆, qui est éventuellement substitué par un à cinq substituant choisis dans le groupe constitué de halogéno, alcoxy en C₁-C₆, hydroxy et cyano, -N(R₉)₂; où les deux substituants R₉ sont indépendants l'un de l'autre, cycloalkyle en C₃-C₈, halogénocycloalkyle en C₃-C₈, alcényle en C₂-C₁₂, halogénoalcényle en C₂-C₁₂, halogénoalcényloxy en C₂-C₁₂, alcynyle en C₂-C₁₂, halogénoalcynyle en C₃-C₁₂, halogénoalcényloxy en C₃-C₁₂, aryle, benzyle ou hétéroaryle ; où les radicaux aryle, benzyle et hétéroaryle sont non substitués ou, en fonction des possibilités de substitution sur le cycle, ils sont mono- à tri-substitués par des substituants choisis dans le groupe constitué de OH, halogéno, CN, NO₂, alkyle en C₁-C₁₂, halogénoalkyle en C₁-C₁₂, alcoxy en C₁-C₁₂, halogénoalcoxy en C₁-C₁₂, alkylthio en C₁-C₁₂, halogénoalkylthio en C₁-C₁₂, alcényle en C₂-C₁₂, halogénoacényle en C₂-C₁₂, halogénoacényloxy en C₂-C₁₂, alcynyle en C₂-C₁₂, halogénoalcynyle en C₂-C₁₂ et halogénoalcynyloxy en C₃-C₁₂ ;
R₁₂ est H, un groupe alkyle en C₁-C₆, cycloalkyle en C₁-C₆, (alcoxy en C₁-C₆)-alkyle en C₁-C₆, (alcoxy en C₁-C₆)-(alcoxy en C₁-C₆)-alkyle en C₁-C₆, alcényle en C₂-C₁₂, alcynyle en C₂-C₁₂, -C(=O)-alkyle en C₁-C₆, -C(=O)-O-alkyle en C₁-C₆, -SO₂-alkyle en C₁-C₆, benzyle, aryle ou hétéroaryle ;
X est O ou S;
ou, le cas échéant, un isomère E/Z, un mélange d'isomères E/Z et/ou un tautomère de celui-ci, dans chaque cas sous trois formes ou sous forme saline.

2. Pesticide qui contient au moins un composé de formule (I) tel que décrit dans la revendication 1, en tant que composé actif, et au moins un auxiliaire.

3. Composition telle que décrite dans la revendication 2 destinée à une utilisation dans un procédé de lutte contre les nuisibles, dans laquelle la composition est appliquée sur les nuisibles ou sur leur habitat.

4. Procédé de préparation d'une composition telle que décrite dans la revendication 2, qui contient au moins un auxiliaire, dans lequel le composé actif est intimement mélangé et/ou broyé avec le ou les auxiliaires.

5. Utilisation d'un composé de formule (I) tel que décrit dans la revendication 1, pour la préparation d'une composition telle que décrite dans la revendication 2.

6. Composition telle que décrite dans la revendication 2, destinée à une utilisation pour lutter contre les nuisibles.

7. Procédé selon la revendication 3, de protection d'une matière de propagation végétale, dans lequel la matière de propagation ou l'endroit où la matière de propagation est plantée est traitée.

8. Matière de propagation végétale traitée selon le procédé décrit dans la revendication 7.

9. Composé de formule (I) selon la revendication 1, destiné à une utilisation pour lutter contre les nuisibles.

10. Composé de formule (I) selon la revendication l, destiné à une utilisation pour protéger des animaux domestiques contre une infection par des mouches, des tiques ou des nématodes.
